# EUROPEAN PATENT APPLICATION

(11) **EP 4 847 514 A1**
(43) Date of publication of application: **02.12.2026**
(21) Application number: 25744709.4
(22) Date of filing: 24.01.2025
(51) Int. Cl.: C07D 498/22, A61K 31/553, A61P 35/00

(54) **PYRIMIDINE POLYCYCLIC DERIVATIVE INHIBITOR, PREPARATION METHOD THEREFOR AND USE THEREOF**

(30) Priority: 24.01.2024 CN 202410102208; 01.02.2024 CN 202410146840
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: LIU, Shiqiang, Shanghai 201203 (CN); YU, Mingcheng, Shanghai 201203 (CN); YUAN, Yida, Shanghai 201203 (CN); LI, Peng, Shanghai 201203 (CN); LI, Haiqing, Shanghai 201203 (CN)
(74) Representative: Cabinet Beau de Loménie
(86) International application number: PCT/CN2025/074536
(87) International publication number: WO 2025/157246

(57) **Abstract**

Provided are a pyrimidine polycyclic derivative inhibitor, a preparation method therefor and the use thereof. In particular, provided are a compound as shown in the general formula, a preparation method therefor, a pharmaceutical composition comprising the compound, and the use thereof in the treatment of cancers and other related diseases.

## Description

### Technical Field

The present invention belongs to the field of drug synthesis, and specifically relates to a pyrimidine polycyclic derivative inhibitor, and a preparation method therefor and the use thereof.

### Background Art

Rat sarcoma (RAS) protein is encoded by proto-oncogenes HRAS, NRAS and KRAS, is divided into four proteins HRAS, NRAS, KRAS4A and KRAS4B, and is a GTP (guanosine triphosphate) binding protein. RAS is located on the inner surface of the cell membrane, and its upstream is receptor tyrosine kinase (RTK). Upon activation, RAS regulates downstream signalling pathways such as PI3K and RAF, thereby regulating cell growth, survival, migration, differentiation and other functions.

RAS has two main states in the body: an inactive state bound to GDP (guanosine diphosphate) and an activated state bound to GTP. Its activity is regulated by two proteins: guanine nucleotide exchange factor (GEF) which promotes the release of GDP from RAS protein, allowing GTP to bind and activate RAS; GTPase activating protein (GAP), which activates the GTPase activity of RAS protein, hydrolyses GTP bound to RAS protein into GDP and inactivates RAS. Under normal circumstances, RAS protein is in an inactive state. After mutation, its conformation changes, and RAS is in a continuously activated state, and the downstream signalling pathways are also continuously activated, leading to the occurrence of various cancers.

RAS is the first confirmed oncogene and the oncogene with the highest mutation rate, accounting for an average of 25% in human cancers. The most common oncogenic mutation in the RAS family is KRAS (85%), while NRAS (12%) and HRAS (3%) are less common. KRAS mutations are mainly found in a series of cancers such as pancreatic cancer (95%), colorectal cancer (52%), and lung cancer (31%). The most common mutation of KRAS is point mutation, which often occurs at G12 and G13 in the p-loop (aa 10 to 17) and at Q61 in the Switch II region (aa 59 to 76), among which G12 mutation is the most common (83%). Epidemiological studies in European and American countries have shown that KRAS G12D is the most common pathogenic mutation in pancreatic cancer, intestinal cancer, endometrial cancer and lung cancer, with incidence rates of 36%, 12%, 6% and 4%, respectively.

Although there is a huge clinical need, there is no drug directly targeting KRAS on the market so far. Currently, the only clinical treatment for patients with KRAS mutations is chemotherapy. There are two main factors that make the development of KRAS inhibitors difficult. Firstly, the RAS protein has a smooth structure, making it difficult for small molecules to bind to the protein surface; secondly, the affinity of RAS GTPase for GTP is as high as picomolar (pM) level, and the level of endogenous GTP is high, making it difficult for small molecule drugs to block the binding of RAS GTPase and GTP. Currently, no KRAS G12D inhibitors have entered clinical trials.

There are currently no specific targeted drugs for KRAS G12D, and there is great clinical demand. KRAS G12D inhibitors with a higher selectivity, a better activity and a better safety have the potential to treat a variety of cancers and have broad market prospects.

### Summary of the Invention

An object of the present invention is to provide a compound represented by general formula (A), or an N-oxide, an oxo derivative, or a pharmaceutically acceptable salt thereof: 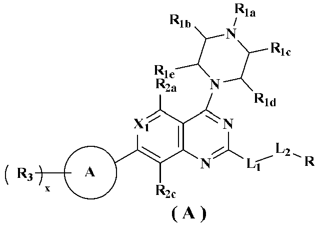 wherein:
X₁ is selected from N or CR₂₆, preferably N; L₁ is selected from O, S or NH, preferably O;
L₂ is C₁-C₄ alkylene, and the C₁-C₄ alkylene is optionally substituted with 1, 2, 3, 4, 5, or 6 Rₐ;
ring A is selected from C₃₋₁₀ cycloalkyl, 3- to 10-membered heterocyclyl, C₆₋₁₂ aryl, or 5- to 12-membered heteroaryl;
R is selected from -OR_{b}, -CH₂OR_{b}, -C(O)R_{b}, -C(O)OR_{b}, 3- to 10-membered heterocyclyl, or 5- to 12-membered heteroaryl, and the 3- to 10-membered heterocyclyl or 5- to 12-membered heteroaryl is optionally substituted with 1, 2, 3, 4, 5, 6, 7, or 8 R₄ₐ substituents;
R₁ₐ is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, -OR_{b}, -OC(O)R_{b}, -OC(O)NR_{b}R_{c}, - OS(O)₂R_{b}, or -OS(O)₂NR_{b}R_{c}, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{1b}, R_{1c}, R_{1d}, and R₁ₑ are each independently selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5-to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, R_{1b} and R_{1c} together with adjacent atoms to which they are attached form 3- to 12-membered heterocyclyl or 5- to 14-membered heteroaryl optionally substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R₂ₐ is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, or (CH₂)n₁ORₑ, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, R₂ₐ and R₁ₑ are attached to form 3- to 12-membered heterocyclyl or 5- to 14-membered heteroaryl optionally substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{2b} is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{2c} is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
alternatively, any two R₃ groups are attached to form C₃₋₈ cycloalkyl, 3- to 8-membered heterocyclyl, C₆₋₁₀ aryl, or 5- to 10-membered heteroaryl, and the C₃₋₈ cycloalkyl, 3- to 8-membered heterocyclyl, C₆₋₁₀ aryl, and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R₄ₐ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, =CRₐₐR_{bb}, or N-OR_{cc}, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, any two R₄ₐ groups are attached to form C₃₋₈ cycloalkyl or 3- to 8-membered heterocyclyl, and the C₃₋₈ cycloalkyl and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, or 5- to 10-membered heteroaryl;
Rₐ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, 2 Rₐ groups on the same carbon atom or on different carbon atoms are attached to form C₃₋₁₂ cycloalkyl or 3- to 12-membered heterocyclyl, which is optionally further substituted with one or more groups selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
R_{c} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
Rₑ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
Rₐₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{bb} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{cc} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
n1 is selected from 0, 1, 2, or 3; x is selected from 0, 1, 2, 3, 4, 5, or 6.

In certain embodiments of the present invention, the compound represented by general formula (A), or the N-oxide, the oxo derivative, or the pharmaceutically acceptable salt thereof is characterised in that the compound is further as represented by general formula (A-1): 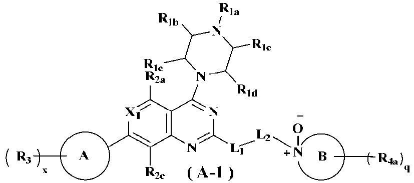 ring B is selected from 3- to 10-membered heterocyclyl or 5- to 12-membered heteroaryl; q is selected from 0, 1, 2, 3, 4, 5, 6, 7, or 8.

In certain embodiments of the present invention, the compound represented by general formula (A), or the N-oxide, the oxo derivative, or the pharmaceutically acceptable salt thereof is characterised in that the compound is further as represented by general formula (A-2): 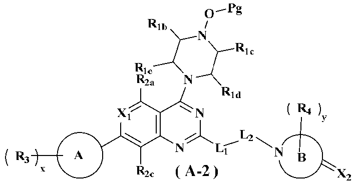
X₂ is selected from O, CR₅ₐR_{5b}, or N-OR_{5c}; ring B is selected from 3- to 10-membered heterocyclyl or 5- to 12-membered heteroaryl;
Pg is selected from hydrogen or a hydroxyl protecting group; the hydroxyl protecting group is selected from methyl, tert-butyl, triphenyl, methylthiomethyl ether, 2-methoxyethoxymethyl ether, methoxymethyl ether, p-methoxybenzyl ether, pivaloyl, benzyl ether group, methoxymethyl, trimethylsilyl, tetrahydrofuryl, tertbutyldimethylsilyl, acetyl, benzoyl, p-toluenesulphonyl, -C(O)R_{b}, -C(O)NR_{b}R_{c}, - S(O)₂R_{b}, or -S(O)₂NR_{b}R_{c};
R₄ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, any two R₄ groups are attached to form C₃₋₈ cycloalkyl or 3- to 8-membered heterocyclyl, and the C₃₋₈ cycloalkyl and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, or 5- to 10-membered heteroaryl;
R₅ₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{5b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{5c} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{dd} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆silyl, C₃₋₈ cycloalkyl, 3- to 8-membered heterocyclyl, C₆₋₁₀ aryl, and 5- to 10-membered heteroaryl;
Rₑₑ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆silyl, C₃₋₈ cycloalkyl, 3- to 8-membered heterocyclyl, C₆₋₁₀ aryl, and 5- to 10-membered heteroaryl;
R_{ff} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆silyl, C₃₋₈ cycloalkyl, 3- to 8-membered heterocyclyl, C₆₋₁₀ aryl, and 5- to 10-membered heteroaryl;
n1 is selected from 0, 1, 2, 3, or 4; and y is selected from 0, 1, 2, 3, 4, 5, 6, 7, or 8.

In certain embodiments of the present invention, the compound represented by general formula (A), or the N-oxide, the oxo derivative, or the pharmaceutically acceptable salt thereof is characterised in that the compound is further as represented by general formula (I) or (I-A): 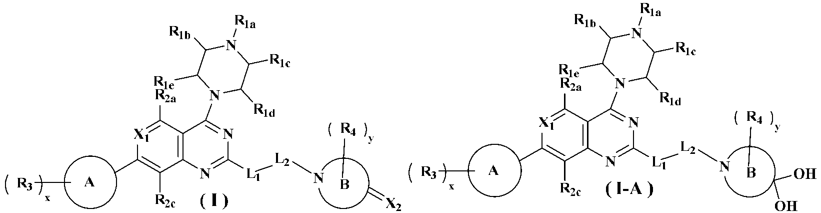 wherein:
X₂ is selected from O, CR₅ₐR_{5b}, or N-OR_{5c}; ring B is selected from 3- to 10-membered heterocyclyl or 5- to 12-membered heteroaryl;
R₄ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, any two R₄ groups are attached to form C₃₋₈ cycloalkyl or 3- to 8-membered heterocyclyl, and the C₃₋₈ cycloalkyl and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, or 5- to 10-membered heteroaryl;
R₅ₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C_{6-1O} aryl, and 5- to 10-membered heteroaryl;
R_{5b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R₅, is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆silyl, C₃₋₈ cycloalkyl, 3- to 8-membered heterocyclyl, C₆₋₁₀ aryl, and 5- to 10-membered heteroaryl;
y is selected from 0, 1, 2, 3, 4, 5, or 6.

In certain embodiments of the present invention, R_{1b} and R_{1c} together with adjacent atoms to which they are attached form the following structure 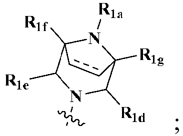 preferably form the following structure 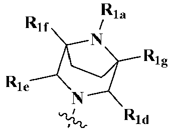 or 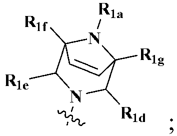 more preferably form the following structure 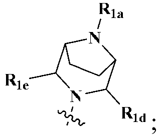 R_{1f} and R_{1g} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl.

In certain embodiments of the present invention, the compound represented by general formula (A), or the N-oxide, the oxo derivative, or the pharmaceutically acceptable salt thereof is characterised in that the compound is further as represented by general formula (II-A) or (II-B): 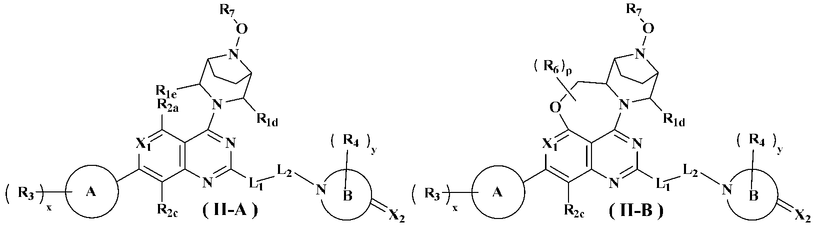
R₆ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, - C(O)-C₁₋₆ alkyl, C₃₋₈ cycloalkyl, 3- to 8-membered heterocyclyl, C₆₋₁₀ aryl, or 5- to 10-membered heteroaryl;
R₇ is selected from hydrogen, C₁₋₆ alkyl, C₂₋₆ alkenyl, C₂₋₆ alkynyl, C₁₋₆ deuterated alkyl, C₁₋₆ haloalkyl, C₃₋₈ cycloalkyl, 3- to 8-membered heterocyclyl, C₆₋₁₀ aryl, 5- to 10-membered heteroaryl, C(O)R_{b}, -C(O)NR_{b}R_{c}, -S(O)₂R_{b}, or - S(O)₂NR_{b}R_{c};
R_{b} is selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
R_{c} is selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
p is selected from 0, 1, 2, 3, or 4.

In certain embodiments of the present invention, the compound represented by general formula (A), or the N-oxide, the oxo derivative, or the pharmaceutically acceptable salt thereof is characterised in that the compound is further as represented by general formula (III-A) or (III-B): 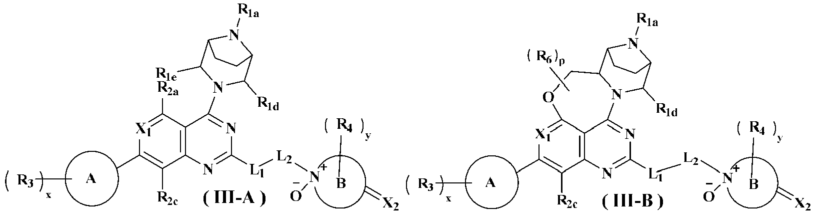 R₆ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, - C(O)-C₁₋₆ alkyl, C₃₋₈ cycloalkyl, 3- to 8-membered heterocyclyl, C₆₋₁₀ aryl, or 5- to 10-membered heteroaryl; p is selected from 0, 1, 2, 3, or 4.

In certain embodiments of the present invention, R₅ₐ is selected from hydrogen, deuterium, halogen, cyano, C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, or 3- to 8-membered heterocyclyl, and the C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; and is preferably hydrogen, deuterium, fluorine, chlorine, bromine, methyl, ethyl, deuterated methyl, deuterated ethyl, halomethyl, haloethyl, or cyclopropyl;

R_{5b} is selected from hydrogen, deuterium, halogen, cyano, C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, or 3- to 8-membered heterocyclyl, and the C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; and is preferably hydrogen, deuterium, fluorine, chlorine, bromine, methyl, ethyl, deuterated methyl, deuterated ethyl, halomethyl, haloethyl, or cyclopropyl.

In certain embodiments of the present invention, R_{5c} is selected from C₁₋₃ alkyl, C₁₋₃ deuterated alkyl, C₂₋₄ alkenyl, C₂₋₄ alkynyl, C₁₋₃ haloalkyl, C₁₋₃ hydroxyalkyl, C₃₋₈ cycloalkyl, or 3- to 8-membered heterocyclyl, and the C₁₋₃ alkyl, C₁₋₃ deuterated alkyl, C₂₋₄ alkenyl, C₂₋₄ alkynyl, C₁₋₃ haloalkyl, C₁₋₃ hydroxyalkyl, C₃₋₈ cycloalkyl, and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; and is preferably methyl, ethyl, deuterated methyl, deuterated ethyl, halomethyl, haloethyl, cyclopropyl, or cyclopropylmethyl.

In certain embodiments of the present invention, ring B is selected from 3- to 8-membered monocyclic heterocyclyl, 7- to 10-membered fused heterocyclyl, 7- to 11-membered spiro heterocyclyl, or 7- to 10-membered bridged heterocyclyl;
and is preferably the following group:

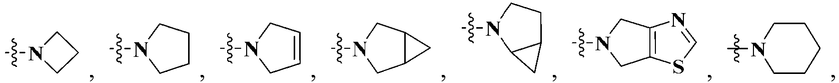

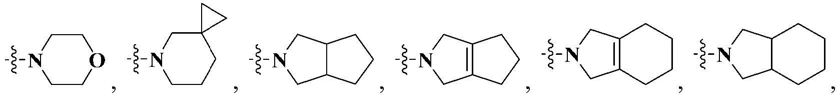

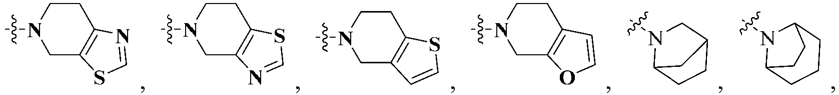

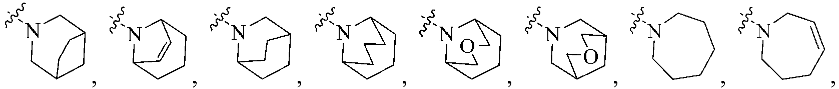

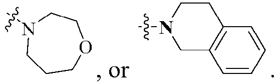

In certain embodiments of the present invention, R₄ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
and is preferably hydrogen, deuterium, fluorine, chlorine, bromine, amino, nitro, hydroxyl, cyano, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, or hydroxyethyl.

In certain embodiments of the present invention, L₂ is selected from 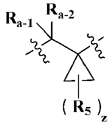 or 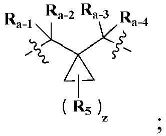 preferably, 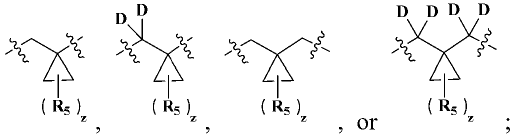 alternatively, L₂ is 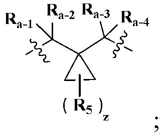 preferably 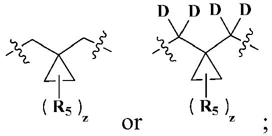
Rₐ₋₁, Rₐ₋₂, Rₐ₋₃ and Rₐ₋₄ are each independently selected from hydrogen, deuterium, halogen, amino, hydroxyl, cyano, C₁₋₆ alkyl, C₁₋₆ deuterated alkyl, C₁₋₆ haloalkyl, or C₁₋₆ hydroxyalkyl; preferably hydrogen or deuterium;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; z is selected from 0, 1, 2, 3, or 4.

In certain embodiments of the present invention, R_{1d} is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, and C₁₋₃ alkylamino may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; preferably hydrogen, deuterium, fluorine, chlorine, or methyl.

In certain embodiments of the present invention, R₁ₑ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, and C₁₋₃ alkylamino may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; preferably hydrogen, deuterium, fluorine, chlorine, or methyl.

In certain embodiments of the present invention, R₂ₐ is selected from hydrogen, deuterium, halogen, amino, hydroxyl, mercapto, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, or -(CH₂)ₙ₁ORₑ, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;

Rₑ is selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl; n1 is selected from 0, 1, 2, or 3.

In certain embodiments of the present invention, R_{2c} is selected from hydrogen, deuterium, halogen, amino, hydroxyl, mercapto, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino; preferably, R_{2c} is selected from hydrogen, deuterium, fluorine, chlorine or bromine; more preferably, R_{2c} is fluorine.

In certain embodiments of the present invention, the compound, or the N-oxide, the oxo derivative, or the pharmaceutically acceptable salt thereof is characterised in that the compound is further as represented by general formula (IV) or (V): 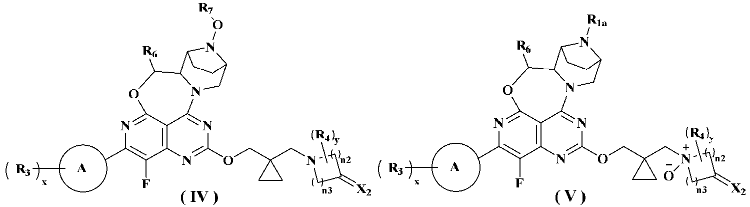 n2 is selected from 0, 1, 2, or 3; n3 is selected from 0, 1, 2, or 3.

In certain embodiments of the present invention, R₆ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ deuteroalkoxy, C₁₋₃ haloalkoxy, C₁₋₃ hydroxyalkyl, -C(O)-C₁₋₃ alkyl, C₃₋₈ cycloalkyl, 3- to 8-membered heterocyclyl, C₆₋₁₀ aryl, or 5- to 10-membered heteroaryl;
and is preferably hydrogen, deuterium, methyl, ethyl, deuterated methyl, deuterated ethyl, halomethyl, or haloethyl;
in certain embodiments of the present invention, 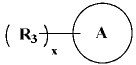 is selected from 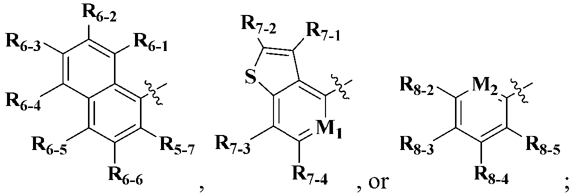
M₁ is selected from CR₇₋₅ or N; M₂ is selected from CR₈₋₁ or N;
and is preferably 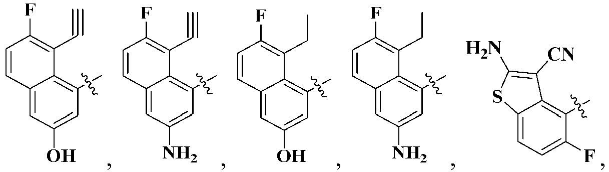 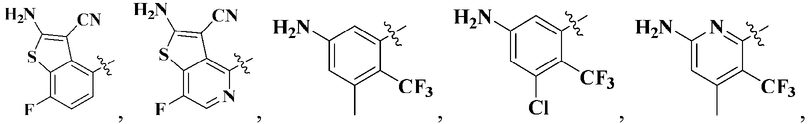 or 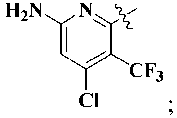
R₆₋₁, R₆₋₂, R₅₆₋₃, R₆₋₄, R₆₋₅, R₆₋₆, and R₆₋₇ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R₇₋₁, R₇₋₂, R₇₋₃, R₇₋₄, and R₇₋₅ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R₈₋₁, R₈₋₂, R₈₋₃, R₈₋₄, and R₈₋₅ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl.

The present invention further provides a compound represented by general formula (IV-C), or an N-oxide, an oxo derivative, or a pharmaceutically acceptable salt thereof: 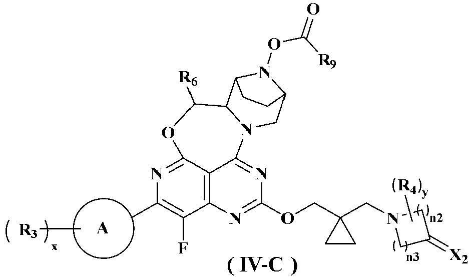
wherein R₉ is selected from C₁₋₆ alkyl, C₁₋₆ deuterated alkyl, C₁₋₆ haloalkyl, C₁₋₆ hydroxyalkyl, C₃₋₁₂ cycloalkyl, 3- to 12-membered heterocyclyl, C₆₋₁₄ aryl, or 5- to 14-membered heteroaryl, and the C₁₋₆ alkyl, C₁₋₆ deuterated alkyl, C₁₋₆ haloalkyl, C₁₋₆ hydroxyalkyl, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
ring A, X₂, R₃, R₄, R₆, n2, n3, x, and y are as defined in any of the preceding embodiments.

The present invention further provides a compound represented by general formula (V-B), or an N-oxide, an oxo derivative, or a pharmaceutically acceptable salt thereof: 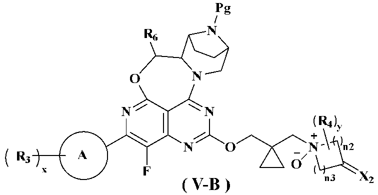
wherein Pg is selected from allyloxycarbonyl, trifluoroacetyl, tert-butylsulphinyl 2,4-dimethoxybenzyl, nitrobenzenesulphonyl, trityl, fluorenylmethoxycarbonyl, 9-fluorenylmethoxycarbonyl, benzyl, p-toluenesulphonyl, p-methoxybenzyl, carboxylate, acetyl, benzyloxycarbonyl, phthaloyl, tert-butyloxycarbonyl, benzyl, or p-methoxyphenyl;
ring A, X₂, R₃, R₄, R₆, n2, n3, x, and y are as defined in any of the preceding embodiments.

The present invention further provides a method for preparing a compound represented by general formula (II-B), or an N-oxide, an oxo derivative or a pharmaceutically acceptable salt thereof, comprising the step of: 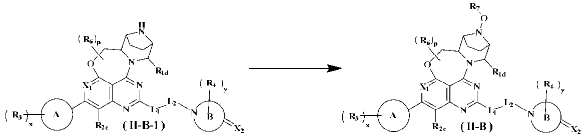
reacting a compound represented by general formula (II-B-1) with a peroxide to obtain the compound represented by general formula (II-B);
wherein ring A, ring B, L₁, L₂, X₁, X₂, R_{1d}, R₂ₐ, R_{2c}, R₃, R₄, R₆, R₇, p, x, and y are as defined in any of the preceding embodiments.

In certain embodiments of the present invention, the preparation method is a method for preparing a compound represented by general formula (IV), comprising: 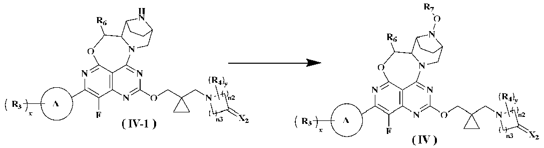
reacting a compound represented by general formula (IV-1) with a peroxide to obtain the compound represented by general formula (IV);
wherein optionally, when R7 is -C(O)R₉, the compound represented by general formula (IV) is further subjected to ester hydrolysis under alkaline conditions to obtain the target compound; the base is selected from sodium hydroxide, potassium hydroxide, sodium carbonate, sodium bicarbonate, potassium carbonate, lithium hydroxide, triethylamine, or pyridine;
ring A, ring B, L₁, L₂, X₁, X₂, R_{1d}, R₂ₐ, R_{2c}, R₃, R₄, R₆, R₇, R₉, p, x, y, n2, and n3 are as defined in any of the preceding embodiments.

The present invention further provides a method for preparing a compound represented by general formula (III-B), or an N-oxide, an oxo derivative or a pharmaceutically acceptable salt thereof, comprising the step of: 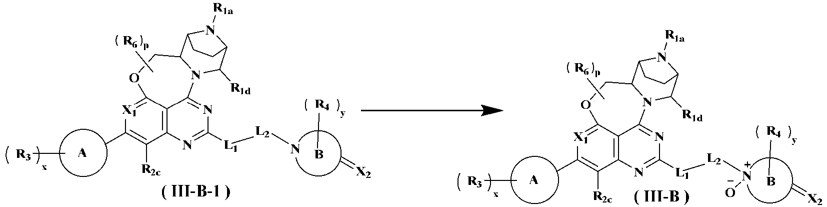
reacting a compound represented by general formula (III-B-1) with a peroxide to obtain the compound represented by general formula (III-B);
wherein ring A, ring B, L₁, L₂, X₁, X₂, R_{1d}, R₂ₐ, R_{2c}, R₃, R₄, R₆, R₇, p, x, and y are as defined in any of the preceding embodiments.

In certain embodiments of the present invention, the preparation method is a method for preparing a compound represented by general formula (V), comprising: 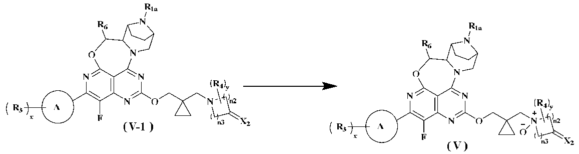
reacting a compound represented by general formula (V-1) with a peroxide to obtain the compound represented by general formula (V);
wherein optionally, when R₁ₐ is Pg, the compound represented by general formula (V) is further subjected to deprotection of Pg under acidic conditions to obtain the target compound; the acid is selected from hydrochloric acid, sulphuric acid, or trifluoroacetic acid;
ring A, ring B, Pg, L₁, L₂, X₁, X₂, R_{1d}, R₂ₐ, R_{2c}, R₃, R₄, R₆, R₇, p, x, y, n2, and n3 are as defined in any of the preceding embodiments.

In certain embodiments of the present invention, the peroxide in the preparation method is selected from hydrogen peroxide, sodium peroxide, perbenzoic acid, m-chloroperoxybenzoic acid, or benzoyl peroxide.

The present invention further provides a pharmaceutical composition, comprising a therapeutically effective dose of the above-mentioned compound, or the N-oxide, the oxo derivative, or the pharmaceutically acceptable salt thereof, as well as one or more pharmaceutically acceptable carriers, diluents or excipients.

In certain embodiments of the present invention, the above-mentioned compound, or the N-oxide, the oxo derivative, or the pharmaceutically acceptable salt thereof is present in the composition in an amount of 0.1% to 95% by weight, preferably 0.5% to 85% by weight, more preferably 1% to 60% by weight, further preferably 10% to 50% by weight, further more preferably 15% to 40% by weight, further more preferably 20% to 30% by weight, and further more preferably 20% to 25% by weight (based on the total weight of the pharmaceutical composition).

In certain embodiments of the present invention, the pharmaceutical composition is selected from a tablet, a capsule, a liquid preparation, an injection, a suspension, a powder, a granule, or a lozenge, preferably further comprises a filler, optionally further comprises a disintegrant, or further comprises one or more of a glidant or a lubricant.

In certain embodiments of the present invention, the pharmaceutical composition is an immediate-release preparation or a sustained-release preparation.

The present invention further relates to the use of the above-mentioned compound, or the N-oxide, the oxo derivative, or the pharmaceutically acceptable salt thereof, or the pharmaceutical composition in the preparation of a KRAS inhibitor drug; preferably a KRAS G12D, KRAS G12V, or KRAS G13D inhibitor drug.

The present invention further relates to the use of the above-mentioned compound, or the N-oxide, the oxo derivative, or the pharmaceutically acceptable salt thereof, or the pharmaceutical composition thereof in the preparation of a drug for treating diseases or conditions such as Noonan syndrome, Leopard syndrome, leukaemia, neuroblastoma, melanoma, oesophageal cancer, head and neck tumour, brain cancer, breast cancer, lung cancer, liver cancer, gastric cancer, renal cancer, bile duct carcinoma, prostate cancer, ovarian cancer, pancreatic cancer, small intestine cancer, and colon cancer; preferably in the preparation of a drug for treating non-small cell lung cancer, colon cancer, pancreatic cancer, oesophageal cancer, and head and neck tumour.

The present invention further relates to the above-mentioned compound, or the N-oxide, the oxo derivative, or the pharmaceutically acceptable salt thereof, or the pharmaceutical composition thereof for use in a method for treating diseases or conditions such as Noonan syndrome, Leopard syndrome, leukaemia, neuroblastoma, melanoma, oesophageal cancer, head and neck tumour, brain cancer, breast cancer, lung cancer, liver cancer, gastric cancer, renal cancer, bile duct carcinoma, prostate cancer, ovarian cancer, pancreatic cancer, small intestine cancer, and colon cancer; preferably a method for treating non-small cell lung cancer, colon cancer, pancreatic cancer, oesophageal cancer, and head and neck tumour.

The present invention also relates to a method for treating and/or preventing diseases or conditions such as Noonan syndrome, Leopard syndrome, leukaemia, neuroblastoma, melanoma, oesophageal cancer, head and neck tumour, brain cancer, breast cancer, lung cancer, liver cancer, gastric cancer, renal cancer, bile duct carcinoma, prostate cancer, ovarian cancer, pancreatic cancer, small intestine cancer, and colon cancer, the method comprising administering to a patient a therapeutically effective dose of the above-mentioned compound, or the N-oxide, the oxo derivative, or the pharmaceutically acceptable salt thereof, or the pharmaceutical composition thereof; preferably a method for treating non-small cell lung cancer, colon cancer, pancreatic cancer, oesophageal cancer, and head and neck tumour.

In certain embodiments of the present invention, the above-mentioned compound, or the N-oxide, the oxo derivative, or the pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

### Detailed Description of the Invention

Unless stated to the contrary, all technical and scientific terms used herein generally have the same meanings as commonly understood by one of ordinary skill in the art. Specifically, the terms used in the description and claims have the following meanings.

The term "alkyl" refers to a linear or branched saturated aliphatic hydrocarbon group which may be optionally substituted with one or more substituents. In particular embodiments, the alkyl refers to a linear saturated hydrocarbon group having 1 to 20 (C₁₋₂₀), 1 to 15 (C₁₋₁₅), 1 to 12 (C₁₋₁₂), 1 to 10 (C₁₋₁₀), 1 to 8 (C₁₋₈), 1 to 6 (C₁₋₆), or 1 to 3 (C₁₋₃) carbon atoms, or a branched saturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. As used herein, linear C₁₋₆ alkyl and branched C₃₋₆ alkyl groups are also referred to as "lower alkyl". For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the C₁₋₆ alkyl contains 1 to 6 (e.g., 1, 2, 3, 4, 5, or 6) carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, etc. In one embodiment, the alkyl is optionally substituted alkyl as described elsewhere herein.

The term "alkylene" refers to alkyl in which one hydrogen atom is further substituted, wherein the "alkyl" is as defined above. Non-limiting examples of "alkylene" include: methylene (-CH₂-), ethylene (-(CH₂)₂-), propylene (-(CH₂)₃-) or butylene (-(CH₂)₄-). In one embodiment, the alkylene is optionally substituted alkyl as described elsewhere herein.

The term "alkenyl" refers to a linear or branched unsaturated aliphatic hydrocarbon group containing at least one carbon-carbon double bond which may be located at any position within the alkenyl, and the alkenyl may be optionally substituted with one or more substituents. In particular embodiments, the alkenyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆) or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkenyl" as used herein includes both linear and branched alkenyl. For example, C₂₋₆ alkenyl refers to a linear unsaturated hydrocarbon group having 2 to 6 carbon atoms or a branched unsaturated hydrocarbon group having 3 to 6 carbon atoms. In one embodiment, the C₂₋₆ alkenyl contains 2 to 6 (e.g., 2, 3, 4, 5 or 6) carbon atoms. Non-limiting examples of alkenyl include: 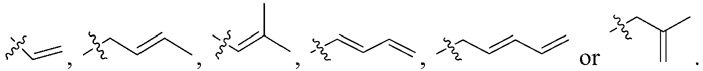 One of ordinary skill in the art can understand that the term "alkenyl" may also include groups having "cis" and "trans" configurations, or alternatively, groups having "E" and "Z" configurations. In one embodiment, the alkenyl is optionally substituted alkenyl as described elsewhere herein.

The term "alkynyl" refers to a linear or branched unsaturated aliphatic hydrocarbon group containing at least one carbon-carbon triple bond which may be located at any position within the alkynyl, and the alkynyl may be optionally substituted with one or more substituents. In particular embodiments, the alkynyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkynyl" as used herein includes both linear and branched alkynyl. For example, C₂₋₆ alkynyl refers to a linear unsaturated hydrocarbon group having 2 to 6 carbon atoms or a branched unsaturated hydrocarbon group having 3 to 6 carbon atoms. In one embodiment, the C₂₋₆ alkynyl contains 2 to 6 (e.g., 2, 3, 4, 5, or 6) carbon atoms. Non-limiting examples of alkynyl include: 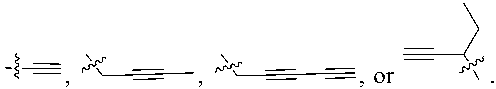 In one embodiment, the alkynyl is optionally substituted alkynyl as described elsewhere herein.

The term "cycloalkyl" refers to a saturated or partially unsaturated aliphatic hydrocarbon cyclic group, which may be monocyclic or polycyclic (containing two or more rings), and which may be optionally substituted with one or more substituents. In particular embodiments, the cycloalkyl ring contains 3 to 20 (C₃₋₂₀), 3 to 12 (C₃₋₁₂), 3 to 8 (C₃₋₈), or 3 to 6 (C₃₋₆) carbon atoms. In one embodiment, the cycloalkyl ring contains 6 to 14 (C₆₋₁₄) or 7 to 10 (C₇₋₁₀) carbon atoms; it may contain one or more double bonds, but does not have a completely conjugated π electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spirocycloalkyl, fused cycloalkyl and bridged cycloalkyl in one embodiment. In one embodiment, the cycloalkyl is optionally substituted cycloalkyl as described elsewhere herein or cycloalkyl that is optionally fused to heterocyclyl, aryl or heteroaryl, and non-limiting examples include indanyl, tetrahydronaphthyl, benzocycloheptyl, etc.

The term "spirocycloalkyl" refers to an aliphatic hydrocarbon polycyclic group with monocyclic rings sharing one carbon atom (called a spiro atom); and it may contain one or more double bonds, but no ring has a completely conjugated π electron system. In particular embodiments, the spirocycloalkyl contains 5 to 20 (C₅₋₂₀), 6 to 14 (C₆₋₁₄) or 7 to 10 (C₇₋₁₀) (e.g., 7, 8, 9, or 10) carbon atoms. According to the number of shared spiro atoms between the rings, the spirocycloalkyl is divided into monospirocycloalkyl, bispirocycloalkyl or polyspirocycloalkyl. In one embodiment, the spirocycloalkyl is monospirocycloalkyl and bispirocycloalkyl. In one embodiment, the spirocycloalkyl is 4-membered/4-membered, 3-membered/5-membered, 4-membered/5-membered, 4-membered/6-membered, 5-membered/5-membered, or 5-membered/6-membered monospirocycloalkyl. In one embodiment, the spirocycloalkyl is optionally substituted spirocycloalkyl as described elsewhere herein. Non-limiting examples of spirocycloalkyl include:

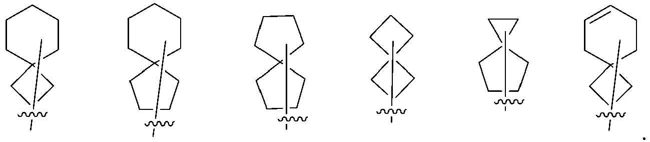

The term "fused cycloalkyl" refers to an all-carbon polycyclic group with each ring in the system sharing a pair of adjacent carbon atoms with another ring in the system, wherein one or more rings may contain one or more double bonds, but no ring has a completely conjugated π electron system. In particular embodiments, the fused cycloalkyl contains 5 to 20 (C₅₋₂₀), 6 to 14 (C₆₋₁₄) or 7 to 10 (C₇₋₁₀) (e.g., 7, 8, 9, or 10) carbon atoms. According to the number of constituent rings, the fused cycloalkyl can be divided into bicyclic, tricyclic, tetracyclic, or polycyclic fused cycloalkyl. In one embodiment, the fused cycloalkyl is bicyclic or tricyclic, and in a further embodiment, the fused cycloalkyl is 3-membered/5-membered, 4-membered/5-membered, 5-membered/5-membered, or 5-membered/6-membered bicyclic cycloalkyl. In one embodiment, the fused cycloalkyl is optionally substituted fused cycloalkyl as described elsewhere herein, or fused cycloalkyl that is optionally fused to heterocyclyl, aryl or heteroaryl. Non-limiting examples of fused cycloalkyl include: 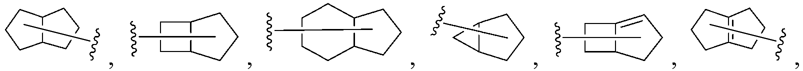 or

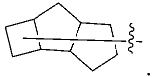

The term "bridged cycloalkyl" refers to an all-carbon polycyclic group in which any two rings share two carbon atoms that are not directly connected; and it may contain one or more double bonds, but no ring has a completely conjugated π electron system. In particular embodiments, the bridged cycloalkyl contains 5 to 20 (C₅₋₂₀), 6 to 14 (C₆₋₁₄), or 7 to 10 (C₇₋₁₀) (e.g., 7, 8, 9, or 10) carbon atoms. According to the number of constituent rings, the bridged cycloalkyl can be divided into bicyclic, tricyclic, tetracyclic, or polycyclic bridged cycloalkyl, preferably bicyclic or tricyclic. In one embodiment, the bridged cycloalkyl is optionally substituted bridged cycloalkyl as described elsewhere herein. Non-limiting examples of bridged cycloalkyl include:

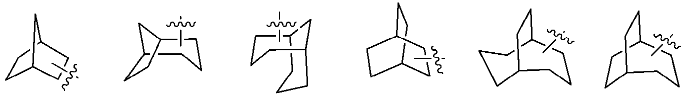

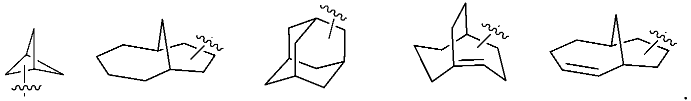

The term "heterocyclyl" refers to a saturated or partially unsaturated monocyclic or polycyclic cyclic hydrocarbon group, wherein one or more ring atoms are heteroatoms selected from nitrogen, oxygen, boron, phosphorus or sulphur, wherein the nitrogen, phosphorus or sulphur atom may be optionally oxidized, the nitrogen atom may be optionally quaternized, the ring carbon atoms may be optionally substituted with oxygen, but excluding ring moieties of -O-O- and -O-S-, and the remaining ring atoms are carbon atoms. The heterocyclyl may contain one or more double bonds but do not have a completely conjugated π electron system. In particular embodiments, the heterocyclyl contains 3 to 20, 3 to 12, 3 to 8, or 3 to 6 ring atoms, of which 1 to 4 are heteroatoms. In one embodiment, the heterocyclyl contains 3 to 6, 4 to 6, 3 to 8, 3 to 10, 6 to 10, or 7 to 11 ring atoms. In one embodiment, the heterocyclyl contains 3 to 8 (e.g., 3, 4, 5, 6, 7, or 8) ring atoms. Non-limiting examples of monocyclic heterocyclyl include tetrahydropyrrolyl, azetidinyl, oxetanyl, oxanyl, tetrahydrofuryl, tetrahydropyranyl, tetrahydrothienyl, dihydroimidazolyl, dihydrofuryl, dihydropyrazolyl, dihydropyrrolyl, piperidinyl, piperazinyl, morpholinyl, thiomorpholinyl, homopiperazinyl, pyranyl, etc. Polycyclic heterocyclyl includes spiroheterocyclyl, fused heterocyclyl and bridged heterocyclyl. In one embodiment, the heterocyclyl is optionally substituted heterocyclyl as described elsewhere herein, or heterocyclyl that is further fused to other cycloalkyl, heterocyclyl, aryl and heteroaryl through any two or more atoms on the ring.

The term "spiroheterocyclyl" refers to a polycyclic heterocyclic group in which the rings share one atom (called a spiro atom), wherein one or more ring atoms are heteroatoms selected from nitrogen, oxygen, boron, phosphorus or sulphur, and the remaining ring atoms are carbon atoms. The spiroheterocyclyl may contain one or more double bonds, but no ring has a completely conjugated π electron system. In particular embodiments, the spiroheterocyclyl contains 5 to 20 or 6 to 14 ring atoms. In one embodiment, it contains 7 to 11 (e.g., 7, 8, 9, 10, or 11) ring atoms. According to the number of shared spiro atoms between the rings, the spiroheterocyclyl is divided into monospiroheterocyclyl, bispiroheterocyclyl or polyspiroheterocyclyl, preferably monospiroheterocyclyl and bispiroheterocyclyl. In one embodiment, the spiroheterocyclyl is 4-membered/4-membered, 4-membered/5-membered, 4-membered/6-membered, 5-membered/5-membered, or 5-membered/6-membered monospiroheterocyclyl. In one embodiment, the spiroheterocyclyl is optionally substituted spiroheterocyclyl as described elsewhere herein. Non-limiting examples of spiroheterocyclyl include:

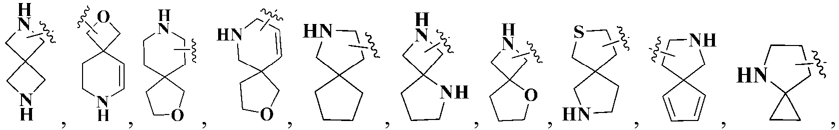

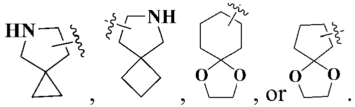

The term "fused heterocyclyl" refers to a polycyclic heterocyclic group with each ring in the system sharing a pair of adjacent atoms with another ring in the system, and one or more rings may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the fused heterocyclyl contains 5 to 20 or 6 to 14 ring atoms, and in one embodiment, the fused heterocyclyl contains 7 to 10 (e.g., 7, 8, 9, or 10) ring atoms. According to the number of constituent rings, the fused heterocyclyl can be divided into bicyclic, tricyclic, tetracyclic, or polycyclic fused heterocyclyl, preferably bicyclic or tricyclic. In one embodiment, the fused heterocyclyl is 5-membered/5-membered or 5-membered/6-membered bicyclic fused heterocyclyl. In one embodiment, the fused heterocyclyl is optionally substituted fused heterocyclyl as described elsewhere herein, or fused heterocyclyl that can be fused to cycloalkyl, heterocyclyl, aryl, or heteroaryl. Non-limiting examples of fused heterocyclyl include:

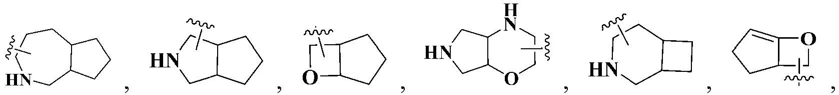

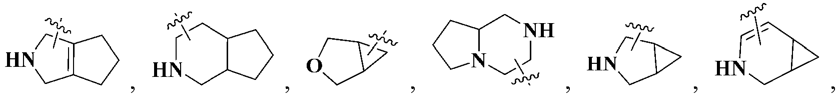

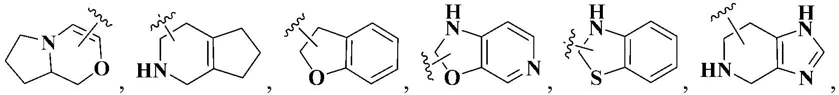

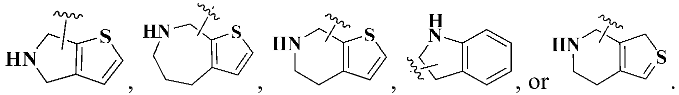

The term "bridged heterocyclyl" refers to a polycyclic heterocyclic group with any two rings sharing two atoms that are not directly connected. The bridged heterocyclyl may contain one or more double bonds, but no ring has a completely conjugated π electron system, wherein one or more ring atoms are heteroatoms selected from nitrogen, oxygen, boron, phosphorus or sulphur, and the remaining ring atoms are carbon atoms. In particular embodiments, the bridged heterocyclyl contains 5 to 20 or 6 to 14 ring atoms. In one embodiment, it contains 7 to 10 (e.g., 7, 8, 9, or 10) ring atoms. According to the number of constituent rings, the bridged heterocyclyl can be divided into bicyclic, tricyclic, tetracyclic, or polycyclic bridged heterocyclyl, preferably bicyclic, tricyclic or tetracyclic bridged heterocyclyl. In one embodiment, the bridged heterocyclyl is bicyclic or tricyclic. In one embodiment, the bridged heterocyclyl is optionally substituted bridged heterocyclyl as described elsewhere herein. Non-limiting examples of bridged heterocyclyl include: 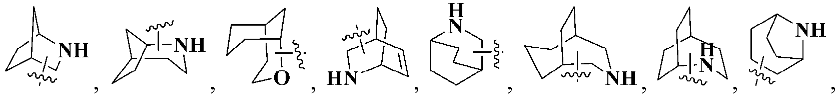 or

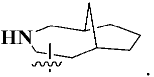

The term "aryl" refers to an all-carbon monocyclic or fused polycyclic (i.e., rings sharing a pair of adjacent carbon atoms) group containing at least one conjugated π electron system, which may be optionally substituted with one or more substituents. In particular embodiments, the aryl contains 6 to 20, 6 to 14, or 6 to 10 ring atoms. In one embodiment, the aryl may further refer to a bicyclic, tricyclic or tetracyclic ring system, in which at least one ring is an aromatic ring and the other rings may be saturated or partially unsaturated carbocyclic rings or rings containing one or more heteroatoms independently selected from O, S and N. In one embodiment, the aryl is selected from benzo 5- to 10-membered heteroaryl, benzo 3- to 10-membered cycloalkyl or benzo 3- to 10-membered heterocyclyl. In one embodiment, the aryl is selected from benzo 5- or 6-membered heteroaryl, benzo 3-to 6-membered cycloalkyl or benzo 3- to 6-membered heterocyclyl, wherein the heterocyclyl is heterocyclyl containing 1 to 3 nitrogen atoms, oxygen atoms or sulphur atoms. Non-limiting examples thereof include phenyl, naphthyl, fluorenyl, azulenyl, anthryl, phenanthrenyl, pyrenyl, biphenyl, terphenyl, dihydronaphthyl, indenyl, tetrahydronaphthyl (tetralinyl), 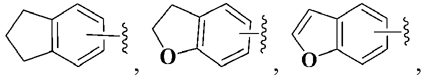 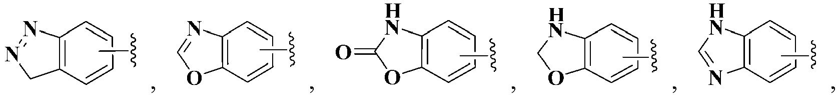 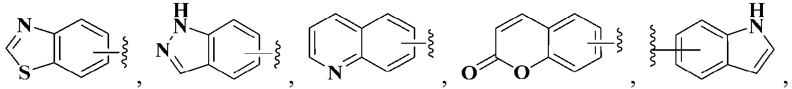 and

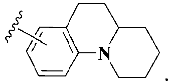

The term "arylene" refers to divalent aryl formed by further substituting one hydrogen atom on aryl, wherein the arylene is optionally substituted or unsubstituted, and the aryl is as defined above.

The term "heteroaryl" refers to an optionally substituted monocyclic or polycyclic group or ring system containing at least one aromatic ring, wherein the aromatic ring has one or more heteroatoms independently selected from O, S and N. In particular embodiments, the heteroaryl contains 5 to 20, 5 to 14, or 5 to 10 ring atoms, of which 1 to 4 are heteroatoms. In one embodiment, the heteroaryl contains 5 or 6 ring atoms. In particular embodiments, the heteroaryl may further refer to a bicyclic, tricyclic or tetracyclic ring, in which at least one ring is an aromatic ring having one or more heteroatoms independently selected from O, S and N, and the other rings may be saturated or partially unsaturated carbocyclic rings or rings containing one or more heteroatoms independently selected from O, S and N. In one embodiment, the heteroaryl is selected from heteroaryl fused 6- to 10-membered aryl, heteroaryl fused 3- to 10-membered cycloalkyl, or heteroaryl fused 3- to 10-membered heterocyclyl. Further in one embodiment, the heteroaryl is selected from 5- or 6-membered heteroaryl fused 6- to 10-membered aryl, 5- or 6-membered heteroaryl fused 3- to 6-membered cycloalkyl, or 5- or 6-membered heteroaryl fused 3- to 6-membered heterocyclyl, wherein the heterocyclyl is heterocyclyl containing 1 to 3 nitrogen atoms, oxygen atoms or sulphur atoms. Non-limiting examples thereof include: furyl, imidazolyl, isothiazolyl, isoxazolyl, oxadiazolyl, oxazolyl, pyrazinyl, pyrazolyl, pyridazinyl, pyridyl, pyrimidyl, pyrrolyl, thiadiazolyl, thiazolyl, thienyl, tetrazolyl, triazinyl, triazolyl, benzofuryl, benzoimidazolyl, benzisoxazolyl, benzopyranyl, benzothiadiazolyl, benzothienyl, benzophenylthio, benzothienyl, benzotriazolyl, imidazopyridyl, imidazothiazolyl, indolizinyl, indolyl, indazolyl, isobenzofuryl, isobenzothienyl, isoindolyl, isoquinolyl, naphthyridinyl, oxazolopyridyl, phthalazinyl, pteridyl, purinyl, pyridopyridyl, pyrrolopyridyl, quinolyl, quinoxalinyl, quinazolinyl, thiadiazolopyrimidyl, thienopyridyl, acridyl, benzoindolyl, carbazolyl, dibenzofuryl, phenanthrolinyl, phenanthridinyl, phenarsazinyl, phenazinyl, phenothiazinyl, phenoxazinyl, xanthenyl,

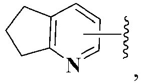

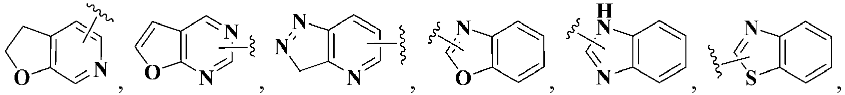

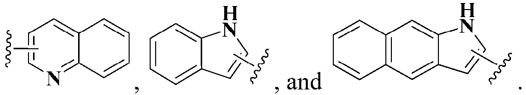

The term "heteroarylene" refers to divalent heteroaryl formed by further substituting one hydrogen atom on cycloalkyl, wherein the heteroarylene is optionally substituted or unsubstituted, and the heteroaryl is as defined above.

The term "heteroalkyl" refers to a stable linear or branched, or cyclic hydrocarbon group, or a combination thereof, and is composed of the indicated number of carbon atoms and one or more (in one embodiment, one to three) heteroatoms selected from O, N, Si, and S, wherein the nitrogen and sulphur atoms are optionally oxidized and the nitrogen heteroatom may be optionally quaternized. In one embodiment, the heteroatoms O, N and S may be placed at any interior position of the heteroalkyl group. In one embodiment, the heteroatom Si may be placed at any position (e.g., interior or terminal position) of the heteroalkyl group, including the position where the alkyl group is connected to the rest of the molecule. Non-limiting examples thereof include: -CH₂-CH₂-O-CH₃, -CH₂-CH₂-NH-CH₃, - CH₂-CH₂-N (CH₃)-CH₃, -CH₂-S-CH₂-CH₃, -CH₂-CH₂-S(O)-CH₃, -CH₂-CH₂-S(O)₂-CH₃, -CH=CH-O-CH₃, -Si (CH₃)₃, -CH₂-CH=N-OCH₃, and-CH=CH-N (CH₃)-CH₃. Up to two heteroatoms can be consecutive, for example, -CH₂-NH-O-CH₃ and -CH₂-O-Si(CH₃)₃. In particular embodiments, the heteroalkyl is optionally substituted heteroalkyl as described elsewhere herein.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentyloxy, or cyclohexyloxy. In one embodiment, the alkoxy is optionally substituted alkoxy as described elsewhere herein.

The term "alkylacyl" refers to -C(O)-alkyl, wherein the alkyl is as defined above.

The term "haloalkyl" refers to alkyl substituted with one or more halogen, wherein the alkyl is as defined above. Non-limiting examples of haloalkyl include: trifluoromethyl, -CH₂CF₃,

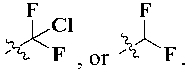

The term "haloalkoxy" refers to alkoxy substituted with one or more halogen, wherein the alkoxy is as defined above.

The term "hydroxyalkyl" refers to alkyl substituted with hydroxyl, wherein the alkyl is as defined above.

The term "alkylthio" refers to -S-(alkyl) and -S-(unsubstituted cycloalkyl), wherein the alkyl or cycloalkyl is as defined above. Non-limiting examples of alkylthio include: methylthio, ethylthio, propylthio, butylthio, cyclopropylthio, cyclobutylthio, cyclopentylthio, or cyclohexylthio. In one embodiment, the alkylthio is optionally substituted alkylthio as described elsewhere herein.

The term "haloalkylthio" refers to alkylthio substituted with one or more halogen, wherein the alkylthio is as defined above.

The term "alkenylcarbonyl" refers to -C(O)-(alkenyl), wherein the alkenyl is as defined above. Non-limiting examples of alkenylcarbonyl include: ethenylcarbonyl, propenylcarbonyl, or butenylcarbonyl. In one embodiment, the alkenylcarbonyl is optionally substituted alkenylcarbonyl as described elsewhere herein.

The term "aminocarbonyl" refers to NH₂-C(O)-.

The term "alkylaminocarbonyl" refers to aminocarbonyl (NH₂-C(O)-) in which one or both of the two hydrogens is substituted with alkyl, wherein the alkyl is as described above.

The term "alkylamino" refers to amino in which one or both of the two hydrogens are substituted with alkyl, wherein the alkyl is as defined above.

The term "carbonyl" refers to a -C(O)-, -(CO)- or -C(=O)- group. All the symbols are used interchangeably in the description.

The term "halogen" refers to fluorine, chlorine, bromine, or iodine.

The term "oxo" or "side oxygen" refers to =O.

The term "hydrogen" includes proton (¹H), deuterium (²H), tritium (³H) and/or a mixture thereof. In particular embodiments, one or more hydrogen-occupied positions in the compound may be enriched with deuterium and/or tritium. Such isotopically enriched analogues may be prepared from suitable isotopically labelled starting materials obtained from commercial sources or by known literature procedures. The hydrogen or hydrogen atom described in the patent comprises isotopes thereof, including hydrogen (¹H), deuterium (²H), tritium (³H) and/or a mixture thereof.

The alkyl, alkylene, alkenyl, alkynyl, cycloalkyl, heterocyclyl, aryl, arylene, heteroaryl, heteroarylene, heteroalkyl, alkoxy, alkylthio, hydroxyalkyl, alkenylcarbonyl, aminocarbonyl, alkylaminocarbonyl, alkylamino, or alkylacyl may be substituted or unsubstituted. In one embodiment, the substituent is selected from one or more of the following groups: alkyl, alkenyl, alkynyl, alkoxy, alkylthio, alkylamino, alkylacyl, halogen, mercapto, hydroxyl, nitro, cyano, azidyl, oximido, phosphate group, oxo, thio, carboxyl, carboxylate group, cycloalkyl, heterocyclyl, aryl, heteroaryl, heterocycloalkoxy, cycloalkylthio, or heterocycloalkylthio.

Different expressions such as "X is selected from A, B, or C", "X is selected from A, B, and C", "X is A, B, or C", and "X is A, B, and C" all express the same meaning, that is, X can be any one or more of A, B, and C.

"Optional" or "optionally" means that the event or circumstance subsequently described may but not necessarily occur, and the description includes the occasion where the event or circumstance occurs or does not occur. For example, "heterocyclic group optionally substituted with alkyl" means that the alkyl may but need not be present, the description includes the case where the heterocyclic group is substituted with alkyl and the case where the heterocyclic group is not substituted with alkyl.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

The term "substituted" means that any one or more hydrogen atoms on the designated atom are substituted with a substituent, provided that the valence state of the designated atom is normal, and the substituted compound is stable in one embodiment. When the substituent is oxo (i.e., =O), it means that two hydrogen atoms are substituted. The term "optionally substituted" means that a group may or may not be substituted. Unless otherwise specified, the type and number of substituents may be arbitrary on the basis of chemical practicability. It goes without saying, the substituents may be only in possible chemical positions thereof, and those skilled in the art can determine the possible or impossible substitutions (by experiment or theory) without paying too much effort. For example, an amino or hydroxyl group with free hydrogen may be unstable when attached to a carbon atom with an unsaturated (e.g., olefinic) bond.

Unless stated to the contrary, the indefinite articles "a" and "an" and the definite article "the" in the present description and claims include plural as well as singular forms.

"Oxo derivative" refers to a compound in which the nitrogen atom in its structure is further substituted with -OH or -OR substituent, in which R is alkyl, alkenyl, alkynyl, cycloalkyl, heterocyclyl, aryl, heteroaryl, C(O)Rₓ, -C(O)NRₓR_{y}, - S(O)₂Rₓ, or -S(O)₂NRₓR_{y};

Rₓ and R_{y} are each independently selected from the following groups: alkyl, alkenyl, alkynyl, alkoxy, alkylthio, alkylamino, alkylacyl, halogen, mercapto, hydroxyl, nitro, cyano, azidyl, oximido, phosphate group, oxo, thio, carboxyl, carboxylate group, cycloalkyl, heterocyclyl, aryl, heteroaryl, heterocycloalkoxy, cycloalkylthio, or heterocycloalkylthio.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

The "pharmaceutically acceptable salt" refers to salts of the compound of the present invention, which are safe and effective when used in mammals, and have appropriate biological activity.

The "stereoisomer" comprises all enantiomerically/diastereomerically/stereoisomerically pure and enantiomerically/diastereomerically/stereoisomerically enriched compounds of the present invention.

"Stereoisomerically pure" means a composition comprising one stereoisomer of a compound and being substantially free of another stereoisomer of the compound. For example, a stereoisomerically pure composition of a compound having one chiral center will be substantially free of the opposite enantiomer of the compound. A stereoisomerically pure composition of a compound having two chiral centers will be substantially free of other diastereomers of the compound. A typical stereoisomerically pure compound comprises greater than about 80% by weight of one stereoisomer of the compound and less than about 20% by weight of another stereoisomer of the compound, greater than about 90% by weight of one stereoisomer of the compound and less than about 10% by weight of another stereoisomer of the compound, greater than about 95% by weight of one stereoisomer of the compound and less than about 5% by weight of another stereoisomer of the compound, greater than about 97% by weight of one stereoisomer of the compound and less than about 3% by weight of another stereoisomer of the compound, or greater than about 99% by weight of one stereoisomer of the compound and less than about 1% by weight of another stereoisomer of the compound.

"Stereoisomerically enriched" means a composition comprising greater than about 55% by weight, greater than about 60% by weight, greater than about 70% by weight, or greater than about 80% by weight of one stereoisomer of a compound.

"Enantiomerically pure" means a stereoisomerically pure composition of a compound having one chiral centre. Similarly, the term "enantiomerically enriched" refers to a stereomerically enriched composition of a compound having one chiral centre.

"Optical activity" and "enantiomeric activity" mean a combination of molecules having an enantiomeric or diastereomeric excess of no less than about 50%, no less than about 70%, no less than about 80%, no less than about 90%, no less than about 91%, no less than about 92%, no less than about 93%, no less than about 94%, no less than about 95%, no less than about 96%, no less than about 97%, no less than about 98%, no less than about 99%, no less than about 99.5%, or no less than about 99.8%. In particular embodiments, the compound comprises about 95% or more of the desired enantiomer or diastereomer and about 5% or less of the less preferred enantiomer or diastereomer, based on the total weight of the racemate.

When describing optically active compounds, the prefixes R and S are used to indicate the absolute configuration of the molecule relative to its chiral centre. (+) and (-) are used to represent the optical rotation of the compound, that is, the direction of the plane of polarized light rotated by the optically active compound. The prefix (-) indicates that the compound is laevorotatory, that is, the compound rotates the plane of polarized light to the left or counterclockwise. The prefix (+) indicates that the compound is dextrorotatory, that is, the compound rotates the plane of polarized light to the right or clockwise. However, the signs of optical rotation (+) and (-) are independent of the absolute configurations R and S of the molecule.

Compounds of the present invention comprise all of their "stereoisomers", "stereoisomerically pure compounds", "enantiomerically enriched compounds", "enantiomerically pure compounds", "optically active compounds", "enantiomerically active compounds", and "optical isomers".

### Detailed Description of Embodiments

The present invention will be further described below in conjunction with examples, but these examples are not meant to limit the scope of the present invention.

### Examples

The structures of the compounds of the present invention are determined by nuclear magnetic resonance (NMR) or/and mass spectrometry (MS). The NMR shift (δ) is given in 10⁻⁶ (ppm). NMR is determined using a Bruker AVANCE-400 nuclear magnetic instrument. The solvents for determination are deuterated dimethyl sulphoxide (DMSO-*d₆*), deuterated chloroform (CDCl₃), and deuterated methanol (CD₃OD), and the internal standard is tetramethylsilane (TMS).

MS is determined with a FINNIGAN LCQAd (ESI) mass spectrometer (manufacturer: Thermo, model: Finnigan LCQ advantage MAX).

HPLC is determined with Agilent 1200DAD high-pressure liquid chromatograph instrument (Sunfire C₁₈ 150 × 4.6 mm chromatographic column) and Waters 2695-2996 high-pressure liquid chromatograph instrument (Gimini C₁₈ 150 × 4.6 mm chromatographic column).

The average kinase inhibition rate and IC₅₀ value are determined with NovoStar microplate reader (BMG Company, Germany).

For thin layer chromatography (TLC), silica gel plates from Yantai Huanghai HSGF254 or Qingdao GF254 are used. Silica gel plates with a thickness of 0.15 mm-0.2 mm are employed for TLC, while plates with a thickness of 0.4 mm-0.5 mm are used for TLC separation and purification.

For column chromatography, Yantai Huanghai silica gel of 200-300 mesh is typically used as a carrier.

The known starting materials of the present invention can be synthesized by methods known in the art, or can be purchased from ABCR GmbH & Co. KG, Acros Organics, Aldrich Chemical Company, Accela ChemBio Inc., Chembee Chemicals Inc. and other companies.

Unless otherwise specially specified in examples, reactions can all be carried out under argon atmosphere or nitrogen atmosphere.

Argon atmosphere or nitrogen atmosphere means that a reaction flask is connected to an argon or nitrogen balloon with a volume of about 1 L.

Hydrogen atmosphere means that a reaction flask is connected to a hydrogen balloon with a volume of about 1 L.

Pressurized hydrogenation reactions are performed using either Parr 3916EKX hydrogenation apparatus with Qinglan QL-500 hydrogen generator or HC2-SS hydrogenation apparatus.

Hydrogenation reactions are typically performed by evacuating the system and refilling it with hydrogen gas, and repeating this operation three times.

Microwave reactions are performed using CEM Discover-S 908860 microwave reactor.

Unless otherwise specially specified in examples, a solution refers to an aqueous solution.

Unless otherwise specially specified in examples, the reaction temperature is room temperature, which is 20°C to 30°C.

The progress of a reaction in the examples is monitored by thin layer chromatography (TLC). Developing agent systems used in the reaction include: A: dichloromethane and methanol system, B: n-hexane and ethyl acetate system, C: petroleum ether and ethyl acetate system, D: acetone, the volume ratio of the solvents is adjusted according to the polarity of the compounds.

Eluent systems for column chromatography and developing agent systems for thin layer chromatography, which are used to purify compounds, include: A: n-hexane and ethyl acetate system, B: n-hexane and tetrahydrofuran system, the volume ratio of the solvents is adjusted according to the polarity of the compounds, and a small amount of basic or acidic reagents such as triethylamine and acetic acid can also be added for adjustment.

### Example 1

### Preparation of (5aS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5a,6,7,8,9,10-hexahydro-5H-4-oxa-3,10a,11,13,14-pentaaza-6,9-methylnaphtho[1,8-ab]heptan-14-ol

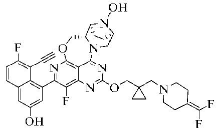

### Step 1: Preparation of 2,4,5,7-tetrachloro-8-fluoropyrido[4,3-d]pyrimidine

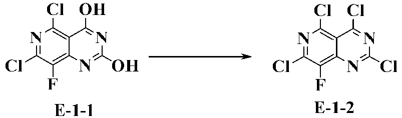

5,7-Dichloro-8-fluoropyrido[4,3-d]pyrimidine-2,4-diphenol (1.1 g, 4.40 mmol) was added to POCl₃ (20 mL), and the resulting mixture was heated to reflux and reacted for 16 hours, and concentrated under reduced pressure to obtain crude compound E-1-2 (1.4 g). MS m/z (ESI):288 [M+H].

### Step 2: Preparation of tert-butyl (1S,2S,5R)-2-(((tert-butyldiphenylsilyl)oxo)methyl)-3-(2,5,7-trichloro-8-fluoropyrido[4,3-d]pyrimidin-4-yl)-3,8-diazabicyclo[3.2.1]octane-8-carboxylate

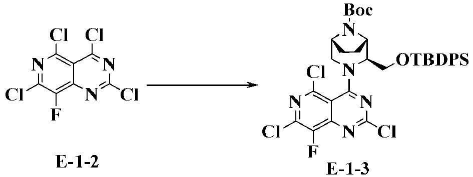

E-1-2 (1.3 g) was added to dichloromethane (20 mL), and tert-butyl (1S,2S,5R)-2-(((tert-butyldiphenylsilyl)oxo)methyl)-3,8-diazabicyclo[3.2.1]octane-8-carboxylate (2.4 g, 4.99 mmol) and triethylamine (1.4 g, 13.84 mmol) were added. The resulting mixture was reacted at room temperature for 3 hours. Water was added and the resulting mixture was extracted with dichloromethane. The organic phases were combined, washed with saturated sodium chloride aqueous solution, dried over anhydrous sodium sulphate, and concentrated under reduced pressure. The residue was purified using a silica gel chromatographic column to obtain E-1-3 (2.8 g).

### Step 3: Preparation of tert-butyl (1S,2S,5R)-2-(((tert-butyldiphenylsilyl)oxo)methyl)-3-(2-((1-(((tert-butyldiphenylsilyl)oxo)methyl)cyclopropyl)methoxy)-5,7-dichloro-8-fluoropyrido[4,3-d]pyrimidin-4-yl)-3,8-diazabicyclo[3.2.1]octane-8-carboxylate

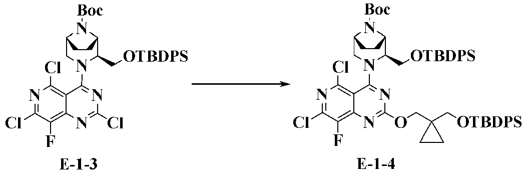

At 0°C, to a solution of E-1-3 (235.7 mg, 692.05 µmol) in THF (15 mL) was added NaH (27.68 mg, 692.05 µmol, 60% purity). After the addition was completed, the resulting mixture was warmed to room temperature and stirred for 15 hours. The reaction mixture was quenched with ammonium chloride aqueous solution and extracted with ethyl acetate. The organic phase was washed with saturated sodium chloride aqueous solution, dried over anhydrous sodium sulphate and concentrated to obtain E-1-4 (622 mg, yield: 99.8%).

### Step 4: Preparation of tert-butyl (5aS,6S,9R)-2-chloro-1-fluoro-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

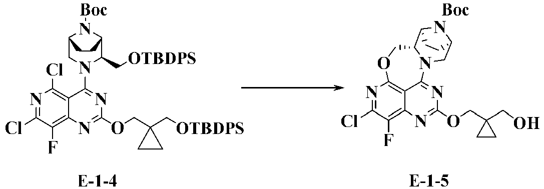

At room temperature, to a solution of E-1-4 (622 mg, 600.83 µmol) in THF (20 mL) was added TBAF (1 M, 3.61 mL), and the resulting mixture was stirred at room temperature for 1 hour. Water was added, and the resulting mixture was extracted with ethyl acetate. The organic phase was washed with saturated sodium chloride aqueous solution, dried over anhydrous sodium sulphate and concentrated. The residue was purified using a silica gel chromatographic column to obtain E-1-5 (210 mg, yield: 66.9%). MS m/z (ESI):522 [M+H].

### Step 5: Preparation of tert-butyl (5aS,6S,9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

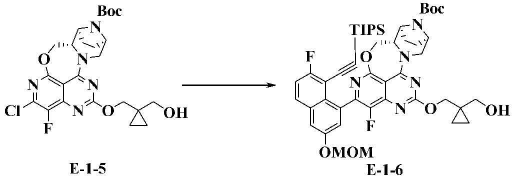

E-1-5 (210 mg, 402.32 µmol), 2-[2-fluoro-6-(methoxymethoxy)-8-(4,4,5,5-tetramethyl-1,3,2-dioxaborolan-2-yl)-1-naphthyl]ethynyl-triisopropyl-silane (14.65 mg, 20.12 µmol), and Cs₂CO₃ (262.17 mg, 804.65 µmol) were added to dioxane (5 mL), and the resulting mixture was heated to 100°C and stirred for 13 hours under a nitrogen atmosphere. Water was added and the resulting mixture was extracted with ethyl acetate. The organic phase was washed with saturated sodium chloride aqueous solution, dried over anhydrous sodium sulphate and concentrated to obtain E-1-6 (350 mg, yield: 99.8%).

### Step 6: Preparation of tert-butyl (5aS,6S,9R)-2-(8-ethynyl-7-fluoro-3-(methoxymethoxy)naphthalen-1-yl)-1-fluoro-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

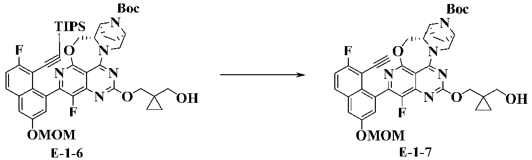

At room temperature, to a solution of E-1-6 (350 mg, 401.34 µmol) in THF (10 mL) was added TBAF (1 M, 1.20 mL). After the addition was completed, the resulting mixture was stirred at room temperature for 1 hour. Water was added, and the resulting mixture was extracted with ethyl acetate. The organic phase was washed with saturated sodium chloride aqueous solution, dried over anhydrous sodium sulphate and concentrated. The residue was purified by a preparative silica gel plate to obtain E-1-7 (120 mg, yield: 41.8%). MS m/z (ESI): 716 [M+H].

### Step 7: Preparation of tert-butyl (5aS,6S,9R)-2-(8-ethynyl-7-fluoro-3-(methoxymethoxy)naphthyl-1-yl)-1-fluoro-12-((1-(((methylsulphonyl)oxo)methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

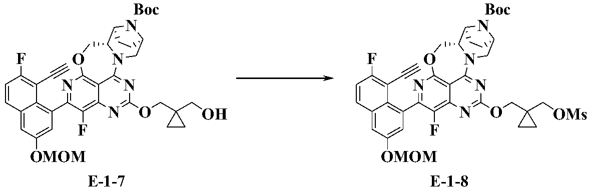

At 0°C, to a solution of E-1-7 (100 mg, 139.72 µmol) in dichloromethane (15 mL) was added triethylamine (42.41 mg, 419.15 µmol, 58.46 µL), followed by methanesulphonyl chloride (32.01 mg, 279.43 µmol), and the resulting mixture was stirred at 0°C for 30 minutes. Sodium bicarbonate aqueous solution was added and the resulting mixture was extracted with dichloromethane. The organic phase was washed with ammonium chloride aqueous solution, then washed with saturated sodium chloride aqueous solution, dried over anhydrous sodium sulphate and concentrated to obtain E-1-8 (110 mg, yield: 99.2%), MS m/z (ESI):794 [M+H].

### Step 8: Preparation of tert-butyl (5aS,6S,9R)-12-((1-((4-(difluoromethylene)piperidin-1-yl)methyl)cyclopropyl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate

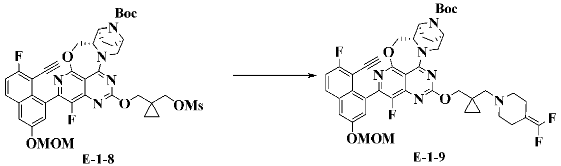

E-1-8 (110 mg, 138.57 µmol), 4-(difluoromethylene)piperidine (55.35 mg, 415.71 µmol), DIPEA (179.09 mg, 1.39 mmol), and NaI (62.31 mg, 415.71 µmol) were added to acetonitrile (8 mL), and the resulting mixture was heated to 50°C and stirred for 13 hours. To the reaction solution was added ammonium chloride aqueous solution. The resulting mixture was extracted with ethyl acetate, and the organic phase was washed with saturated sodium chloride aqueous solution, dried over anhydrous sodium sulphate and concentrated. The residue was purified by a preparative silica gel plate to obtain E-1-9 (70 mg, yield: 60.8%). MS m/z (ESI): 831 [M+H].

### Step 9: Preparation of 4-((5aS,6S,9R)-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylenenaphtho[1,8-ab]heptalen-2-yl)-5-ethynyl-6-fluoronaphthalen-2-ol

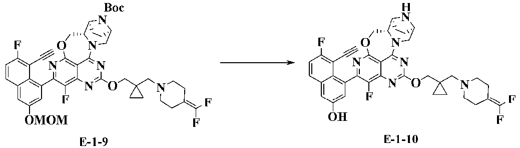

At 0°C, to a solution of E-1-9 (70 mg, 84.25 µmol) in dichloromethane (3 mL) and methanol (0.5 mL) was added HCl (3.07 mg, 84.25 µmol, 3 mL). After the addition was completed, the resulting mixture was stirred at room temperature for 30 minutes. The reaction solution was slowly added to sodium bicarbonate aqueous solution and the resulting mixture was extracted with dichloromethane/methanol. The organic phase was washed with saturated sodium chloride aqueous solution, dried over anhydrous sodium sulphate and concentrated. The residue was purified by preparative HPLC to obtain E-1-10 (30 mg, yield: 51.8%). MS m/z (ESI): 687 [M+H].

### Step 10: Preparation of (5aS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5a,6,7,8,9,10-hexahydro-5H-4-oxa-3,10a,11,13,14-pentaaza-6,9-methylnaphtho[1,8-ab]heptan-14-yl benzoate

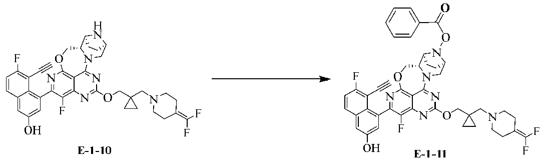

At room temperature, to a solution of E-1-10 (50 mg, 0.07 mmol) and potassium hydrogen phosphate (38 mg, 0.22 mmol) in N,N-dimethylformamide (1.5 mL) was added benzoyl peroxide (35 mg, 0.14 mmol). After the addition was completed, the resulting mixture was stirred at room temperature for 3 hours. The reaction solution was quenched with water and extracted with ethyl acetate. The organic phase was washed with ammonium chloride aqueous solution and saturated sodium chloride aqueous solution, dried over anhydrous sodium sulphate, filtered and concentrated to obtain E-1-11 (54 mg, yield: 92%). MS m/z (ESI): 807 [M+H].

### Step 11: Preparation of (5aS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5a,6,7,8,9,10-hexahydro-5H-4-oxa-3,10a,11,13,14-pentaaza-6,9-methylnaphtho[1,8-ab]heptan-14-ol

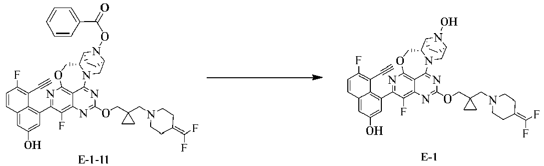

E-1-11 (54 mg, 0.067 mmol) and potassium carbonate (28 mg, 0.2 mmol) were added to methanol (5 mL), and the resulting mixture was stirred at room temperature for 1 hour. The reaction solution was filtered and concentrated. The residue was separated and purified by high-performance liquid chromatography and lyophilized to obtain E-1 (18 mg, yield: 39%). MS m/z (ESI): 703 [M+H]

¹H NMR (400 MHz, MeOD) δ 7.88 - 7.78 (m, 1H), 7.34 - 7.14 (m, 3H), 5.18 - 5.05 (m, 1H), 4.72 - 4.59 (m, 1H), 4.56 - 4.32 (m, 4H), 4.22 (d, *J* = 8 Hz, 1H), 3.95 - 3.79 (m, 2H), 2.98 - 2.56 (m, 6H), 2.35 (s, 4H), 2.11 - 1.75 (m, 5H), 0.83 (s, 2H), 0.64 (s, 2H).

### Example 17

### Preparation of (5S,5aS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylenenaphtho[1,8-ab]heptalen-14-ol

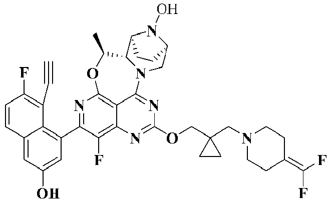

### Step 1: Preparation of tert-butyl (1S,2S,5R)-2-[(1S)-1-(7-chloro-8-fluoro-4-hydroxyl-2-methylsulphanyl-pyrido[4,3-d]pyrimidin-5-yl)oxy-ethyl]-3,8-diazabicyclo[3.2.1]octane-8-carboxylate

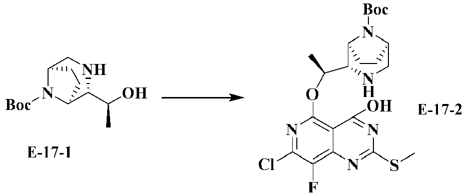

5,7-Dichloro-8-fluoro-2-methylsulphanyl-pyrido[4,3-d]pyrimidin-4-ol (5 g, 17.9 mmol) was dissolved in tetrahydrofuran (100 mL) and tert-butyl (1S,2S,5R)-2-(1-hydroxyethyl)-3,8-diazabicyclo[3.2.1]octane-8-carboxylate (4.3 g, 16.8 mmol) was added. The resulting mixture was cooled to 0°C and sodium hydride (2.3 g, 57.5 mmol, purity: 60%) was added. After the addition was completed, the resulting mixture was stirred at room temperature for 1 hour. The reaction solution was slowly poured into 10% ammonium chloride aqueous solution (100 mL) and ethyl acetate (100 mL). A solid precipitated out and was filtered, washed with water and dried to obtain E-17-2 (5.2 g, yield: 58.2%). MS m/z (ESI): 500 [M+H].

### Step 2: Preparation of tert-butyl (5S,5aS,6R,9S)-2-chloro-1-fluoro-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate

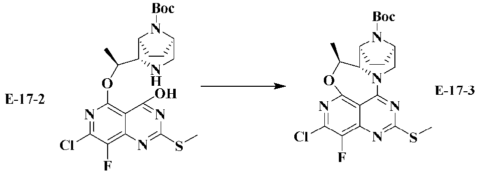

At 0°C, to a solution of E-17-2 (5.1 g, 10.2 mmol) in dichloromethane (250 mL) was added N,N-diisopropylethylamine (6.5 g, 50.3 mmol). Then phosphorus oxychloride (3.7 g, 24.1 mmol) was added dropwise. After the addition was completed, the resulting mixture was stirred at room temperature for 1 hour. The reaction solution was poured into sodium bicarbonate aqueous solution and the resulting mixture was extracted with dichloromethane. The organic phase was washed with ammonium chloride aqueous solution and saturated sodium chloride aqueous solution, dried over anhydrous sodium sulphate, filtered and concentrated. The residue was purified using a silica gel chromatographic column to obtain E-17-3 (3.9 g, yield: 79.3%). MS m/z (ESI): 482 [M+H].

### Step 3: Preparation of tert-butyl (5S,5aS,6R,9S)-2-chloro-1-fluoro-5-methyl-12-(methylsulphonyl)-5a,6,7,8,9,10-hexahydro-5H-4-oxa-3,10a,11,13,14-pentaaza-6,9-methylnaphtho[1,8-ab]heptene-4-carboxylate

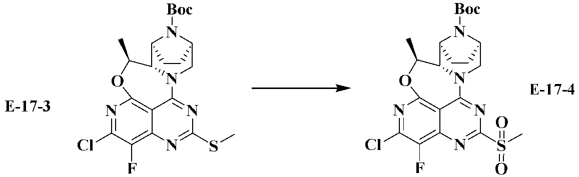

E-17-3 (3.8 g, 7.88 mmol) was dissolved in dichloromethane (100 mL), and the resulting mixture was cooled to 0-5°C. m-Chloroperoxybenzoic acid (4.0 g, 19.7 mmol, purity: 85%) was added. After the addition was completed, the resulting mixture was stirred for 1.5 hours. The reaction solution was filtered and to the filtrate were added sodium thiosulphate aqueous solution (100 mL) and ethyl acetate (100 mL). The resulting mixture was stirred for 30 minutes and subjected to phase separation. The aqueous phase was extracted with ethyl acetate. The organic phases were combined, washed with sodium bicarbonate aqueous solution and saturated sodium chloride aqueous solution, dried over anhydrous sodium sulphate, filtered and concentrated. The residue was purified using a silica gel chromatographic column to obtain E-17-4 (3.3 g, yield: 81.5%). MS m/z (ESI): 514 [M+H].

### Step 4: Preparation of tert-butyl (5S,5aS,6S,9R)-2-chloro-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate

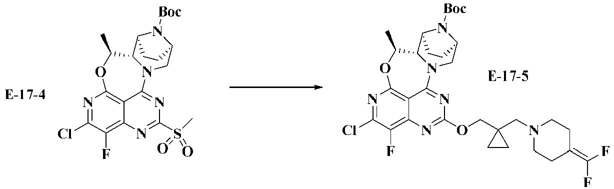

E-17-4 (3.2 g, 6.23 mmol) and [1-[[4-(difluoromethylene)-1-piperidyl]methyl]cyclopropyl]methanol (1.76 g, 8.10 mmol) were dissolved in tetrahydrofuran (70 mL), and the resulting mixture was purged with nitrogen. In an ice bath, lithium bis(trimethylsilyl)amide (1 M, 18.7 mL) was added dropwise, and the resulting mixture was reacted in an ice bath for 30 minutes. The reaction mixture was quenched with saturated ammonium chloride solution, extracted with ethyl acetate, dried over anhydrous sodium sulphate, filtered and concentrated. The residue was purified using a silica gel chromatographic column to obtain E-17-5 (2.7 g, yield: 66.7%). MS m/z (ESI): 651[M+H].

### Step 5: Preparation of tert-butyl (SS,SaS,6S,9R)-12-((1-((4-(difluoromethylene)piperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-14 carboxylate

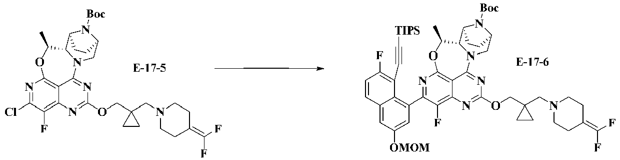

In an egg-shaped bottle, 2-[2-fluoro-6-(methoxymethoxy)-8-(4,4,5,5-tetramethyl-1,3,2-dioxyborolan-2-yl)-1-naphthyl]ethynyltriisopropylsilane (2.05 g, 4.0 mmol), E-17-5 (2.6 g, 3.99 mmol), methanesulphonato[n-butylbis(1-adamantyl)phosphine](2-amino-1,1'-biphenyl-2-yl)palladium (II) (73 mg, 0.1 mmol), and caesium carbonate (3.9 g, 11.97 mmol) were dissolved in mixed solvents of dioxane (30 mL) and water (6 mL). The resulting mixture was purged with nitrogen and reacted in an oil bath at 85°C for 1 hour. Upon completion of the reaction, the reaction mixture was concentrated and the residue was purified using a silica gel chromatographic column to obtain E-17-6 (1.8 g, yield: 57.1%). MS m/z (ESI): 1001[M+H].

### Step 6: Preparation of tert-butyl (5S,5aS,6S,9R)-12-((1-((4-(difluoromethylene)piperidin-1-yl)methyl)cyclopropyl)methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxo-3,10a,11,13,14-pentaaza-6,9-methylnaphtho[1,8-ab]heptene-4-carboxylate

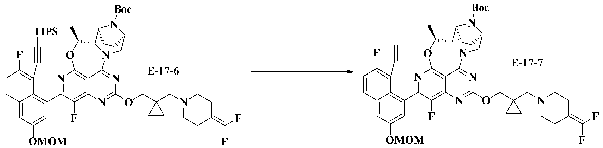

In an egg-shaped bottle, E-17-6 (1.8 g, 1.80 mmol) was dissolved in tetrahydrofuran (120 mL) and tetrabutylammonium fluoride (1 M, 10 mL) was added. The resulting mixture was reacted at room temperature for 1 hour. The reaction solution was concentrated and the residue was purified using a silica gel chromatographic column to obtain E-17-7 (1.4 g, yield: 92.1%). MS m/z (ESI): 845[M+H].

### Step 7: Preparation of 4-((SS,SaS,6S,9R)-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

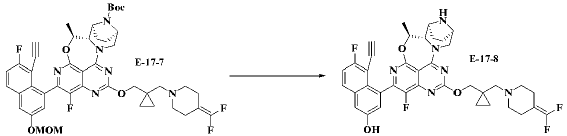

E-17-7 (200 mg, 0.24 mmol) was dissolved in a solution of dichloromethane (20 mL) and methanol (4 mL), and in an ice bath, the mixture was cooled for 10 minutes and purged with nitrogen. 4N hydrogen chloride-dioxane (10 mL) was slowly added and in an ice bath, the resulting mixture was reacted for 1.5 hours. The reaction solution was quenched with sodium bicarbonate solution and extracted with dichloromethane/methanol. The organic phase was dried over anhydrous sodium sulphate, filtered and concentrated. The residue was purified using a silica gel chromatographic column to obtain E-17-8 (140 mg, yield: 84.3%). MS m/z(ESI):701 [M+H].

### Step 8: Preparation of (SS,SaS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylenenaphtho[1,8-ab]heptalen-14-yl benzoate

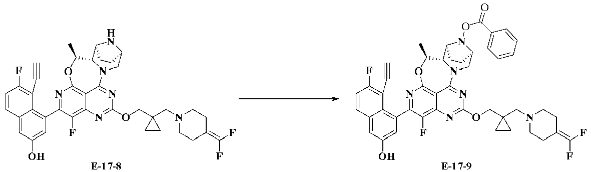

At room temperature, to a solution of E-17-8 (120 mg, 0.17 mmol) and potassium hydrogen phosphate (90 mg, 0.52 mmol) in N,N-dimethylformamide (10 mL) was added benzoyl peroxide (82 mg, 0.34 mmol). After the addition was completed, the resulting mixture was stirred at room temperature for 3 hours. The reaction solution was quenched with water and extracted with ethyl acetate. The organic phase was washed with ammonium chloride aqueous solution and saturated sodium chloride aqueous solution, dried over anhydrous sodium sulphate, filtered and concentrated to obtain E-17-9 (150 mg). MS m/z (ESI): 821 [M+H].

### Step 9: Preparation of (5S,5aS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylenenaphtho[1,8-ab]heptalen-14-ol

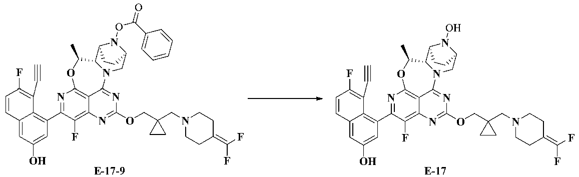

E-17-9 (150 mg) was dissolved in methanol (5 mL), potassium carbonate (70 mg, 0.51 mmol) was added, and the resulting mixture was stirred at room temperature for 1 hour. The reaction solution was filtered and concentrated. The residue was separated and purified by high-performance liquid chromatography and lyophilized to obtain E-17 (27 mg, yield: 22%). MS m/z (ESI): 717 [M+H]

¹H NMR(400 MHz, MeOD)δ 7.89-7.77(m, 1H), 7.37-7.26(m, 2H), 7.25-7.06(m, 1H), 5.46-5.37(m, 1H), 4.63- 4.51(m, 1H), 4.50- 4.29(m, 2H), 4.12(d, *J=* 8.7 Hz, 1H), 3.74-3.64(m, 1H), 3.59(s, 1H), 3.49-3.34(m, 1H), 3.27-3.14(m, 1H), 2.84- 2.53(m, 6H), 2.37-2.23(m, 4H), 2.20-1.87(m, 3H), 1.67-1.50(m, 4H), 0.79(s, 2H), 0.59(s, 2H).

### Alternatively, Examples 21, 33, 102 and 263 were prepared using the following methods:

### Example 21

### Preparation of 4-(difluoromethylene)-1-(1-(((5S,5aR,6S,9R)-2-(8-ethynyl-7-fluoro-3-hydroxylnaphthalen-1-yl)-1-fluoro-5-methyl)-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)piperidine 1-oxide

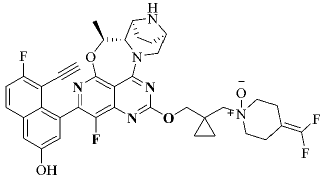

### Step 1: Preparation of compound tert-butyl (SS,SaR,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptane-14 carboxylate

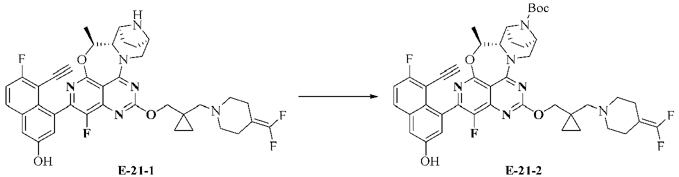

To a solution of 4-((5S,5aS,6S,9R)-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (1 g, 1.43 mmol) in dichloromethane (20 mL) were added triethylamine (433 mg, 4.28 mmol) and di-tert-butyl dicarbonate (467 mg, 2.14 mmol), and the resulting mixture was reacted at room temperature for 1 hour. The reaction mixture was washed with saturated ammonium chloride solution, extracted with dichloromethane and subjected to phase separation. The organic phase was collected, washed with water and saturated sodium chloride aqueous solution, dried over anhydrous sodium sulphate and filtered. The organic phase was concentrated and purified using a silica gel chromatographic column to obtain E-21-2 (1.14 g, yield: 100%). MS m/z (ESI): 801 [M+H].

### Step 2: Preparation of compound 1-(1-(((SS,SaR,6S,9R)-14-(tert-butyloxycarbonyl)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)-4-(difluoromethylene)piperidine 1-oxide

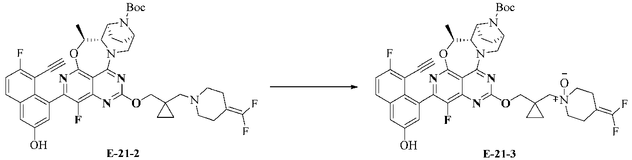

To a solution of E-21-2 (1 g, 1.25 mmol) in dichloromethane (30 mL) was added m-chloroperoxybenzoic acid (323 mg, 1.87 mmol) and the resulting mixture was reacted at room temperature for 1 hour. The reaction solution was washed with sodium thiosulphate solution and the organic phase was dried over anhydrous sodium sulphate, filtered and concentrated to obtain E-21-3 (1.02 g, yield: 100%). MS m/z (ESI): 817 [M+H].

### Step 3: Preparation of compound 4-(difluoromethylene)-1-(1-(((5S,5aR,6S,9R)-2-(8-ethynyl-7-fluoro-3-hydroxylnaphthalen-1-yl)-1-fluoro-5-methyl)-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)piperidine 1-oxide

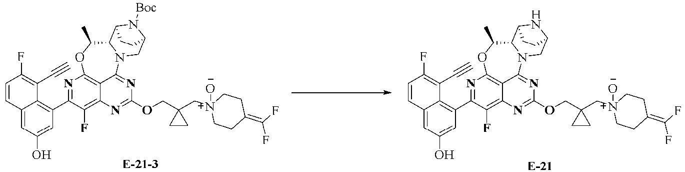

At 0°C, E-21-3 (1 g, 1.22 mmol) was dissolved in a mixture solution of dichloromethane (10 mL) and MeOH (2.5 mL), HCl (4 M, 10 mL) was added, and the resulting mixture was transferred to room temperature and reacted for 1 hour. The reaction solution was concentrated and the residue was purified by a preparative liquid chromatographic column to obtain E-21 (619 mg, yield: 55%). MS m/z (ESI): 717 [M+H].

¹H NMR (400 MHz, MeOD) δ 7.85(dt, *J* = 9.1, 6.1 Hz, 1H), 7.36 - 7.27(m, 2H), 7.17(dd, *J* = 40.4, 2.6 Hz, 1H), 5.55(dt, *J =* 14.8, 3.4 Hz, 1H), 4.73 - 4.57(m, 3H), 4.30 (dd, *J* = 8.8, 4.1 Hz, 1H), 4.19(d, *J* = 5.8 Hz, 1H), 4.09(q, *J =* 5.8 Hz, 1H), 3.79(s, 2H), 3.68(d, *J* = 9.2 Hz, 2H), 3.50 - 3.36(m, 3H), 2.80 - 2.66(m, 2H), 2.52(d, *J* = 15.0 Hz, 2H), 2.35 - 1.91(m, 5H), 1.60(t, *J* = 6.3 Hz, 3H), 1.04 - 0.87(m, 4H).

### Example 33

### Preparation of (5S,5aS,6R,9S)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptan-14-ol

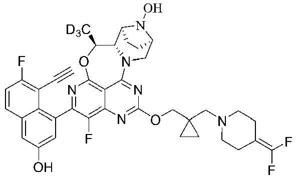

### Step 1: Preparation of compound (SS,SaS,6R,9S)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptan-14-yl benzoate

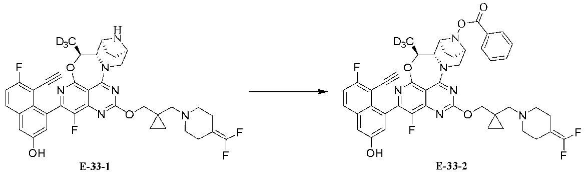

At room temperature, to a solution of E-33-1 (1 g, 1.42 mmol) and potassium hydrogen phosphate (743 mg, 4.26 mmol) in DMF (15 mL) was added benzoyl peroxide (688 mg, 2.84 mmol). After the addition was completed, the resulting mixture was stirred at room temperature for 3 hours. The reaction solution was quenched with water and extracted with ethyl acetate. The organic phase was washed with water and saturated sodium chloride aqueous solution, dried over anhydrous sodium sulphate, filtered and concentrated. The residue was purified using a silica gel chromatographic column to obtain E-33-2 (550 mg, yield: 47.0%). MS m/z(ESI): 824 [M+H].

### Step 2: Preparation of compound (SS,SaS,6R,9S)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptan-14-ol

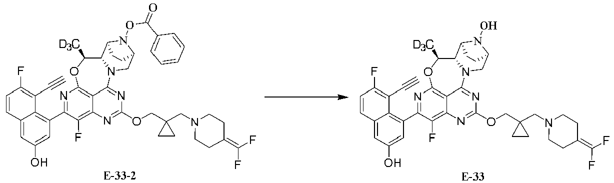

E-33-2 (550 mg, 0.67 mmol) and potassium carbonate (277 mg, 2 mmol) were added to methanol (8 mL), and the resulting mixture was stirred at room temperature for 1 hour. The reaction solution was filtered and concentrated, and the residue was purified by preparative liquid chromatography and lyophilized to obtain E-33 (200 mg, yield: 37.2%). MS m/z(ESI): 720 [M+H].

¹H NMR(400 MHz, MeOD)δ 7.90- 7.77(m, 1H), 7.36 - 7.27(m, 2H), 7.25(d, *J = 2.3* Hz, 0.5H), 7.14(d, *J* = 2.5 Hz, 0.5H), 5.40(dd, *J* = 13.4, 3.4 Hz, 1H), 4.61 - 4.28(m, 3H), 4.12(d, *J =* 8.8 Hz, 1H), 3.74 - 3.63(m, 1H), 3.58(d, *J* = 5.6 Hz, 1H), 3.49 - 3.35(m, 1H), 3.22(t, *J* = 13.0 Hz, 1H), 2.96 - 2.67(m, 6H), 2.35(d, *J* = 5.2 Hz, 4H), 2.22 - 1.80(m, 3H), 1.75 - 1.49(m, 1H), 0.83(d, *J = 4.5* Hz, 2H), 0.64(s, 2H).

### Example 102

### Preparation of 1-((1-((((5S,5aS,6R,9S)-2-(5-amino-3-chloro-2-(trifluoromethyl)phenyl)-1-fluoro-5-methyl-5a,6,7,8,9,10-hexahydro-5H-4-oxa-3,10a,11,13,14-pentaaza-6,9-methylenenaphtho[1,8-ab]heptalen-12-yl)oxo)methyl)cyclopropyl)methyl)piperidin-4-one

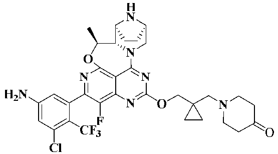

### Step 1: Preparation of tert-butyl (SS,SaS,6R,9S)-12-((1-((((tert-butyldiphenylsilyl)oxy)methyl)cyclopropyl)methoxy)-2-chloro-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate

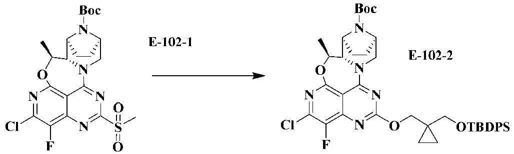

Tert-butyl (5S,5aS,6R,9S)-2-[(1S)-1-(7-chloro-8-fluoro-4-hydroxyl-2-methylsulphanyl-pyrido[4,3-d]pyrimidin-5-yl)oxy-ethyl]-3,8-diazabicyclo[3.2.1]octane-8-carboxylate (2 g, 3.89 mmol) was dissolved in tetrahydrofuran (20 mL), [1-[[tert-butyl(diphenyl)silyl]oxymethyl]cyclopropyl] methanol (1.71 g, 5.03 mmol) was added, and the mixture was cooled to 0°C. Lithium bis(trimethylsilyl)amide (1 M, 11.6 mL) was added dropwise. After the addition was completed, the mixture was reacted at 0°C for 15 minutes. The reaction solution was quenched with ammonium chloride aqueous solution (50 mL) and extracted with ethyl acetate. The organic phase was washed with saturated sodium chloride aqueous solution, dried over anhydrous sodium sulphate, filtered and concentrated. The residue was purified using a silica gel chromatographic column to obtain E-102-2 (2.6 g, yield: 86.4%). MS m/z (ESI): 774 [M+H].

### Step 2: Preparation of tert-butyl (5S,5aS,6R,9S)-2-chloro-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14 carboxylate

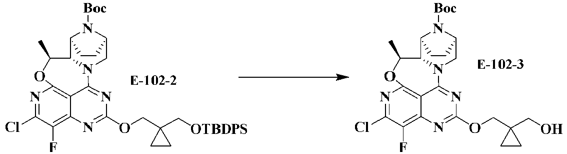

At room temperature, to a solution of E-102-2 (2.5 g, 3.23 mmol) in tetrahydrofuran (30 mL) was added tetrabutylammonium fluoride (1 M, 20 mL). After the addition was completed, the resulting mixture was stirred at room temperature for 1 hour. To the reaction solution was added water. The resulting mixture was extracted with ethyl acetate and the organic phase was washed with water and saturated sodium chloride aqueous solution, dried over anhydrous sodium sulphate, filtered and concentrated. The residue was purified using a silica gel chromatographic column to obtain E-102-3 (2 g, yield: 96%). MS m/z (ESI): 536 [M+H].

### Step 3: Preparation of tert-butyl (5S,5aS,6R,9S)-2-chloro-1-fluoro-5-methyl-12-(1-(((methylsulphonyl)oxy)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate

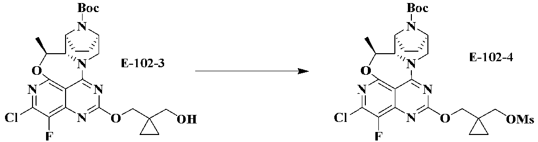

At 0°C, to a solution of E-102-3 (2 g, 3.73 mmol) in dichloromethane (30 mL) were added triethylamine (1.13 g, 11.13 mmol) and methanesulphonyl chloride (850 mg, 7.42 mmol). After the addition was completed, the resulting mixture was stirred at 0°C for 30 minutes. The reaction solution was added to sodium bicarbonate aqueous solution. The resulting mixture was extracted with dichloromethane and the organic phase was washed with ammonium chloride aqueous solution, dried over anhydrous sodium sulphate, filtered and concentrated to obtain E-102-4 (2.1 g, crude). MS m/z (ESI): 614 [M+H].

### Step 4: Preparation of tert-butyl (5S,5aS,6R,9S)-2-chloro-1-fluoro-12-((1-((4-(methoxyimino)piperidine-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14 carboxylate

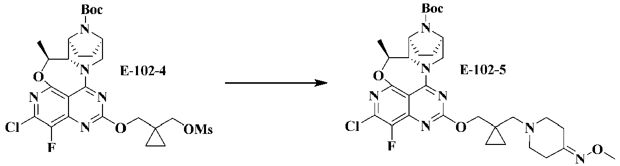

E-102-4 (2.1 g), N-methoxypiperidine-4-imine trifluoroacetate (2.07 g, 8.54 mmol), sodium iodide (1.67 g, 11.13 mmol), and N,N-diisopropylethylamine (4.8 g, 37.1 mmol, 6.46 mL) were added to acetonitrile (40 mL), and the resulting mixture was heated to 50°C and stirred for 13 hours. The reaction mixture was cooled and to the reaction solution was added water. The resulting mixture was extracted with ethyl acetate and the organic phase was dried over anhydrous sodium sulphate, filtered and concentrated. The residue was purified using a silica gel chromatographic column to obtain E-102-5 (1.3 g, two-step yield: 53.9%). MS m/z (ESI): 646 [M+H].

### Step 5: Preparation of tert-butyl (5S,5aS,6R,9S)-2-(5-amino-3-chloro-2-(trifluoromethyl)phenyl)-1-fluoro-12-(1-((4-(methoxyimino)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14 carboxylate

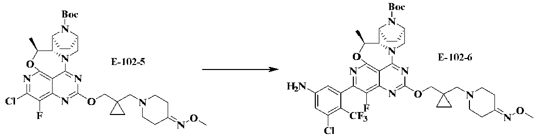

E-102-5 (1.2 g, 1.86 mmol) and methanesulphonato(dimethyl-n-butylphosphino)-2'-amino-1,1'-biphenyl-2-yl)palladium (II) dichloromethane adduct (157 mg, 0.22 mmol) were added to dioxane (20 mL), and the mixture was heated to 80°C and stirred for 0.5 hours. The reaction mixture was cooled to room temperature, 3-chloro-5-(4,4,5,5-tetramethyl-1,3,2-dioxaboran-2-yl)-4-(trifluoromethyl)aniline (1.79 g, 5.57 mmol) and sodium hydroxide aqueous solution (2 M, 4 mL) were added, and the resulting mixture was heated to 70°C and stirred for 3 hours. The reaction mixture was cooled to room temperature, and saturated ammonium chloride aqueous solution was added. The resulting mixture was extracted with ethyl acetate and the organic phase was dried over anhydrous sodium sulphate, filtered and concentrated. The residue was purified using a silica gel chromatographic column to obtain E-102-6 (1.1 g, yield: 73.3%). MS m/z (ESI): 805[M+H].

### Step 6: Preparation of 1-((1-((((5S,5aS,6R,9S)-2-(5-amino-3-chloro-2-(trifluoromethyl)phenyl)-1-fluoro-5-methyl-5a,6,7,8,9,10-hexahydro-5H-4-oxa-3,10a,11,13,14-pentaaza-6,9-methylenenaphtho[1,8-ab]heptalen-12-yl)oxo)methyl)cyclopropyl)methyl)piperidin-4-one

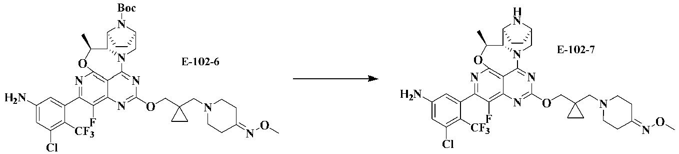

At room temperature, to a solution of E-102-6 (1.1 g, 1.37 mmol) in dichloromethane (30 mL) was added trifluoroacetic acid (6 mL). After the addition was completed, the resulting mixture was stirred at room temperature for 30 min. After concentration, the residue was purified using a silica gel chromatographic column to obtain E-102-7 (750 mg, yield: 77.9%). MS m/z (ESI): 705 [M+H].

### Step 7: Preparation of 1-((1-((((5S,5aS,6R,9S)-2-(5-amino-3-chloro-2-(trifluoromethyl)phenyl)-1-fluoro-5-methyl-5a,6,7,8,9,10-hexahydro-5H-4-oxa-3,10a,11,13,14-pentaaza-6,9-methylenenaphtho[1,8-ab]heptalen-12-yl)oxo)methyl)cyclopropyl)methyl)piperidin-4-one

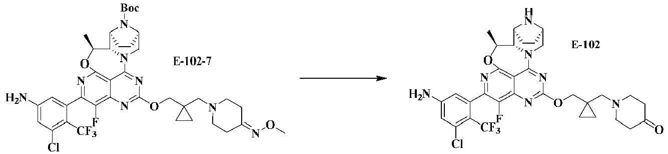

At room temperature, to a solution of E-102-7 (150 mg, 0.21 mmol) in dichloromethane (10 mL) was added dilute hydrochloric acid (1 M, 2 mL). After the addition was completed, the reaction mixture was stirred at room temperature for 5 hours. The mixture was then poured into saturated sodium bicarbonate aqueous solution. The resulting mixture was extracted with ethyl acetate and the organic phase was dried over anhydrous sodium sulphate, filtered and concentrated. The residue was purified using a high-performance liquid chromatographic column and lyophilized to obtain E-102 (62 mg, yield: 43.1%). MS m/z (ESI): 676 [M+H].

### Example 263

### 4-(Difluoromethylene)-1-(1-(((5S,6S,9R)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)piperidine 1-oxide

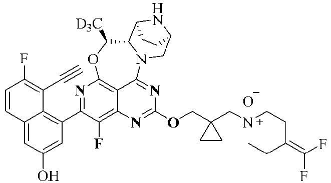

### Step 1: Preparation of compound (SS,6S,9R)-12-(1-((4-(difluoromethylene)piperidin-1-yl)methyl)cyclopropyl)methoxy)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptane-14 carboxylate

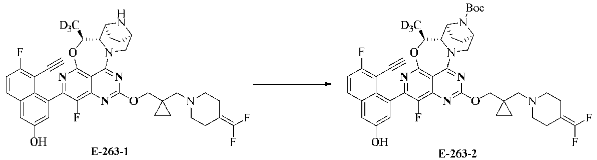

To a solution of E-263-1 (500 mg, 0.71 mmol) in dichloromethane (10 mL) were added triethylamine (216 mg, 2.13 mmol) and di-tert-butyl dicarbonate (155 mg, 0.71 mmol), and the mixture was reacted at room temperature for 1 hour. The reaction mixture was washed with saturated ammonium chloride solution, extracted with dichloromethane and subjected to phase separation. The organic phase was collected, washed with water and saturated sodium chloride aqueous solution, dried over anhydrous sodium sulphate, filtered and concentrated. The residue was purified using a silica gel chromatographic column to obtain E-263-2 (560 mg, yield: 97%). MS m/z (ESI): 804 [M+H].

### Step 2: Preparation of compound 1-(SS,6S,9R)-14-(tert-butyloxycarbonyl)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)-4-(difluoromethylene)piperidine 1-oxide

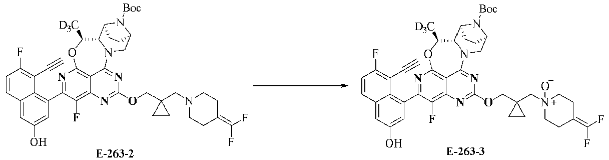

To a solution of E-263-2 (560 mg, 0.69 mmol) in dichloromethane (20 mL) was added m-chloroperoxybenzoic acid (227 mg, 1.12 mmol), and the resulting mixture was reacted at room temperature for 1 hour. The reaction solution was washed with sodium thiosulphate solution and the organic phase was dried over anhydrous sodium sulphate, filtered and concentrated to obtain crude E-263-3 (565 mg, yield: 100%). MS m/z (ESI): 820 [M+H].

### Step 3: Preparation of compound 4-(difluoromethylene)-1-(1-(((SS,6S,9R)-2-(8-ethynyl-7-fluoro-3-hydroxylnaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)piperidine 1-oxide

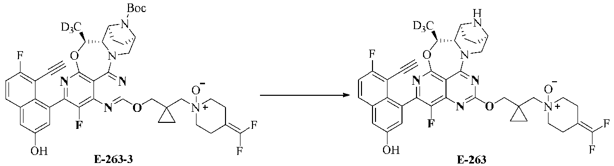

At 0°C, E-263-3 (565 mg, 0.69 mmol) was dissolved in a mixed solution of dichloromethane (10 mL) and MeOH (3 mL), HCl (4 M, 5 mL) was added, and the resulting mixture was transferred to room temperature and reacted for 2 hours. The reaction solution was concentrated and the crude was purified by preparative liquid chromatography to obtain E-263 (275 mg, yield: 50%). MS m/z (ESI): 720 [M+H].

¹H NMR (400 MHz, MeOD) δ 7.86 (dt, *J* = 8.9, 6.1 Hz, 1H), 7.37 - 7.25 (m, 2H), 7.18 (dd, *J =* 40.0, 2.7 Hz, 1H), 5.56 (dt, *J* = 14.6, 3.0 Hz, 1H), 4.72 - 4.56 (m, 3H), 4.31 (dd, *J* = 8.9, 3.6 Hz, 1H), 4.17 (d, *J* = 6.0 Hz, 1H), 4.11 (q, *J* = 6.0 Hz, 1H), 3.78 (s, 2H), 3.66 (d, *J* = 9.1 Hz, 2H), 3.52 - 3.38 (m, 3H), 2.81 - 2.67 (m, 2H), 2.54 (d, *J =* 14.8 Hz, 2H), 2.35 - 1.93 (m, 5H), 1.05 - 0.89 (m, 4H).

| **Example** | **Structure** | **MS m/z (ESI) [M+H]** | **Preparation** |
|---|---|---|---|
| **2** | 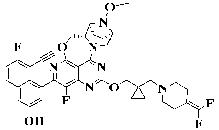 | 717 | Refer to Example 1 |
| **3** | 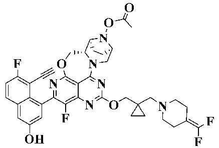 | 745 | Refer to Example 1 |
| **4** | 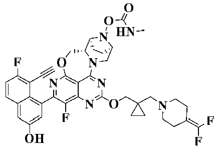 | 760 | Refer to Example 1 |
| **5** | 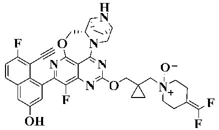 | 702 | Refer to Example 1 |
| **6** | 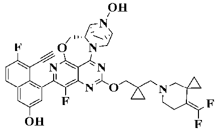 | 729 | Refer to Example 1 |
| **7** | 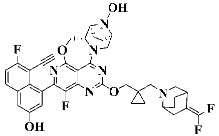 | 729 | Refer to Example 1 |
| **8** | 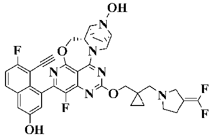 | 689 | Refer to Example 1 |
| **9** | 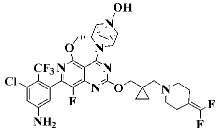 | 712 | Refer to Example 1 |
| **10** | 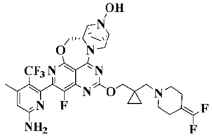 | 693 | Refer to Example 1 |
| **11** | 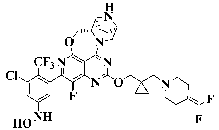 | 712 | Refer to Example 1 |
| **12** | 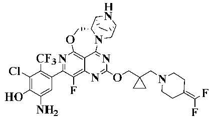 | 712 | Refer to Example 1 |
| **13** | 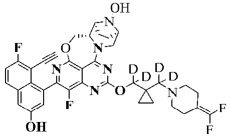 | 707 | Refer to Example 1 |
| **14** | 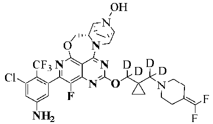 | 716 | Refer to Example 1 |
| **15** | 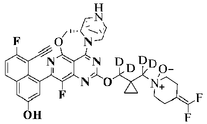 | 706 | Refer to Example 1 |
| **16** | 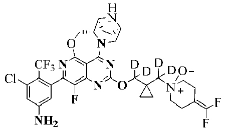 | 716 | Refer to Example 1 |
| **18** | 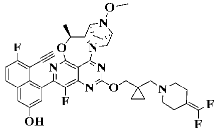 | 730 | Refer to Example 17 |
| **19** | 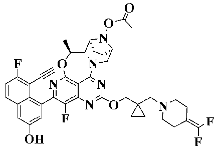 | 759 | Refer to Example 17 |
| **20** | 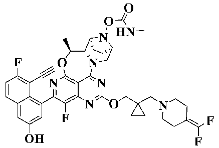 | 774 | Refer to Example 17 |
| **21** | 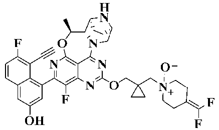 | 716 | Refer to Example 17 |
| **22** | 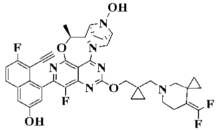 | 743 | Refer to Example 17 |
| **23** | 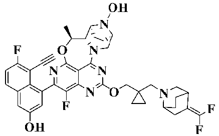 | 743 | Refer to Example 17 |
| **24** | 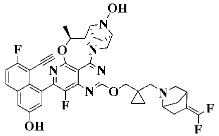 | 729 | Refer to Example 17 |
| **25** | 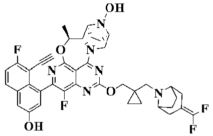 | 743 | Refer to Example 17 |
| **26** | 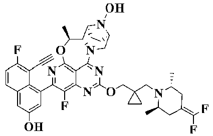 | 745 | Refer to Example 17 |
| **27** | 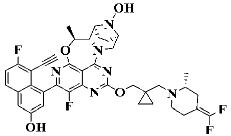 | 731 | Refer to Example 17 |
| **28** | 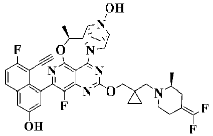 | 731 | Refer to Example 17 |
| **29** | 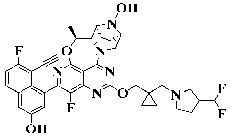 | 703 | Refer to Example 17 |
| **30** | 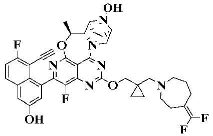 | 731 | Refer to Example 17 |
| **31** | 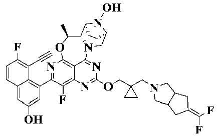 | 743 | Refer to Example 17 |
| **32** | 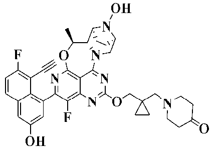 | 683 | Refer to Example 17 |
| **33** | 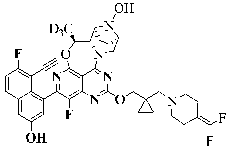 | 720 | Refer to Example 17 |
| **34** | 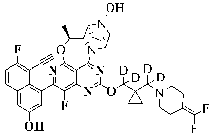 | 721 | Refer to Example 17 |
| **35** | 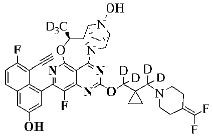 | 724 | Refer to Example 17 |
| **36** | 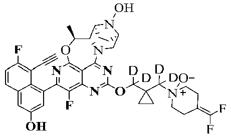 | 736 | Refer to Example 17 |
| **37** | 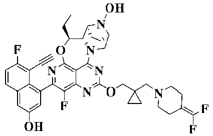 | 731 | Refer to Example 17 |
| **38** | 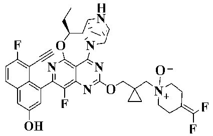 | 730 | Refer to Example 17 |
| **39** | 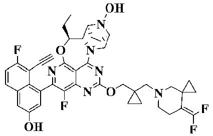 | 757 | Refer to Example 17 |
| **40** | 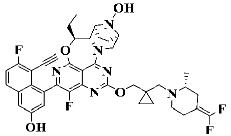 | 745 | Refer to Example 17 |
| **41** | 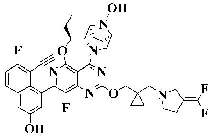 | 717 | Refer to Example 17 |
| **42** | 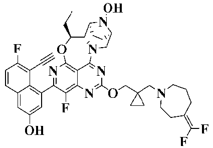 | 745 | Refer to Example 17 |
| **43** | 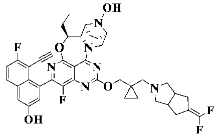 | 757 | Refer to Example 17 |
| **44** | 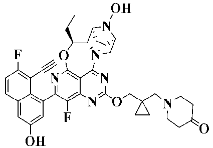 | 697 | Refer to Example 102 |
| **45** | 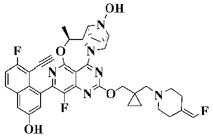 | 699 | Refer to Example 17 |
| **46** | 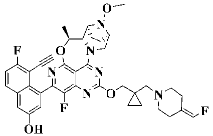 | 713 | Refer to Example 17 |
| **47** | 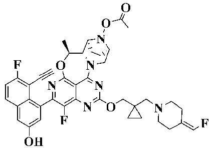 | 741 | Refer to Example 17 |
| **48** | 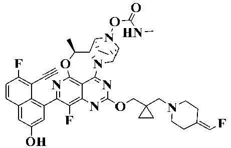 | 756 | Refer to Example 17 |
| **49** | 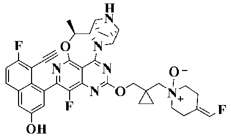 | 698 | Refer to Example 17 |
| **50** | 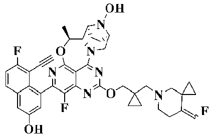 | 725 | Refer to Example 17 |
| **51** | 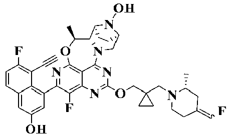 | 713 | Refer to Example 17 |
| **52** | 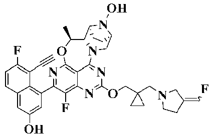 | 685 | Refer to Example 17 |
| **53** | 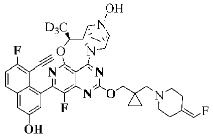 | 702 | Refer to Example 17 |
| **54** | 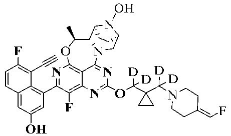 | 703 | Refer to Example 17 |
| **55** | 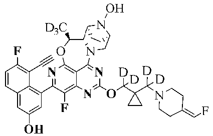 | 706 | Refer to Example 17 |
| **56** | 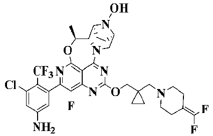 | 726 | Refer to Example 17 |
| **57** | 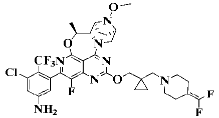 | 740 | Refer to Example 17 |
| **58** | 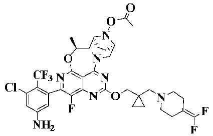 | 768 | Refer to Example 17 |
| **59** | 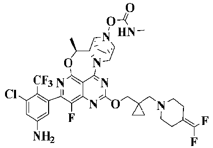 | 783 | Refer to Example 17 |
| **60** | 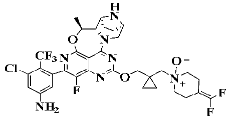 | 725 | Refer to Example 17 |
| **61** | 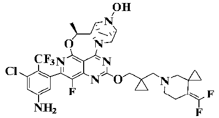 | 752 | Refer to Example 17 |
| **62** | 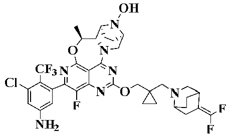 | 752 | Refer to Example 17 |
| **63** | 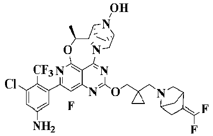 | 738 | Refer to Example 17 |
| **64** | 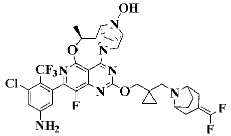 | 752 | Refer to Example 17 |
| **65** | 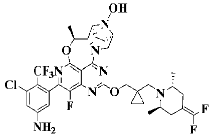 | 754 | Refer to Example 17 |
| **66** | 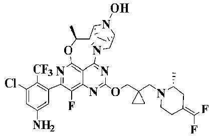 | 740 | Refer to Example 17 |
| **67** | 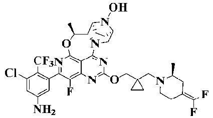 | 740 | Refer to Example 17 |
| **68** | 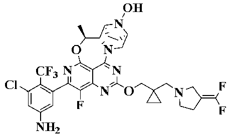 | 712 | Refer to Example 17 |
| **69** | 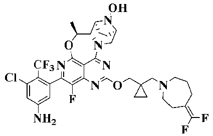 | 740 | Refer to Example 17 |
| **70** | 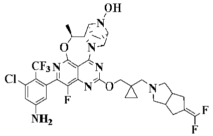 | 752 | Refer to Example 17 |
| **71** | 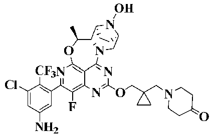 | 692 | Refer to Example 102 |
| **72** | 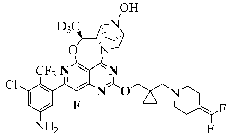 | 729 | Refer to Example 17 |
| **73** | 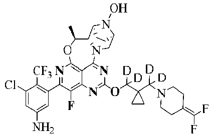 | 730 | Refer to Example 17 |
| **74** | 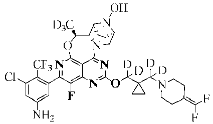 | 733 | Refer to Example 17 |
| **75** | 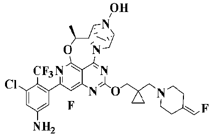 | 708 | Refer to Example 17 |
| **76** | 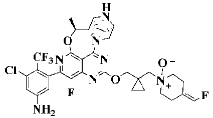 | 707 | Refer to Example 17 |
| **77** | 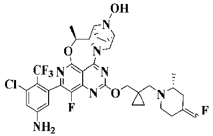 | 722 | Refer to Example 17 |
| **78** | 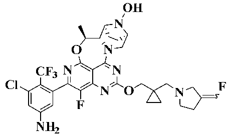 | 694 | Refer to Example 17 |
| **79** | 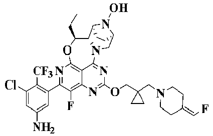 | 722 | Refer to Example 17 |
| **80** | 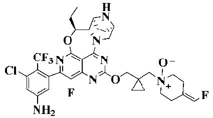 | 721 | Refer to Example 17 |
| **81** | 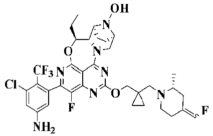 | 736 | Refer to Example 17 |
| **82** | 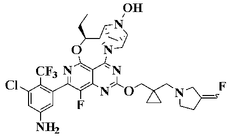 | 708 | Refer to Example 17 |
| **83** | 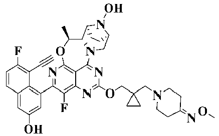 | 712 | Refer to Example 17 |
| **84** | 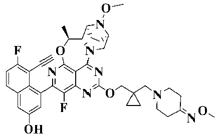 | 726 | Refer to Example 17 |
| **85** | 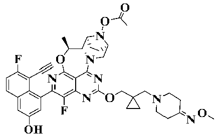 | 754 | Refer to Example 17 |
| **86** | 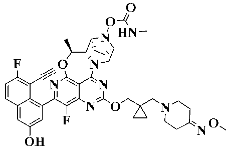 | 769 | Refer to Example 17 |
| **87** | 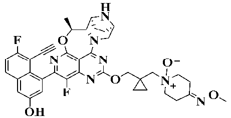 | 711 | Refer to Example 17 |
| **88** | 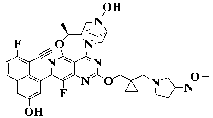 | 698 | Refer to Example 17 |
| **89** | 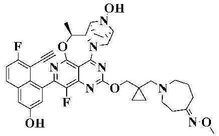 | 726 | Refer to Example 17 |
| **90** | 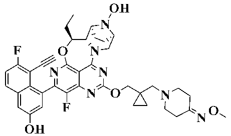 | 726 | Refer to Example 17 |
| **91** | 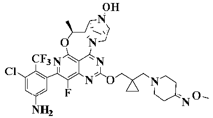 | 721 | Refer to Example 17 |
| **92** | 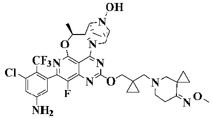 | 747 | Refer to Example 17 |
| **93** | 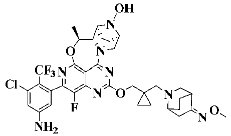 | 747 | Refer to Example 17 |
| **94** | 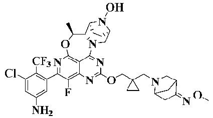 | 733 | Refer to Example 17 |
| **95** | 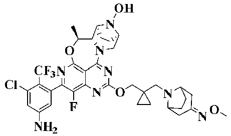 | 747 | Refer to Example 17 |
| **96** | 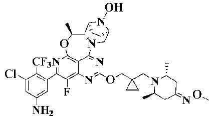 | 749 | Refer to Example 17 |
| **97** | 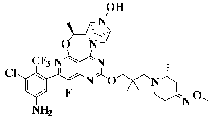 | 735 | Refer to Example 17 |
| **98** | 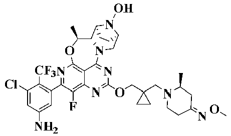 | 735 | Refer to Example 17 |
| **99** | 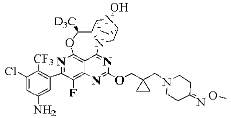 | 724 | Refer to Example 17 |
| **100** | 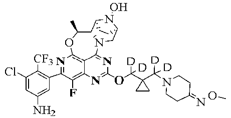 | 725 | Refer to Example 17 |
| **101** | 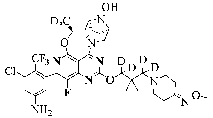 | 728 | Refer to Example 17 |
| **103** | 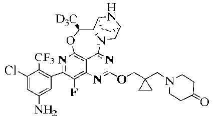 | 679 | Refer to Example 102 |
| **104** | 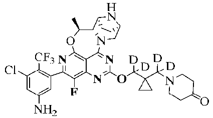 | 680 | Refer to Example 102 |
| **105** | 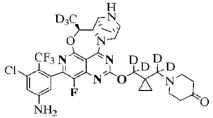 | 683 | Refer to Example 102 |
| **106** | 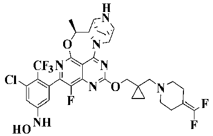 | 726 | Refer to Example 17 |
| **107** | 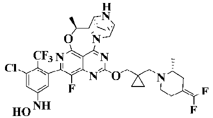 | 740 | Refer to Example 17 |
| **108** | 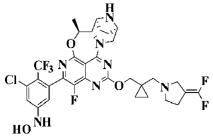 | 712 | Refer to Example 17 |
| **109** | 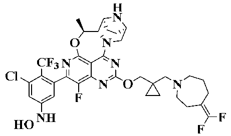 | 740 | Refer to Example 17 |
| **110** | 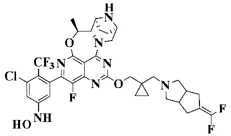 | 752 | Refer to Example 17 |
| **111** | 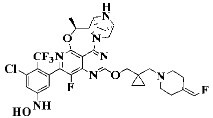 | 708 | Refer to Example 17 |
| **112** | 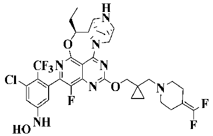 | 740 | Refer to Example 17 |
| **113** | 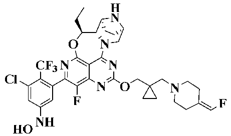 | 722 | Refer to Example 17 |
| **114** | 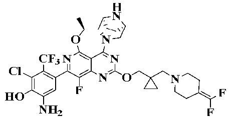 | 726 | Refer to Example 17 |
| **115** | 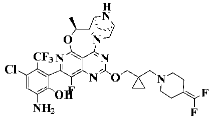 | 726 | Refer to Example 17 |
| **116** | 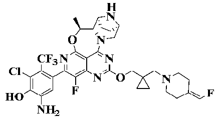 | 708 | Refer to Example 17 |
| **117** | 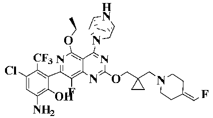 | 708 | Refer to Example 17 |
| **118** | 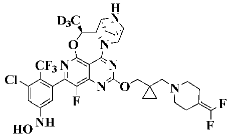 | 729 | Refer to Example 17 |
| **119** | 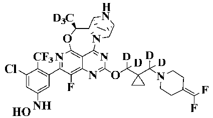 | 733 | Refer to Example 17 |
| **120** | 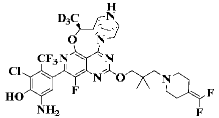 | 729 | Refer to Example 17 |
| **121** | 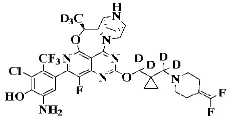 | 733 | Refer to Example 17 |
| **122** | 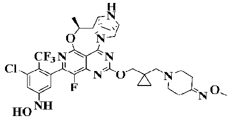 | 721 | Refer to Example 17 |
| **123** | 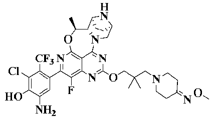 | 721 | Refer to Example 17 |
| **124** | 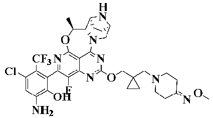 | 721 | Refer to Example 17 |
| **125** | 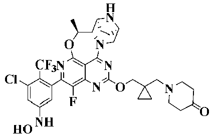 | 692 | Refer to Example 102 |
| **126** | 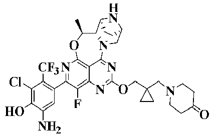 | 692 | Refer to Example 102 |
| **127** | 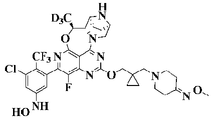 | 724 | Refer to Example 17 |
| **128** | 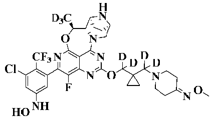 | 728 | Refer to Example 17 |
| **129** | 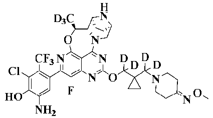 | 728 | Refer to Example 17 |
| **130** | 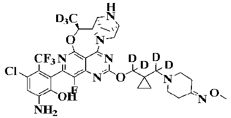 | 728 | Refer to Example 17 |
| **131** | 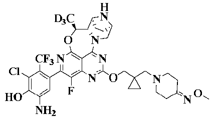 | 724 | Refer to Example 17 |
| **132** | 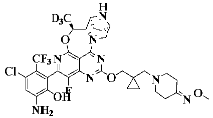 | 724 | Refer to Example 17 |
| **133** | 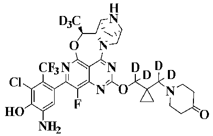 | 699 | Refer to Example 102 |
| **134** | 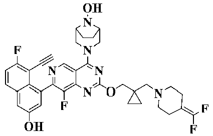 | 675 | Refer to Example 17 |
| **135** | 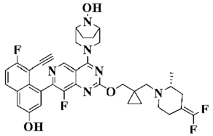 | 689 | Refer to Example 17 |
| **136** | 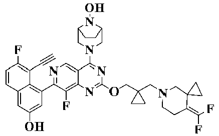 | 701 | Refer to Example 17 |
| **137** | 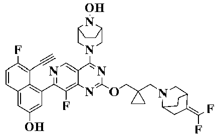 | 701 | Refer to Example 17 |
| **138** | 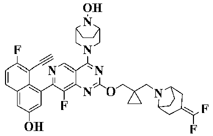 | 701 | Refer to Example 17 |
| **139** | 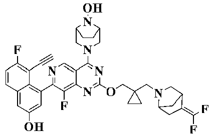 | 687 | Refer to Example 17 |
| **140** | 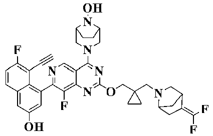 | 661 | Refer to Example 17 |
| **141** | 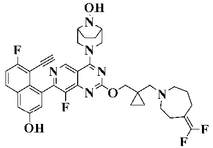 | 689 | Refer to Example 17 |
| **142** | 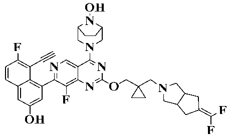 | 701 | Refer to Example 17 |
| **143** | 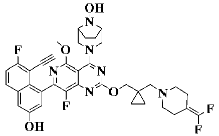 | 705 | Refer to Example 17 |
| **144** | 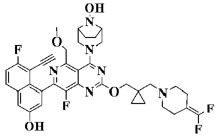 | 719 | Refer to Example 17 |
| **145** | 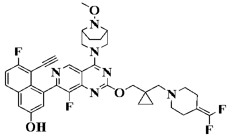 | 689 | Refer to Example 17 |
| **146** | 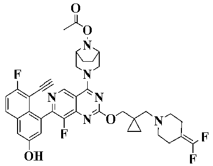 | 717 | Refer to Example 17 |
| **147** | 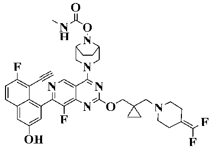 | 732 | Refer to Example 17 |
| **148** | 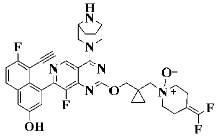 | 674 | Refer to Example 17 |
| **149** | 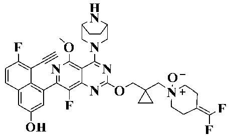 | 704 | Refer to Example 17 |
| **150** | 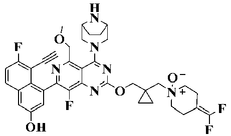 | 718 | Refer to Example 17 |
| **151** | 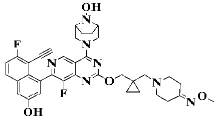 | 670 | Refer to Example 17 |
| **152** | 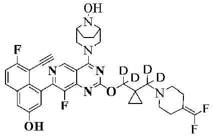 | 679 | Refer to Example 17 |
| **153** | 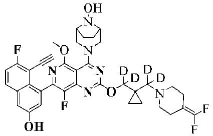 | 709 | Refer to Example 17 |
| **154** | 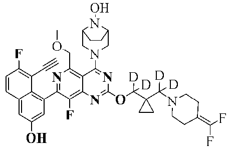 | 723 | Refer to Example 17 |
| **155** | 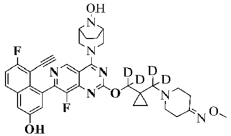 | 674 | Refer to Example 17 |
| **156** | 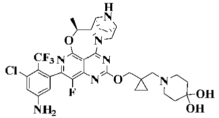 | 694 | Refer to Example 102 |
| **157** | 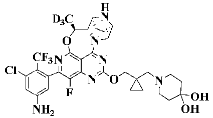 | 697 | Refer to Example 102 |
| **158** | 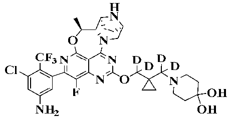 | 698 | Refer to Example 102 |
| **159** | 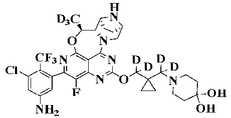 | 701 | Refer to Example 102 |
| **160** | 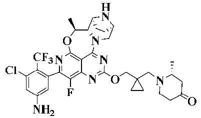 | 690 | Refer to Example 102 |
| **161** | 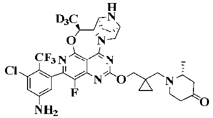 | 693 | Refer to Example 102 |
| **162** | 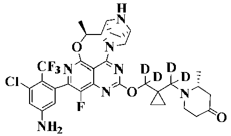 | 694 | Refer to Example 102 |
| **163** | 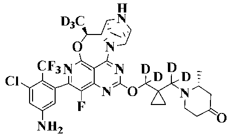 | 697 | Refer to Example 102 |
| **164** | 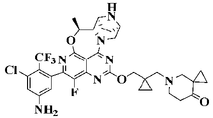 | 702 | Refer to Example 102 |
| **165** | 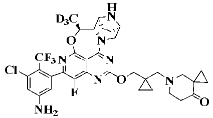 | 705 | Refer to Example 102 |
| **166** | 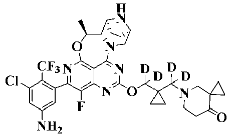 | 706 | Refer to Example 102 |
| **167** | 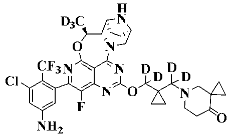 | 709 | Refer to Example 102 |
| **168** | 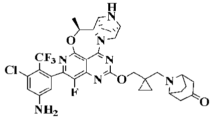 | 702 | Refer to Example 102 |
| **169** | 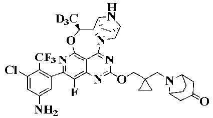 | 705 | Refer to Example 102 |
| **170** | 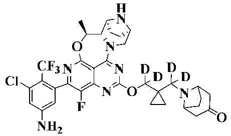 | 706 | Refer to Example 102 |
| **171** | 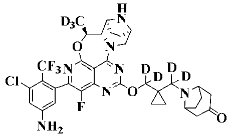 | 709 | Refer to Example 102 |
| **172** | 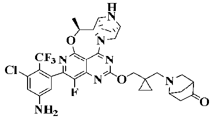 | 688 | Refer to Example 102 |
| **173** | 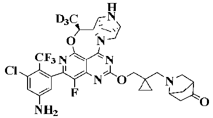 | 691 | Refer to Example 102 |
| **174** | 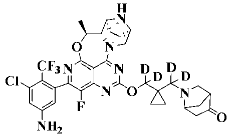 | 692 | Refer to Example 102 |
| **175** | 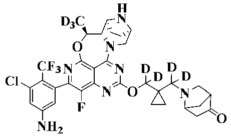 | 695 | Refer to Example 102 |
| **176** | 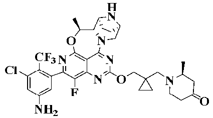 | 690 | Refer to Example 102 |
| **177** | 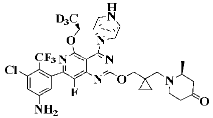 | 693 | Refer to Example 102 |
| **178** | 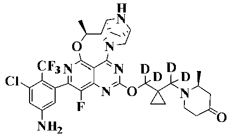 | 694 | Refer to Example 102 |
| **179** | 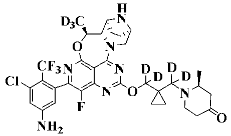 | 697 | Refer to Example 102 |
| **180** | 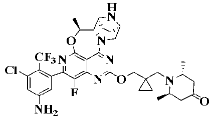 | 704 | Refer to Example 102 |
| **181** | 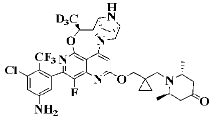 | 707 | Refer to Example 102 |
| **182** | 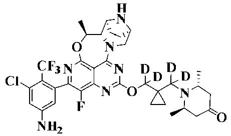 | 708 | Refer to Example 102 |
| **183** | 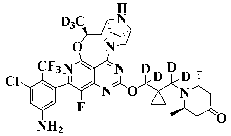 | 711 | Refer to Example 102 |
| **184** | 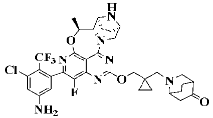 | 702 | Refer to Example 102 |
| **185** | 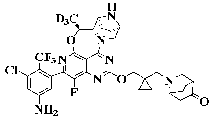 | 705 | Refer to Example 102 |
| **186** | 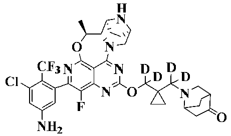 | 706 | Refer to Example 102 |
| **187** | 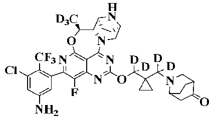 | 709 | Refer to Example 102 |
| **188** | 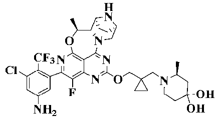 | 708 | Refer to Example 102 |
| **189** | 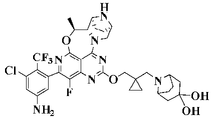 | 720 | Refer to Example 102 |
| **190** | 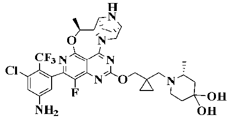 | 708 | Refer to Example 102 |
| **191** | 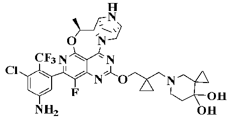 | 720 | Refer to Example 102 |
| **192** | 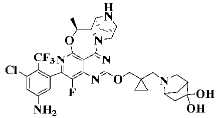 | 706 | Refer to Example 102 |
| **193** | 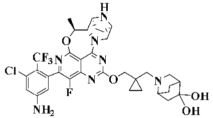 | 720 | Refer to Example 102 |
| **194** | 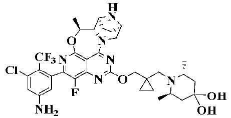 | 722 | Refer to Example 102 |
| **195** | 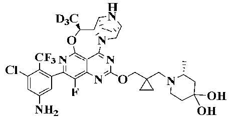 | 711 | Refer to Example 102 |
| **196** | 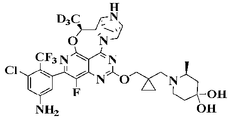 | 711 | Refer to Example 102 |
| **197** | 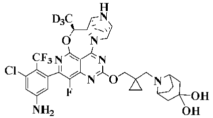 | 723 | Refer to Example 102 |
| **198** | 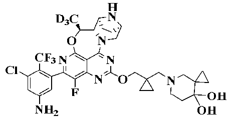 | 723 | Refer to Example 102 |
| **199** | 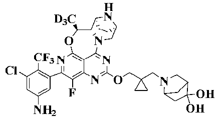 | 709 | Refer to Example 102 |
| **200** | 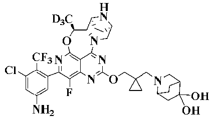 | 723 | Refer to Example 102 |
| **201** | 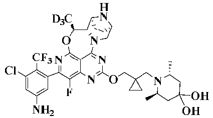 | 725 | Refer to Example 102 |
| **202** | 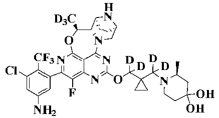 | 715 | Refer to Example 102 |
| **203** | 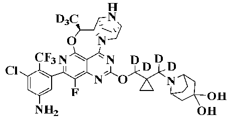 | 727 | Refer to Example 102 |
| **204** | 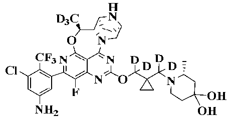 | 715 | Refer to Example 102 |
| **205** | 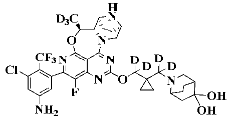 | 727 | Refer to Example 102 |
| **206** | 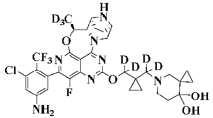 | 727 | Refer to Example 102 |
| **207** | 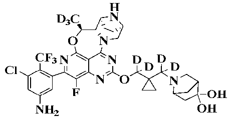 | 713 | Refer to Example 102 |
| **208** | 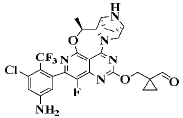 | 593 | Refer to Example 102 |
| **209** | 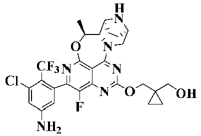 | 595 | Refer to Example 102 |
| **210** | 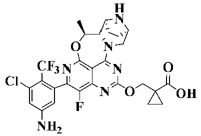 | 609 | Refer to Example 102 |
| **211** | 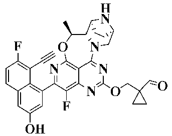 | 584 | Refer to Example 102 |
| **212** | 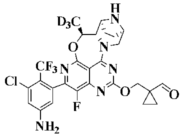 | 596 | Refer to Example 102 |
| **213** | 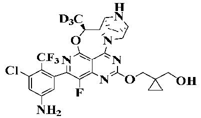 | 598 | Refer to Example 102 |
| **214** | 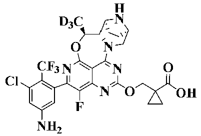 | 612 | Refer to Example 102 |
| **215** | 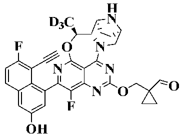 | 587 | Refer to Example 102 |
| **216** | 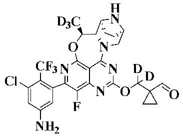 | 598 | Refer to Example 102 |
| **217** | 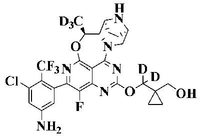 | 600 | Refer to Example 102 |
| **218** | 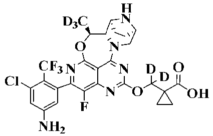 | 614 | Refer to Example 102 |
| **219** | 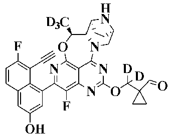 | 589 | Refer to Example 102 |
| **220** | 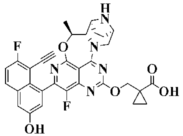 | 600 | Refer to Example 102 |
| **221** | 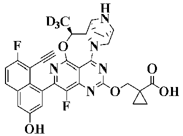 | 603 | Refer to Example 102 |
| **222** | 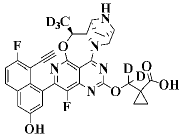 | 605 | Refer to Example 102 |
| **223** | 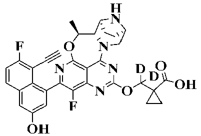 | 602 | Refer to Example 102 |
| **224** | 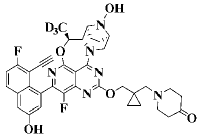 | 686 | Refer to Example 17 |
| **225** | 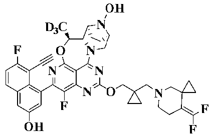 | 746 | Refer to Example 17 |
| **226** | 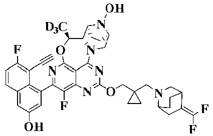 | 746 | Refer to Example 17 |
| **227** | 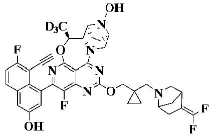 | 732 | Refer to Example 17 |
| **228** | 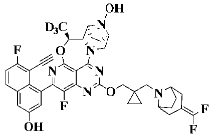 | 746 | Refer to Example 17 |
| **229** | 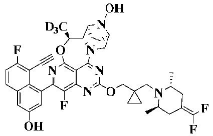 | 748 | Refer to Example 17 |
| **230** | 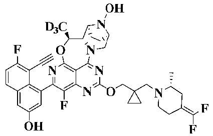 | 734 | Refer to Example 17 |
| **231** | 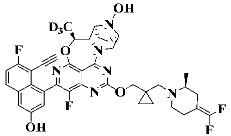 | 734 | Refer to Example 17 |
| **232** | 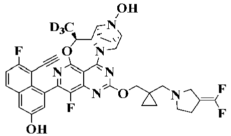 | 706 | Refer to Example 17 |
| **233** | 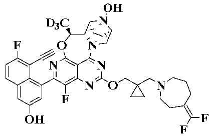 | 734 | Refer to Example 17 |
| **234** | 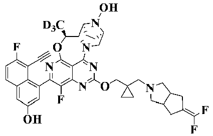 | 746 | Refer to Example 17 |
| **235** | 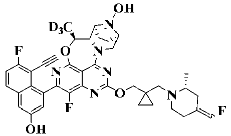 | 716 | Refer to Example 17 |
| **236** | 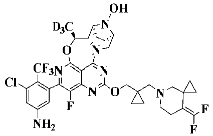 | 755 | Refer to Example 17 |
| **237** | 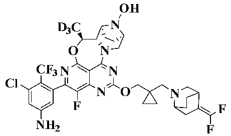 | 755 | Refer to Example 17 |
| **238** | 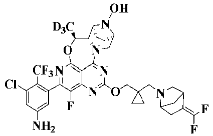 | 741 | Refer to Example 17 |
| **239** | 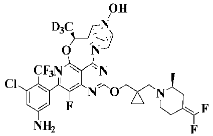 | 743 | Refer to Example 17 |
| **240** | 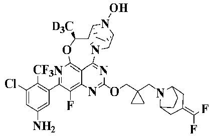 | 755 | Refer to Example 17 |
| **241** | 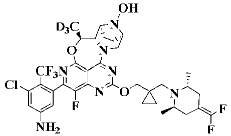 | 757 | Refer to Example 17 |
| **242** | 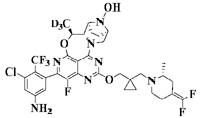 | 743 | Refer to Example 17 |
| **243** | 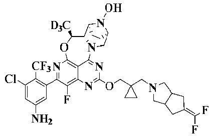 | 755 | Refer to Example 17 |
| **244** | 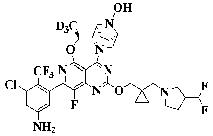 | 715 | Refer to Example 17 |
| **245** | 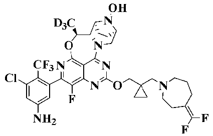 | 743 | Refer to Example 17 |
| **246** | 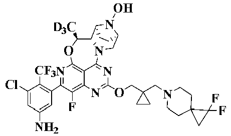 | 743 | Refer to Example 17 |
| **247** | 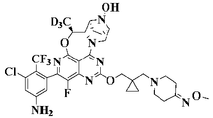 | 723 | Refer to Example 17 |
| **248** | 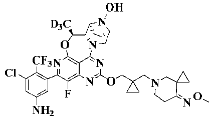 | 750 | Refer to Example 17 |
| **249** | 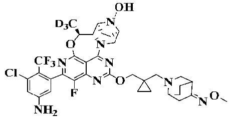 | 750 | Refer to Example 17 |
| **250** | 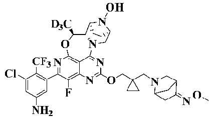 | 736 | Refer to Example 17 |
| **251** | 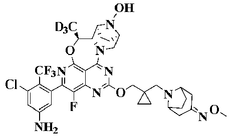 | 750 | Refer to Example 17 |
| **252** | 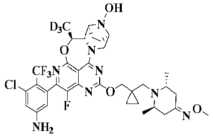 | 752 | Refer to Example 17 |
| **253** | 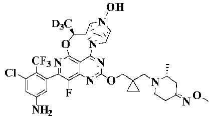 | 738 | Refer to Example 17 |
| **254** | 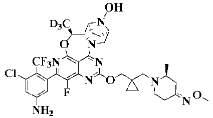 | 738 | Refer to Example 17 |
| **255** | 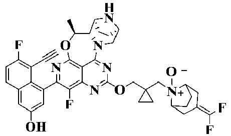 | 742 | Refer to Example 17 |
| **256** | 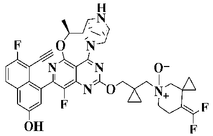 | 742 | Refer to Example 17 |
| **257** | 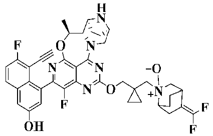 | 742 | Refer to Example 17 |
| **258** | 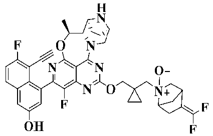 | 728 | Refer to Example 17 |
| **259** | 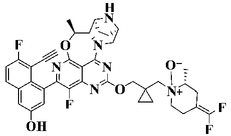 | 730 | Refer to Example 17 |
| **260** | 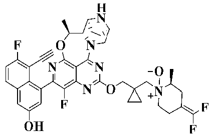 | 730 | Refer to Example 17 |
| **261** | 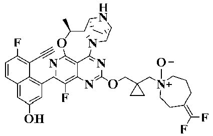 | 730 | Refer to Example 17 |
| **262** | 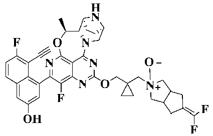 | 742 | Refer to Example 17 |
| **263** | 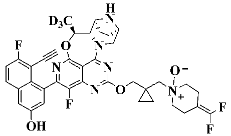 | 719 | Refer to Example 17 |
| **264** | 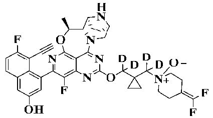 | 720 | Refer to Example 17 |
| **265** | 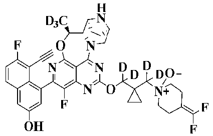 | 723 | Refer to Example 17 |
| **266** | 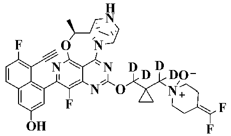 | 720 | Refer to Example 17 |
| **267** | 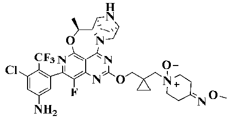 | 720 | Refer to Example 17 |
| **268** | 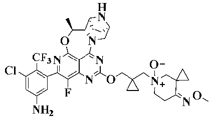 | 747 | Refer to Example 17 |
| **269** | 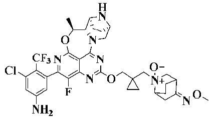 | 747 | Refer to Example 17 |
| **270** | 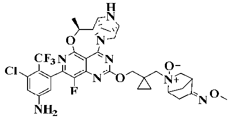 | 732 | Refer to Example 17 |
| **271** | 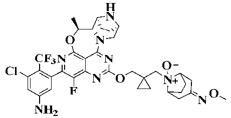 | 747 | Refer to Example 17 |
| **272** | 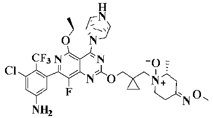 | 734 | Refer to Example 17 |
| **273** | 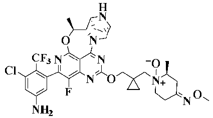 | 734 | Refer to Example 17 |
| **274** | 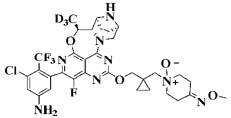 | 723 | Refer to Example 17 |
| **275** | 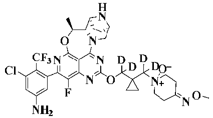 | 724 | Refer to Example 17 |
| **276** | 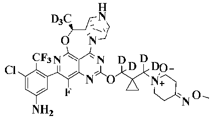 | 727 | Refer to Example 17 |
| **277** | 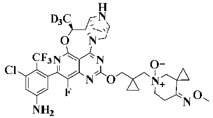 | 749 | Refer to Example 17 |
| **278** | 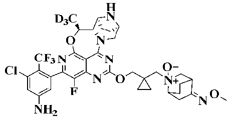 | 749 | Refer to Example 17 |
| **279** | 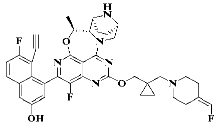 | 683 | Refer to Example 33 |
| **280** | 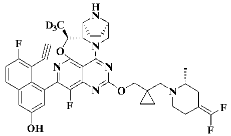 | 718 | Refer to Example 33 |
| **281** | 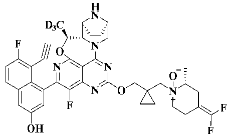 | 734 | Refer to Example 21 |
| **282** | 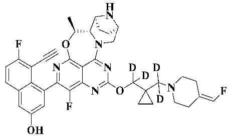 | 687 | Refer to Example 33 |
| **283** | 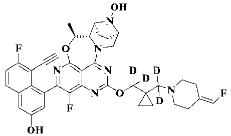 | 703 | Refer to Example 33 |
| **284** | 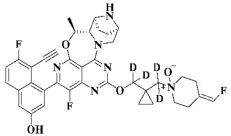 | 703 | Refer to Example 21 |

### Biological assay and evaluation

The present invention will be further described and explained below in conjunction with test examples, but these examples are not meant to limit the scope of the present invention.

### I. Determination of the activity of the compounds of the present invention in blocking KRAS G12D-SOS1 protein binding

1. Experimental objective: The purpose of this test example is to test the ability of the compounds to block the binding of KRAS G12D and SOS1 proteins.
2. Experimental reagents and instruments
   2.1 Experimental instruments: Centrifuge (5810R) was purchased from Eppendorf AG; pipettes were purchased from Eppendorf AG or Rainin LLC; and the microplate reader was purchased form BioTek (United States) with a model of SynergyH1 multifunctional microplate reader.
   2.2 Experimental reagents: KRAS-G12D/SOS1 Binding Assay Kit was purchased from Cisbio, with a catalogue number of 63ADK000CB17PEG; GTP was purchased from Sigma, with a catalogue number of G8877; 384-well plate was purchased from Perkin Elmer, with a catalogue number of 6007299.
3. Experimental methods: The diluent buffer in the kit was used to dilute the compound into 10× compound solution of different concentrations, and 2 uL was added to each well of the 384-well plate. Tag2-KRAS G12D protein and GTP were formulated into a mixed solution of 5×KRAS G12D-GTP protein using diluent buffer, and Tag1-SOS1 protein was formulated into a 5×SOS1 protein solution using diluent buffer. Each solution was added to the 384-well plate separately at 4 uL per well, and incubated at room temperature for 15 min. A mixed solution of 2× anti-Tag1-Tb³⁺ and anti-Tag2-XL665 formulated using detection buffer was added to the 384-well plate at 10 µL per well, and incubated at 4°C in the dark for 3 h. The plate was read using the time-resolved fluorescence program of the Biotek Synergy H1 instrument to detect fluorescence values at emission wavelengths of 665 nm and 620 nm.
4. Experimental processing method: The signal ratio (665 nm/620 nm*10,000) was calculated, and the nonlinear fitting of the signal ratio and sample concentration was performed using a four-parameter equation in GraphPad Prism 6 to obtain the IC₅₀ value.
5. Experimental results:
   By the above scheme, it was concluded that the compounds shown in the present invention showed a biological activity of about 0.01 nM to 1000 nM (IC₅₀) in the KRAS G12D-SOS1 binding experiment.
   In some embodiments, the compounds of the present invention have an IC₅₀ of less than 50 nM against KRAS G12D, preferably less than about 10 nM, further preferably less than about 5 nM, more preferably less than about 1 nM, and most preferably less than 0.1 nM among the compounds listed in the present invention.
6. Experimental conclusion: The example compounds of the present invention can effectively block the binding of KRAS G12D and SOS1 protein.

### II. Determination of the inhibition activity of the compounds of the present invention on the proliferation of KRAS G12D mutant tumour cell lines

1. Experimental objective: The inhibition activity of the example compounds on the proliferation of 5 KRAS G12D mutant cell lines SNU-1, HPAF-II, Panc0403, AsPC-1 and GP2D was determined.
2. Experimental instruments:
2.1 Instruments: Microplate reader (BioTek Synergy H1); Pipette (Eppendorf & Rainin).
2.2 Reagents: HPAF-II, PANC0403, AsPC-1 and GP2D were purchased from COBIOER BIOSCIENCES CO., LTD; SNU-1 was purchased from the cell bank of Chinese Academy of Sciences, Cell Titer-Glo was purchased from Promega, with a catalogue number of G7573; RPMI 1640 was purchased from Gibco, with a catalogue number of 22400089; DMEM was purchased from Gibco, with a catalogue number of 11995065; FBS was purchased from Gibco, with a catalogue number of 10091148; PBS was purchased from Gibco, with a catalogue number of 10010023; trypsin was purchased from Gibco, with a catalogue number of 25200056; cell culture plates were purchased from Corning Company, with a catalogue number of 3610.

3. Experimental methods: When SNU-1, HPAF-II, Panc0403, AsPC-1 or GP2D cells were cultured to the appropriate confluence, the SNU-1, HPAF-II, Panc0403, AsPC-1 or GP2D cells were collected. The cells were adjusted to the appropriate cell density using complete culture medium, the cell suspension was plated onto a 96-well plate at 90 µL per well, and the plate was placed in the incubator at 37°C and 5% CO₂ for adherent culture overnight. Solutions of the compound with different concentrations were formulated using DMSO and culture medium. A vehicle control was set up. The solutions of the compound were added to the 96-well plate at 10 µL per well, and the plate was placed in the incubator at 37°C and 5% CO₂ for further culture for about 72 h. Then CellTiter-Glo solution was added, and the obtained mixture was shaken and mixed evenly, incubated in the dark for 10 min, and read with a BioTek Synergy H1 microplate reader.
4. Experimental data processing method: The inhibition rate was calculated using the luminescence signal values, and the concentrations along with the inhibition rates were subjected to non-linear regression curve fitting using Graphpad Prism software to obtain the IC₅₀ values.
5. Experimental results:

**Table 1: Inhibition activity of the example compounds of the present invention on the proliferation of GP2D and AsPC-1 cells**

| **Example** | **GP2D IC₅₀ (nM)** | **AsPC-1 IC₅₀ (nM)** |
|---|---|---|
| **Example 21** | 0.71 | 35 |
| **Example 103** | 0.11 | 2.7 |
| **Example 105** | 0.16 | 2.5 |
| **Example 263** | 0.62 | 25 |
| **Example 276** | 0.49 | 14 |
| **Example 279** | 0.17 | 3.6 |
| **Example 280** | 0.20 | 4.0 |
| **Example 282** | 0.15 | 4.6 |
| **E-17-8** | 0.16 | 3.1 |
| **E-33-1** | 0.94 | 4.4 |

6. Experimental conclusion: The example compounds of the present invention have significant inhibition effects on the proliferation of SNU-1, HPAF-II, Panc0403, AsPC-1 and GP2D cells.

### III. Inhibition activity of the compounds of the present invention on pERK in AsPC-1 cells

1. Experimental objective: The inhibition activity of the example compounds on the phosphorylated ERK level in KRAS G12D mutant cells AsPC-1 was determined.
2. Experimental instruments:
   2.1 Instruments: Microplate reader (BioTek Synergy H1); Pipette (Eppendorf & Rainin).
   2.2 Reagents: Phosphorylated ERK1/2 (T202-Y204) LANCE Ultra Cellular Detection Kit was purchased from PerkinElmer, with a catalogue number of TRF4000M; DMEM was purchased from Gibco, with a catalogue number of 11995065; FBS was purchased from Gibco, with a catalogue number of 10091148; PBS was purchased from Gibco, with a catalogue number of 10010023; trypsin was purchased from Gibco, with a catalogue number of 25200056; cell culture plates were purchased from Corning Company, with a catalogue number of 3610; White opaque OptiPlate^{™}-384 plates were purchased from PerkinElmer, with a catalogue number of 6007290.
3. Experimental methods: When AsPC-1 cells were cultured to appropriate confluence, the AsPC-1 cells were collected, and the cell density was adjusted to 1 × 10⁶/mL using complete culture medium. The cell suspension was plated onto a 96-well plate at 50 µL per well, and the plate was placed in the incubator at 37°C and 5% CO₂ for adherent culture overnight. Solutions of the compound with different concentrations were formulated using DMSO and complete culture medium, and a vehicle control was set up. The solutions of the compound were added to the 96-well plate at 25 µL per well, and the plate was placed in the incubator at 37°C and 5% CO₂ for further culture for 2 h. Then, the supernatant of the cell culture plate was discarded, 50 µL of lysis buffer was added to each well, the cells were lysed at room temperature under shaking for 30 min, then placed in a centrifuge and centrifuged at 1000 rpm for 1 min. 15 µL of supernatant was transferred to a 384-well plate, and 5 µL of detection mixture was added to each well (Eu-labelled anti-ERK1/2 (T202-Y204) Antibody at a final detection concentration of 0.5 nM and ULight labelled anti-ERK1/2 Antibody at a final detection concentration of 5 nM), centrifuged at 1000 rpm for 1 min, mixed evenly, and reacted at room temperature overnight. The plate was read using BioTek Synergy H1 and the signal values at the emission wavelengths of 620 nm and 665 nm were detected using a time-resolved fluorescence program.
4. Experimental data processing method: The ratio of the signal values at the emission wavelengths of 665 nm and 620 nm was calculated, and the inhibition rate was calculated using the ratio. Graphpad Prism software was used to fit the concentration and inhibition rate to a nonlinear regression curve, so as to obtain the IC₅₀ value.
5. Experimental results: The IC₅₀ values of the example compounds of the present invention for inhibiting pERK in AsPC-1 cells were obtained through the above method.
   In some embodiments, the compounds of the present invention have an IC₅₀ of less than about 100 nM for pERK inhibition in AsPC-1 cells, preferably less than about 50 nM, further preferably less than about 5 nM, more preferably less than about 1 nM, and most preferably less than 0.1 nM among the compounds listed in the present invention.
6. Experimental conclusion: The example compounds of the present invention have good inhibition effect on pERK in AsPC-1 cells.

### IV. Pharmacokinetic determination in mice

1. Study objective: Balb/c mice were used as test animals to study the pharmacokinetic behaviour of the compounds in mice (plasma) after oral administration.
2. Experimental protocol
   2.1 Test drugs: The example compounds of the present invention, made in house.
   2.2 Experimental animals:
      Balb/c mice, male, purchased from Shanghai Jiesijie Experimental Animal Co., Ltd., animal production license number (SCXK (Shanghai) 2013-0006 N0.311620400001794).
   2.3 Drug formulation: Formulation of drug for oral administration: 10% Captisol in 50 mM citrate buffer pH 5.0

Formulation of 50 mM citric acid: 4.8 g of citric acid was weighed into a 1000 ml glass bottle, ultrapure water was added to 300 ml, and the mixture was magnetically stirred until it was completely dissolved, additional ultrapure water was then added to 500 ml to formulate 50 mM citric acid.

Formulation of a solution containing 50 mM citric acid (10% Captisol, PH = 5): 50 g of Captisol powder was weighed and added to a 1000 ml glass bottle, 300 ml of 50 mM citric acid was added, and the mixture was magnetically stirred until it was completely dissolved, then additional citric acid was added to 500 ml, and the PH was adjusted to 5 using 10 M NaOH.

The example compounds were weighed and added to a 4-mL glass bottle separately, 2.4 mL of the solution was added and the mixture was ultrasonicated for 10 min to obtain a colourless clear solution with a concentration of 3 mg/mL.

Formulation of drug for intravenous administration: 5% DMSO + 10% Solutol HS15 + 85% PBS

The example compounds were weighed, and 5% DMSO was added based on the total administration volume; the mixture was vortexed and ultrasonicated for 2 min to ensure complete dissolution; then, 10% Solutol HS15 was added, and the mixture was vortexed and ultrasonicated for 2 min to ensure complete dissolution; finally, 85% PBS was added, and the mixture was vortexed and ultrasonicated for 5 min, and filtered through a 0.22 um filter membrane to obtain a colourless and transparent clear solution with a concentration of 0.2 mg/mL.

2.4 Administration: 3 Balb/c mice, male, were fasted overnight and then respectively administered PO at a dose of 30 mg/kg and a volume of 10 mL/kg. 3 Balb/C mice, male; were administered intravenously (IV) at a dose of 1 mg/kg with an administration volume of 5 mL/kg.

2.5 Sample collection: 0.04 mL of blood was collected from the orbit before administration and at 0.25 h, 0.5 h, 1 h, 2 h, 4 h, 6 h, 8 h, and 24 h after administration and placed in an EDTA-2K test tube, and the test tube was centrifuged at 6000 rpm for 6 min at 4°C to separate the plasma, which was stored at -20°C; The animals were fed 4 h after administration.

3 Determination results: The final determination results obtained by applying the LCMS/MS method are shown in the table below:

**Table 2: Pharmacokinetic parameters of the compounds administered orally in mice**

| **Example (PO)** | **Tmax (hr)** | **Cmax (ng/mL)** | **AUC_{0-∞} (ng/mL*hr)** | **T_{1/2} (hr)** |
|---|---|---|---|---|
| **Example 17** | 2.0 | 270 | 1077 | 1.0 |
| **Example 21** | 2.0 | 236 | 1179 | 8.3 |
| **E-17-8** | 1.0 | 316 | 1606 | 7.2 |
| **E-33-1** | 2.0 | 221 | 1548 | / |

4 Experimental conclusion: The results show that the example compounds have a high oral exposure, and the N-oxides are mainly converted into non-N-oxides *in vivo.*

### V. Pharmacokinetic determination in rats

1. Study objective: SD rats were used as test animals to study the pharmacokinetic behaviour of the example compounds in rats (plasma) after oral administration.
2. Experimental protocol
   2.1 Test drugs: The example compounds of the present disclosure, made in house.
   2.2 Experimental animals: 3 SD rats/group, male, from Shanghai Jiesijie Laboratory Animal Co., Ltd., animal production license number (SCXK (Shanghai) 2013-0006 N0.311620400001794).
   2.3 Drug formulation: Formulation of drug for oral administration: 10% Captisol in 50 mM citrate buffer pH 5.0

Formulation of 50 mM citric acid: 4.8 g of citric acid was weighed into a 1000 ml glass bottle, ultrapure water was added to 300 ml, and the mixture was magnetically stirred until it was completely dissolved, additional ultrapure water was then added to 500 ml to formulate 50 mM citric acid.

Formulation of a solution containing 50 mM citric acid (10% Captisol, PH = 5): 50 g of Captisol powder was weighed and added to a 1000 ml glass bottle, 300 ml of 50 mM citric acid was added, and the mixture was magnetically stirred until it was completely dissolved, then additional citric acid was added to 500 ml, and the PH was adjusted to 5 using 10 M NaOH.

The example compounds were weighed and dissolved in the solution and the mixture was shaken well and ultrasonicated for 15 min to obtain a colourless clear solution with a concentration of 3 mg/mL. Formulation of drug for intravenous administration: 5% DMSO + 10% Solutol HS15 + 85% PBS

The example compounds were weighed, and 5% DMSO was added based on the total administration volume; the mixture was vortexed and ultrasonicated for 2 min to ensure complete dissolution; then, 10% Solutol HS15 was added, and the mixture was vortexed and ultrasonicated for 2 min to ensure complete dissolution; finally, 85% PBS was added, and the mixture was vortexed and ultrasonicated for 5 min, and filtered through a 0.22 um filter membrane to obtain a colourless and transparent clear solution with a concentration of 0.2 mg/mL.

2.4 Administration: 3 SD rats/group, male; were fasted overnight and then respectively administered PO at a dose of 30 mg/kg and a volume of 10 mL/kg. 3 SD rats/group, male; were administered intravenously (IV) at a dose of 1 mg/kg with an administration volume of 5 mL/kg.

2.5 Sample collection: 0.2 mL of blood was collected from the jugular vein before administration and at 0.25 h, 0.5 h, 1.0 h, 2.0 h, 4.0 h, 6.0 h, 8.0 h, and 24.0 h after administration and placed in an EDTA-2K test tube, and the test tube was centrifuged at 6000 rpm for 6 min at 4°C to separate the plasma, which was stored at -20°C. The animals were fed 4 h after administration.

3 Experimental results: The final determination results obtained by applying the LCMS/MS method are shown in the table below:

**Table 3: Pharmacokinetic parameters of the compounds administered orally in rats**

| **Example (PO)** | **Tmax (hr)** | **Cmax (ng/mL)** | **AUC_{0-∞} (ng/mL*hr)** | **T_{1/2} (hr)** | **F (%)** |
|---|---|---|---|---|---|
| **Example 21** | 4.0 | 409 | 3159 | 6.5 | / |
| **Example 279** | 1.0 | 103 | 938 | 9.6 | 5.4 |
| **Example 280** | 4.0 | 89 | 1591 | 10.5 | 10.3 |
| **E-17-8** | 2.0 | 329 | 3076 | 8.2 | 5.4 |
| **E-33-1** | 6.0 | 254 | 2887 | 6.9 | 7 |

### 4 Experimental conclusion:

The results show that the example compounds have a high oral exposure, and the N-oxides are mainly converted into non-N-oxides *in vivo.*

### VI. Anti-tumour efficacy experiment in mouse transplanted tumour model of human pancreatic cancer cell line (HPAC)

1. Experimental objective: 8-10 week old female conventional immunodeficient mice (BALB/c nude) with a body weight of 18 g-22 g were used as experimental animals, and a transplanted tumour model of human pancreatic cancer cell line (HPAC) was used to conduct *in vivo* efficacy experiments to test the anti-tumour results of the example compounds after gavage administration.
2. Experimental instruments and reagents:
2.1 Instruments: Ultra-clean workbench (BSC-1300II A2, Shanghai Boxun Industrial Co., Ltd. Medical Equipment Factory); CO₂ incubator (Thermo-311, Thermo); centrifuge (Centrifuge 5720R, Eppendorf); automated cell counter (Countess II, Life Technologies); Pipette (10-20 µL, Eppendorf); Microscope (Ts 2, Nikon); vernier calliper (CD-6"AX, Mitutoyo, Japan); cell culture flask (T25/T75/T225, Corning); Thermostat water tank (HWS12, Shanghai Yiheng Science).
2.2 Reagents: DMEM/F12 (11330-032, Gibco); foetal bovine serum (FBS) (10091-148, Gibco); Insulin (51500-056, Gibco); EGF (PHG0311, Gibco); 0.25% trypsin (25200-056, Gibco); phosphate buffer solution (PBS) (10010-023, Gibco); Matrigel (356234, Corning); Gln (25030-081, Gibco).

3. Experimental methods
3.1 Experimental compounds: The example compounds of the present disclosure, made in house.
3.2 Compound formulation: A given amount of the example compound was weighed, and an appropriate amount of 10% vehicle (Solutol HS15) was added. The mixture was stirred on a magnetic stirrer for 0.5 h to formulate a homogeneous suspension at the desired concentration.
3.3 Cell culture and passaging: Human pancreatic cancer cell line (HPAC) was obtained from COBIOER BIOSCIENCES CO., LTD, cultured on DMEM/F12 medium (Gibco, 11330-032) supplemented with 10% foetal calf serum (Gibco, 10091-148), 1×Insulin (51500-056, Gibco) and 10 ng/ml of EGF (PHG0311, Gibco) and observed under a microscope (Thermo Fisher, 311). The cells were routinely passaged when the cells reached 80%-90% confluence in the culture flask. The passaged cells were cultured in a 37°C, 5% CO₂ incubator (Thermo Fisher, 311).
3.4 Cell seeding, grouping and administration: Under aseptic conditions, human pancreatic cancer cells (HPAC) in the logarithmic growth phase were harvested using 0.25% trypsin-EDTA (Gibco, 25200-056), washed and resuspended with phosphate buffer. The cell suspension was mixed with Matrigel (Coming, 356234) at a 1:1 ratio and implanted subcutaneously into the right anterior dorsal region of immunodeficient mice (BALB/c nude). Each mouse was inoculated with 3*10⁶ cells in a volume of 100 microliters (µL). After inoculation, when the tumour volume reached 100-200 mm³, the mice were evenly randomized into 5 groups based on the tumour size, with 5 mice per group, for *in vivo* efficacy experiments. The negative control group was the vehicle group.
   Mode of administration: The example compounds were administered orally. The dosage and administration period are as shown in the table.
3.5 Tumour measurement and tumour inhibition rate calculation: Twice a week, the long and short diameters of the tumour were measured using a vernier calliper, and the tumour volume (mm³) was calculated using the formula: V = 0.5*D*d*d, in which D and d are the long and short diameters of the tumour, respectively. The anti-tumour efficacy was determined by dividing the average increase in tumour volume of compound-treated animals by the average increase in tumour volume of untreated animals. The calculation formulas for tumour inhibition rate: when there is no tumour regression, TGI(%) = 100-[(Vₜ-V₀) administration group/(Vₜ-V₀) vehicle control group]*100%; and when tumour regression occurs, TGI(%)=[1-(Vₜ-V₀) administration group/V₀ vehicle control group]*100%. All animals were euthanized after the experiment.

4. Experimental results and conclusions

**Table 4: Efficacy parameters of the compounds in mice with transplanted tumours**

| **Group** | **Tumour volume (mm³, Mean ± SD)** | | **ΔT/ΔC (%)** | **TGI (%)** |
|---|---|---|---|---|
| | Day 0 | Day 21 | Day 21 | Day 21 |
| **Example 263,** 100 mpk, PO*BID | 208 | 308 | 26.90 | 89.33 |
| **Example 279,** 100 mpk, PO*BID | 209 | 231 | 20.27 | 97.63 |
| **Example 280**, 100 mpk, PO*BID | 210 | 155 | 13.54 | 126.15 |
| **Example 282,** 100 mpk, PO*BID | 210 | 153 | 13.29 | 127.51 |
| **E-33-1,** 100 mpk, PO*BID | 209 | 131 | 11.47 | 137.45 |

As shown in the table above, the example compounds at an oral dose of 100 mg/kg can achieve the effects of inhibiting tumour proliferation or causing tumour regression, and the body weight of mice did not significantly decrease, exhibiting that orally administered example compounds demonstrate potent tumour inhibitory effects and possess favourable safety.

## Claims

1. A compound represented by general formula (A), or an N-oxide, an oxo derivative, or a pharmaceutically acceptable salt thereof: 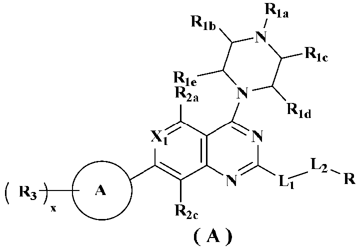 **characterised in that**:
X₁ is selected from N or CR_{2b}, preferably N;
L₁ is selected from O, S or NH, preferably O;
L₂ is C₁-C₄ alkylene, and the C₁-C₄ alkylene is optionally substituted with 1, 2, 3, 4, 5, or 6 Rₐ;
ring A is selected from C₃₋₁₀ cycloalkyl, 3- to 10-membered heterocyclyl, C₆₋₁₂ aryl, or 5- to 12-membered heteroaryl;
R is selected from -OR_{b}, -CH₂OR_{b}, -C(O)R_{b}, -C(O)OR_{b}, 3- to 10-membered heterocyclyl, or 5- to 12-membered heteroaryl, and the 3- to 10-membered heterocyclyl or 5- to 12-membered heteroaryl is optionally substituted with 1, 2, 3, 4, 5, 6, 7, or 8 R₄ₐ substituents;
R₁ₐ is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, -OR_{b}, -OC(O)R_{b}, -OC(O)NR_{b}R_{c}, - OS(O)₂R_{b}, or -OS(O)₂NR_{b}R_{c}, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{1b}, R_{1c}, R_{1d}, and R₁ₑ are each independently selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5-to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, R_{1b} and R_{1c} together with adjacent atoms to which they are attached form 3- to 12-membered heterocyclyl or 5- to 14-membered heteroaryl optionally substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R₂ₐ is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, or (CH₂)n₁ORₑ, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, R₂ₐ and R₁ₑ are attached to form 3- to 12-membered heterocyclyl or 5- to 14-membered heteroaryl optionally substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{2b} is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{2c} is selected from hydrogen, deuterium, halogen, amino, hydroxyl, mercapto, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R₃ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
alternatively, any two R₃ groups are attached to form C₃₋₈ cycloalkyl, 3- to 8-membered heterocyclyl, C₆₋₁₀ aryl, or 5- to 10-membered heteroaryl, and the C₃₋₈ cycloalkyl, 3- to 8-membered heterocyclyl, C₆₋₁₀ aryl, and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R₄ₐ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, 5- to 14-membered heteroaryl, =CRₐₐR_{bb}, or N-OR_{cc}, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, any two R₄ₐ groups are attached to form C₃₋₈ cycloalkyl or 3- to 8-membered heterocyclyl, and the C₃₋₈ cycloalkyl and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, or 5- to 10-membered heteroaryl;
Rₐ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, 2 Rₐ groups on the same carbon atom or on different carbon atoms are attached to form C₃₋₁₂ cycloalkyl or 3- to 12-membered heterocyclyl, which is optionally further substituted with one or more groups selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
R_{b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
R_{c} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
Rₑ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
Rₐₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{bb} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{cc} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
n1 is selected from 0, 1, 2, or 3;
x is selected from 0, 1, 2, 3, 4, 5, or 6.

2. The compound represented by general formula (A), or the N-oxide, the oxo derivative, or the pharmaceutically acceptable salt thereof according to claim 1, **characterised in that** the compound is further as represented by general formula general formula (I) or (I-A): 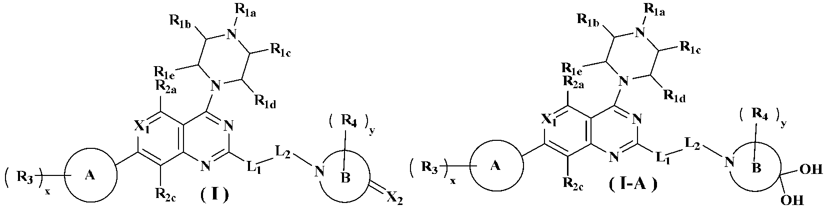 wherein:
X₂ is selected from O, CR₅ₐR_{5b}, or N-OR_{5c};
ring B is selected from 3- to 10-membered heterocyclyl or 5- to 12-membered heteroaryl;
R₄ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
alternatively, any two R₄ groups are attached to form C₃₋₈ cycloalkyl or 3- to 8-membered heterocyclyl, and the C₃₋₈ cycloalkyl and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, or 5- to 10-membered heteroaryl;
R₅ₐ is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{5b} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R_{5c} is selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
y is selected from 0, 1, 2, 3, 4, 5, or 6.

3. The compound, or the N-oxide, the oxo derivative, or the pharmaceutically acceptable salt thereof according to claim 1 or 2, **characterised in that** R_{1b} and R_{1c} together with adjacent atoms to which they are attached form the following structure 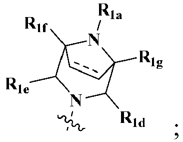 preferably form the following structure 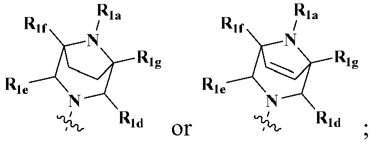 more preferably form the following structure 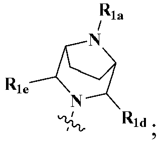 R_{1f} and R_{1g} are each independently selected from hydrogen, deuterium, halogen, amino, hydroxyl, cyano, nitro, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl.

4. The compound, or the N-oxide, the oxo derivative, or the pharmaceutically acceptable salt thereof according to claims 1-3, **characterised in that** the compound is further as represented by general formula (II-A) or (II-B): 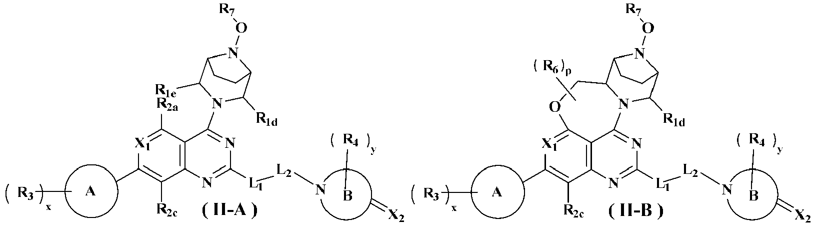
R₆ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, - C(O)-C₁₋₆ alkyl, C₃₋₈ cycloalkyl, 3- to 8-membered heterocyclyl, C₆₋₁₀ aryl, or 5- to 10-membered heteroaryl;
R₇ is selected from hydrogen, C₁₋₆ alkyl, C₂₋₆ alkenyl, C₂₋₆ alkynyl, C₁₋₆ deuterated alkyl, C₁₋₆ haloalkyl, C(O)R_{b}, -C(O)NR_{b}R_{c}, -S(O)₂R_{b}, or -S(O)₂NR_{b}R_{c};
R_{b} is selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
R_{c} is selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
p is selected from 0, 1, 2, 3, or 4.

5. The compound, or the N-oxide, the oxo derivative, or the pharmaceutically acceptable salt thereof according to claims 1-3, **characterised in that** the compound is further as represented by general formula (III-A) or (III-B): 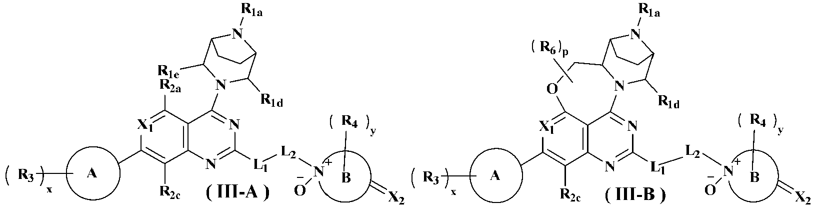
R₆ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, - C(O)-C₁₋₆ alkyl, C₃₋₈ cycloalkyl, 3- to 8-membered heterocyclyl, C₆₋₁₀ aryl, or 5- to 10-membered heteroaryl;
p is selected from 0, 1, 2, 3, or 4.

6. The compound, or the N-oxide, the oxo derivative, or the pharmaceutically acceptable salt thereof according to any one of claims 1-5, **characterised in that**
R₅ₐ is selected from hydrogen, deuterium, halogen, cyano, C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, or 3- to 8-membered heterocyclyl, and the C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; and is preferably hydrogen, deuterium, fluorine, chlorine, bromine, methyl, ethyl, deuterated methyl, deuterated ethyl, halomethyl, haloethyl, or cyclopropyl;
R_{5b} is selected from hydrogen, deuterium, halogen, cyano, C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, or 3- to 8-membered heterocyclyl, and the C₁₋₃ alkyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₃₋₈ cycloalkyl, and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; and is preferably hydrogen, deuterium, fluorine, chlorine, bromine, methyl, ethyl, deuterated methyl, deuterated ethyl, halomethyl, haloethyl, or cyclopropyl; ,
R_{5c} is selected from C₁₋₃ alkyl, C₁₋₃ deuterated alkyl, C₂₋₄ alkenyl, C₂₋₄ alkynyl, C₁₋₃ haloalkyl, C₁₋₃ hydroxyalkyl, C₃₋₈ cycloalkyl, or 3- to 8-membered heterocyclyl, and the C₁₋₃ alkyl, C₁₋₃ deuterated alkyl, C₂₋₄ alkenyl, C₂₋₄ alkynyl, C₁₋₃ haloalkyl, C₁₋₃ hydroxyalkyl, C₃₋₈ cycloalkyl, and 3- to 8-membered heterocyclyl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl; and is preferably methyl, ethyl, deuterated methyl, deuterated ethyl, halomethyl, haloethyl, cyclopropyl, or cyclopropylmethyl.

7. The compound, or the N-oxide, the oxo derivative, or the pharmaceutically acceptable salt thereof according to any one of claims 1-6, **characterised in that**
ring B is selected from 3- to 8-membered monocyclic heterocyclyl, 7- to 10-membered fused heterocyclyl, 7- to 11-membered spiro heterocyclyl, or 7- to 10-membered bridged heterocyclyl;
and is preferably the following group:

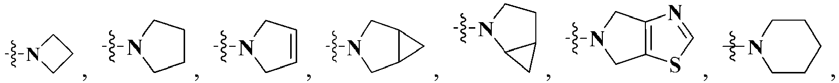

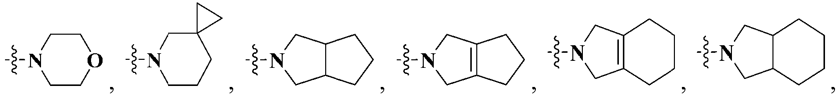

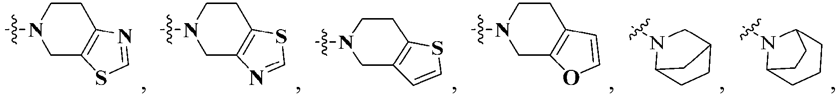

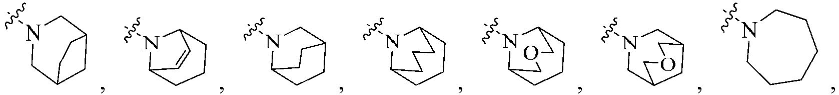

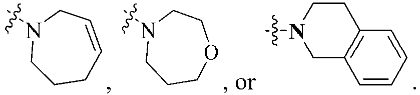

8. The compound, or the N-oxide, the oxo derivative, or the pharmaceutically acceptable salt thereof according to any one of claims 1-7, **characterised in that**
R₄ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, and 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
and is preferably hydrogen, deuterium, fluorine, chlorine, bromine, amino, nitro, hydroxyl, cyano, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, or hydroxyethyl.

9. The compound, or the N-oxide, the oxo derivative, or the pharmaceutically acceptable salt thereof according to any one of claims 1-8, **characterised in that** L₂ is selected from 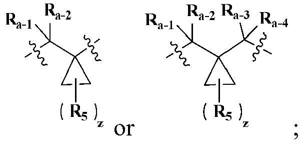 preferably, 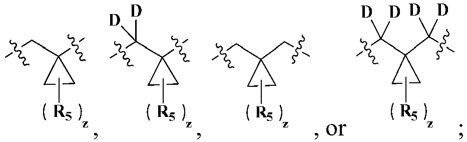
alternatively, L₂ is 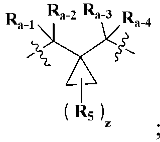 preferably 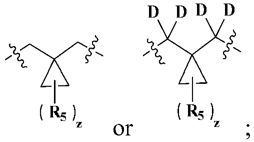
Rₐ₋₁, Rₐ₋₂, Rₐ₋₃ and Rₐ₋₄ are each independently selected from hydrogen, deuterium, halogen, amino, hydroxyl, cyano, C₁₋₆ alkyl, C₁₋₆ deuterated alkyl, C₁₋₆ haloalkyl, or C₁₋₆ hydroxyalkyl; preferably hydrogen or deuterium;
R₅ is selected from hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl, and the amino, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ alkylthio, C₁₋₆ deuteroalkoxy, C₁₋₆ haloalkoxy, C₁₋₆ hydroxyalkyl, C₁₋₆ alkylamino, C₃₋₁₂ cycloalkyl, 3- to 12-membered heterocyclyl, C₆₋₁₄ aryl, or 5- to 14-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
z is selected from 0, 1, 2, 3, or 4.

10. The compound, or the N-oxide, the oxo derivative, or the pharmaceutically acceptable salt thereof according to any one of claims 1-9, **characterised in that**
R_{1d} is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, and C₁₋₃ alkylamino may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
preferably hydrogen, deuterium, fluorine, chlorine, or methyl;
R₁ₑ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, and C₁₋₃ alkylamino may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
preferably hydrogen, deuterium, fluorine, chlorine or methyl;
R₂ₐ is selected from hydrogen, deuterium, halogen, amino, hydroxyl, mercapto, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, or -(CH₂)ₙ₁ORₑ, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5- to 10-membered heteroaryl;
Rₑ is selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, C₃₋₈ cycloalkyl, 3- to 8-membered heterocyclyl, C₆₋₁₀ aryl, and 5-to 10-membered heteroaryl;
n1 is selected from 0, 1, 2, or 3;
R_{2c} is selected from hydrogen, deuterium, halogen, amino, hydroxyl, mercapto, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, or C₁₋₃ alkylamino;
preferably, R_{2c} is selected from hydrogen, deuterium, fluorine, chlorine or bromine;
more preferably, R_{2c} is fluorine.

11. The compound, or the N-oxide, the oxo derivative, or the pharmaceutically acceptable salt thereof according to any one of claims 1-10, **characterised in that** the compound is further as represented by general formula (IV) or (V): 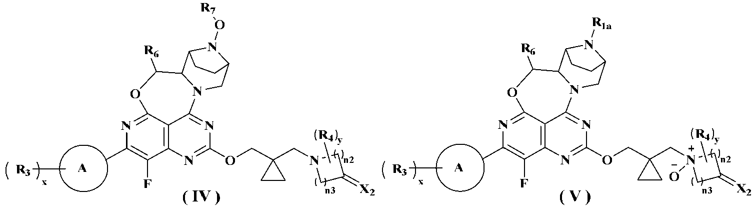
n2 is selected from 0, 1, 2, or 3;
n3 is selected from 0, 1, 2, or 3.

12. The compound, or the N-oxide, the oxo derivative, or the pharmaceutically acceptable salt thereof according to any one of claims 4-11, **characterised in that**
R₆ is selected from hydrogen, deuterium, halogen, amino, hydroxyl, cyano, nitro, oxo, thio, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ deuteroalkoxy, C₁₋₃ haloalkoxy, C₁₋₃ hydroxyalkyl, - C(O)-C₁₋₃ alkyl, C₃₋₈ cycloalkyl, 3- to 8-membered heterocyclyl, C₆₋₁₀ aryl, or 5- to 10-membered heteroaryl;
and is preferably hydrogen, deuterium, methyl, ethyl, deuterated methyl, deuterated ethyl, halomethyl, or haloethyl.

13. The compound, or the N-oxide, the oxo derivative, or the pharmaceutically acceptable salt thereof according to any one of claims 1-12, **characterised in that** 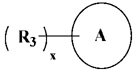 is selected from 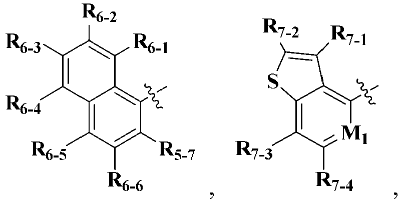 or 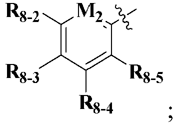
M₁ is selected from CR₇₋₅ or N;
M₂ is selected from CR₈₋₁ or N;
and is preferably 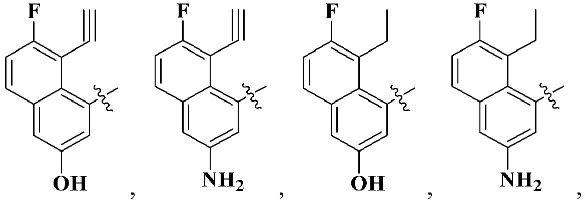 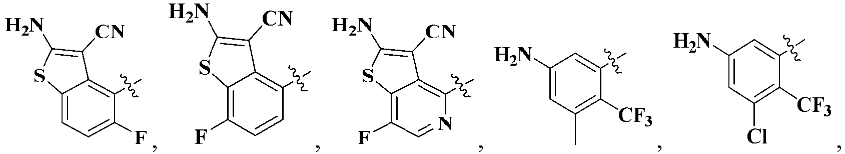 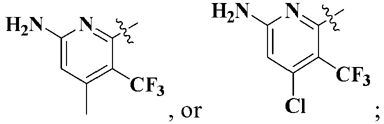
R₆₋₁, R₆₋₂, R₆₋₃, R₆₋₄, R₆₋₅, R₆₋₆, and R₆₋₇ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R₇₋₁, R₇₋₂, R₇₋₃, R₇₋₄, and R₇₋₅ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl;
R₈₋₁, R₈₋₂, R₈₋₃, R₈₋₄, and R₈₋₅ are each independently selected from hydrogen, deuterium, halogen, amino, hydroxyl, cyano, nitro, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl, and the amino, C₁₋₃ alkyl, C₂₋₄ alkenyl, C₂₋₄ alkynyl, C₁₋₃ deuterated alkyl, C₁₋₃ haloalkyl, C₁₋₃ alkoxy, C₁₋₃ alkylthio, C₁₋₃ deuteroalkoxy, C₁₋₃ haloalkoxy, C₁₋₃ hydroxyalkyl, C₁₋₃ alkylamino, C₃₋₈ cycloalkyl, 3- to 8-membered heterocyclyl, C₆₋₁₀ aryl, or 5- to 10-membered heteroaryl may optionally be further substituted with one or more of hydrogen, deuterium, halogen, amino, hydroxyl, cyano, nitro, oxo, thio, C₁₋₆ alkyl, C₂₋₆ alkenyl, C₂₋₆ alkynyl, C₁₋₆ deuterated alkyl, C₁₋₆ haloalkyl, C₁₋₆ alkoxy, C₁₋₆ deuteroalkoxy, C₁₋₆ haloalkoxy, C₁₋₆ hydroxyalkyl, -C(O)-C₁₋₆ alkyl, -C(O)O-C₁₋₆ alkyl, C₁₋₆ silyl, C₃₋₈ cycloalkyl, 3- to 8-membered heterocyclyl, C₆₋₁₀ aryl, and 5- to 10-membered heteroaryl.

14. The compound, or the N-oxide, the oxo derivative, or the pharmaceutically acceptable salt thereof according to any one of claims 1-13, **characterised in that** the compound is selected from the following compounds:

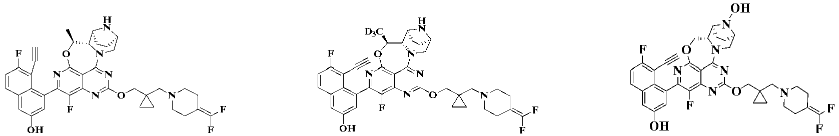

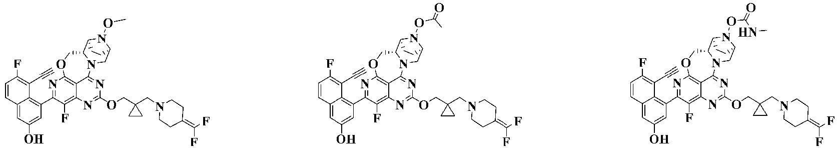

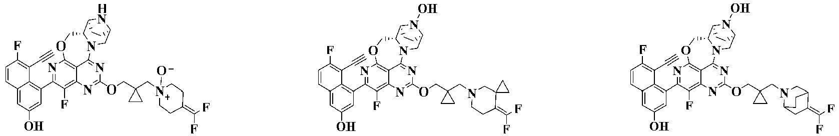

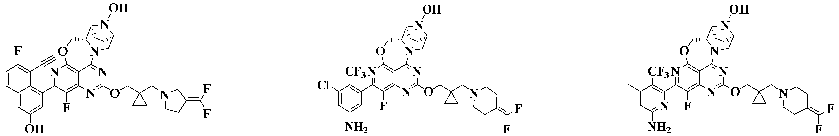

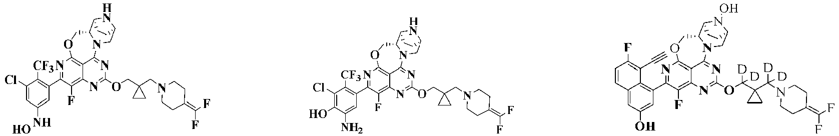

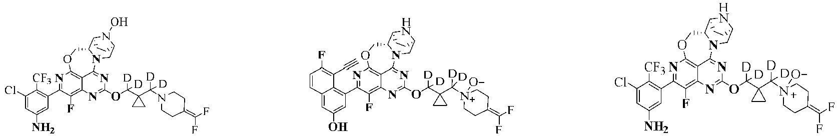

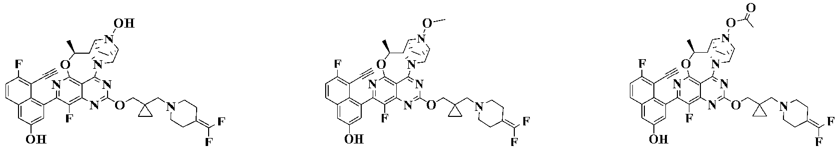

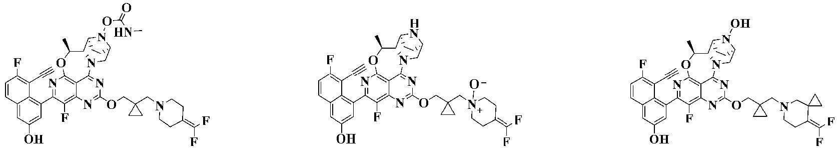

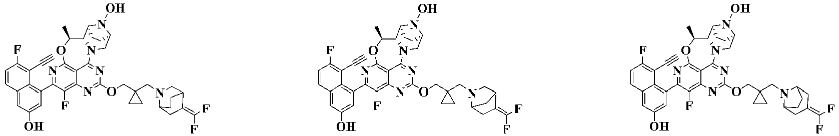

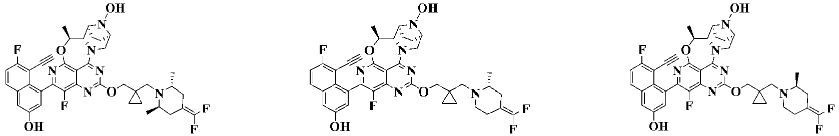

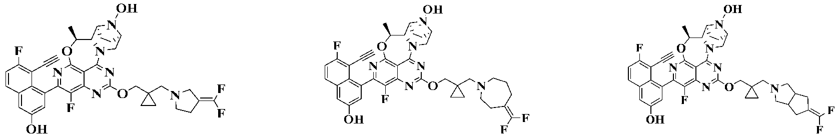

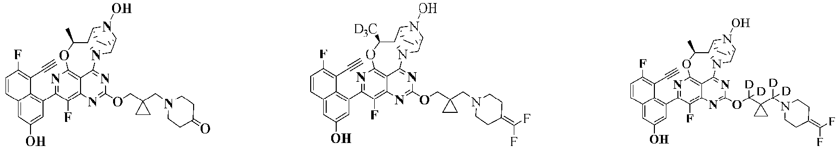

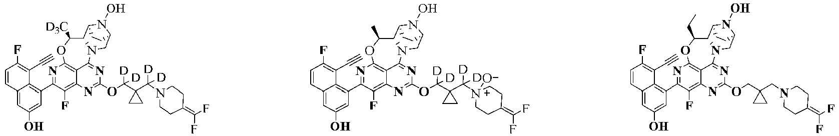

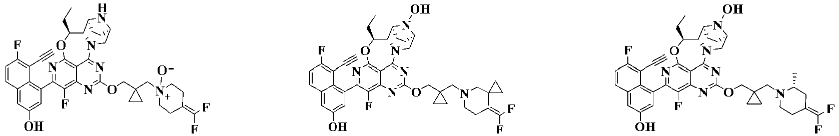

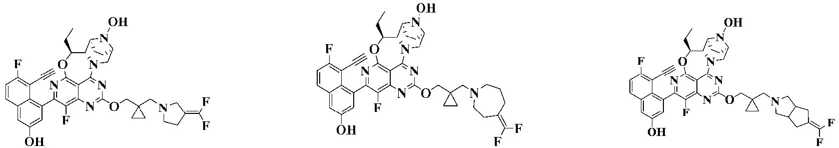

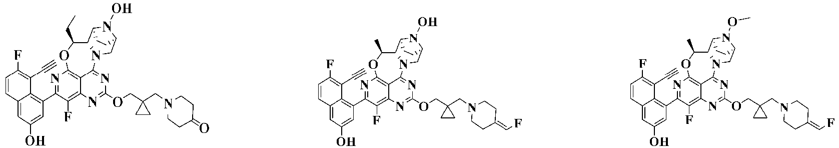

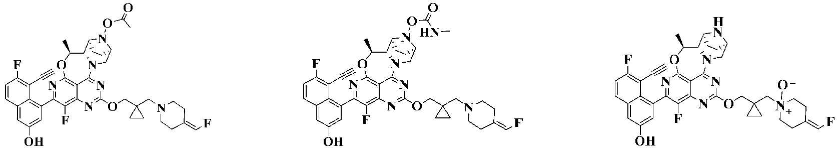

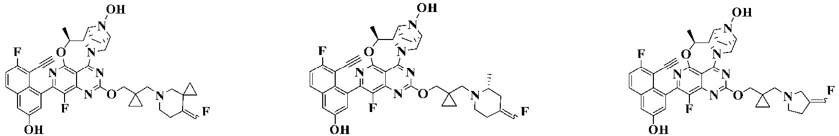

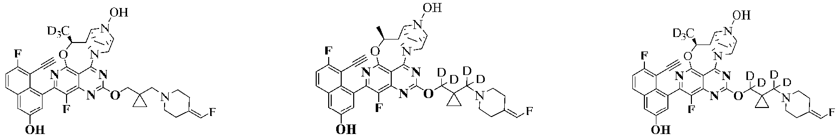

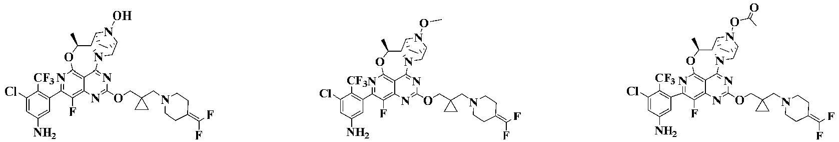

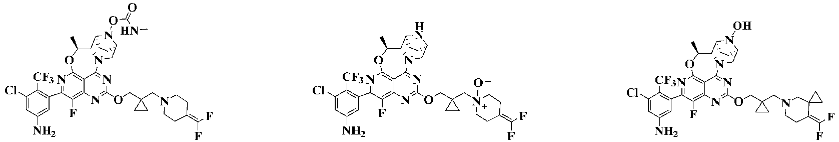

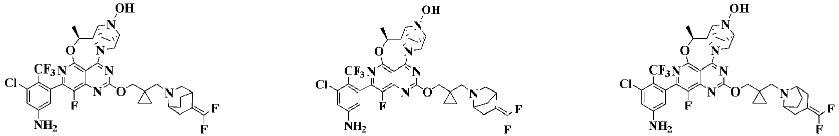

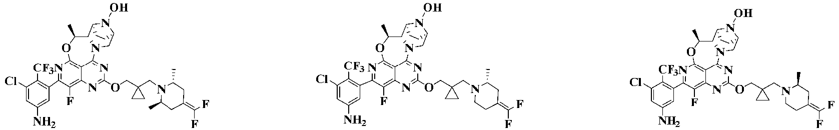

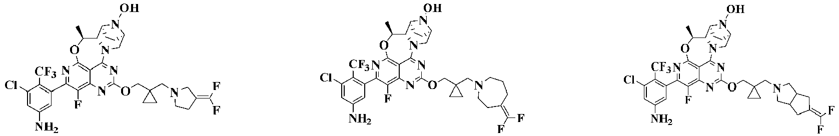

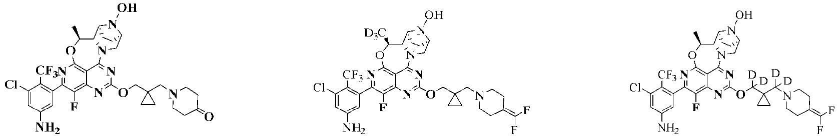

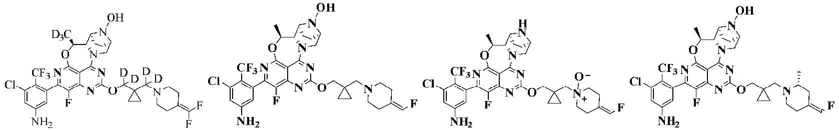

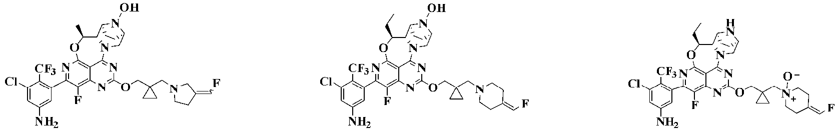

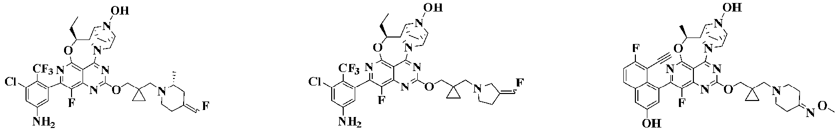

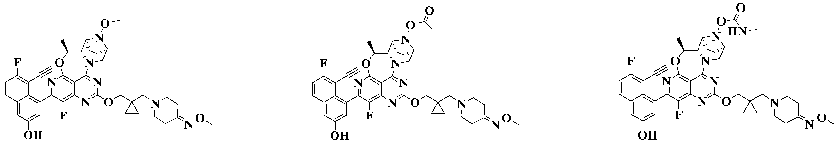

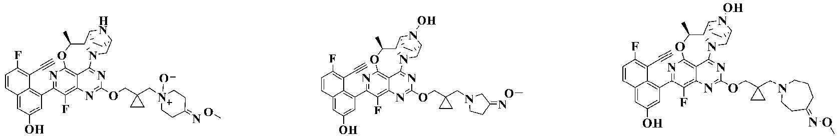

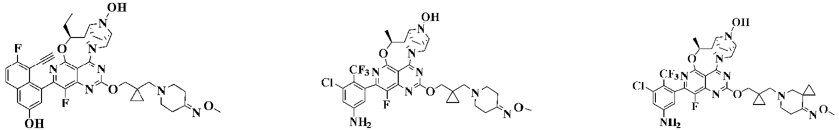

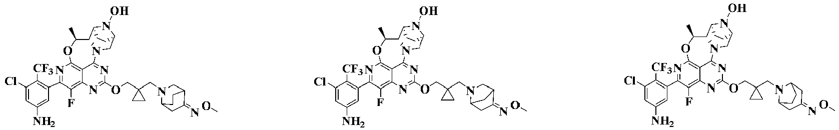

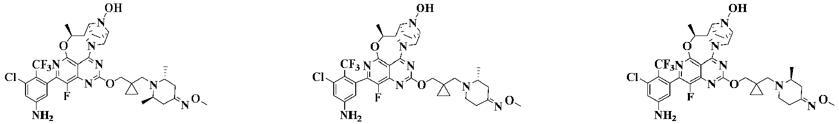

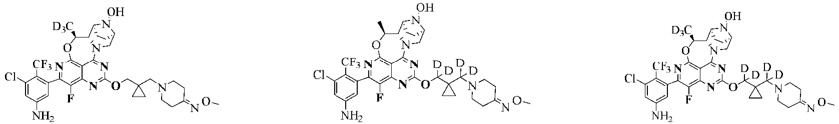

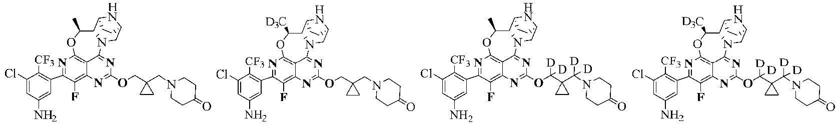

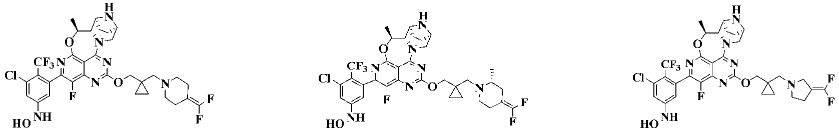

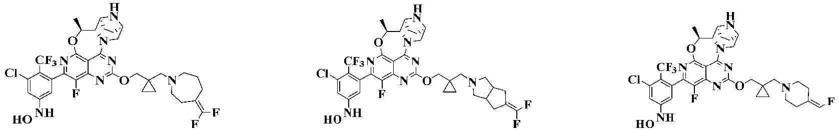

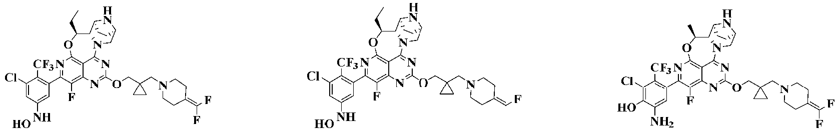

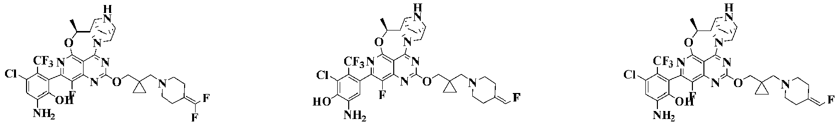

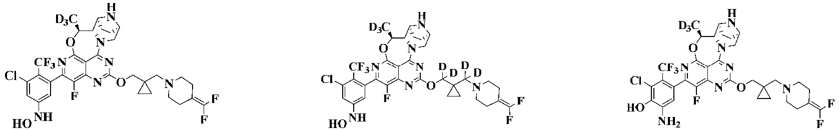

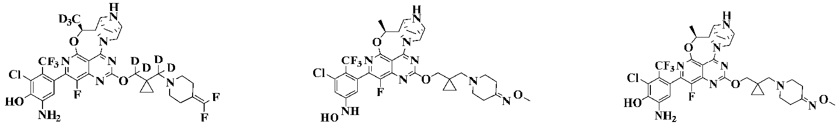

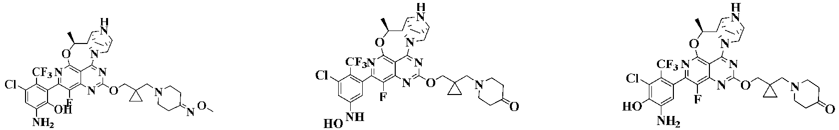

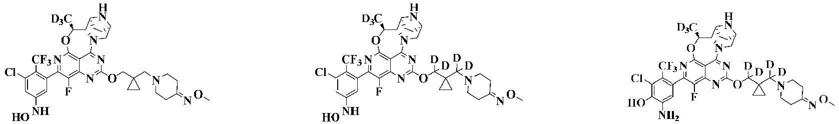

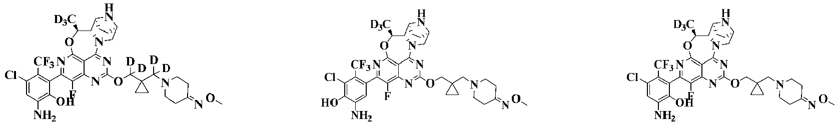

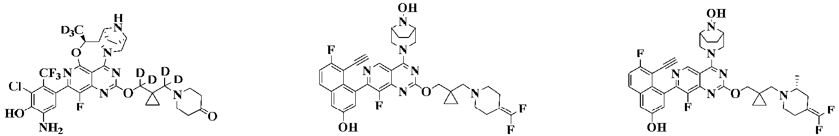

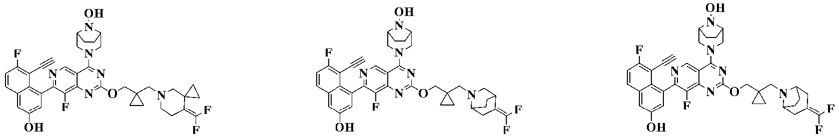

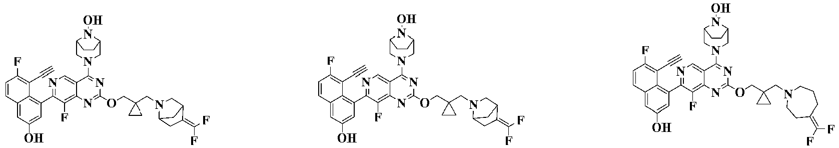

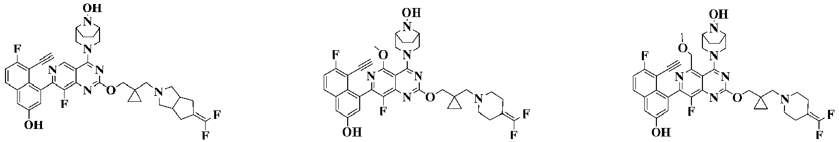

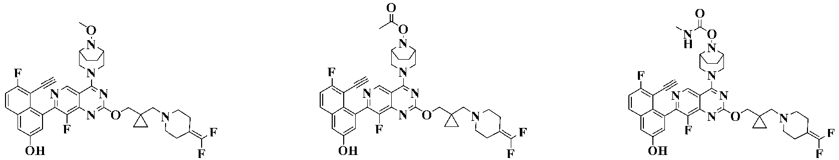

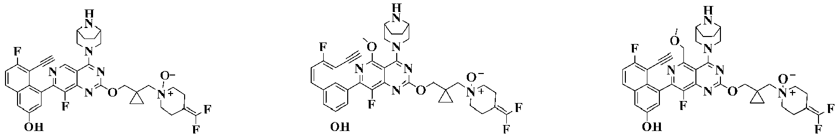

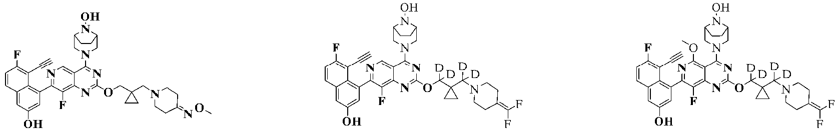

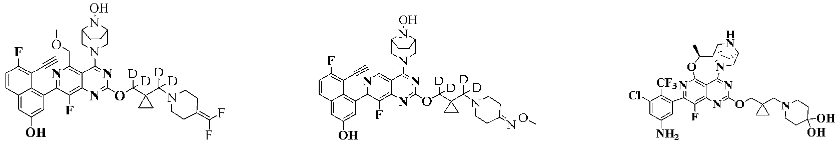

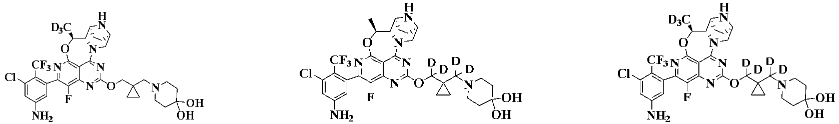

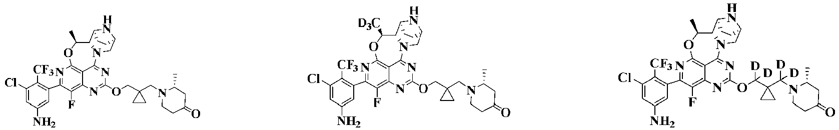

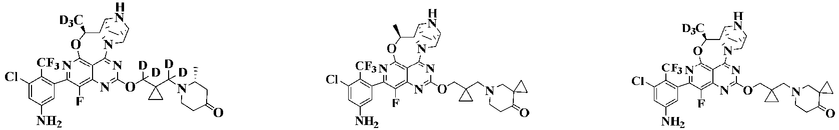

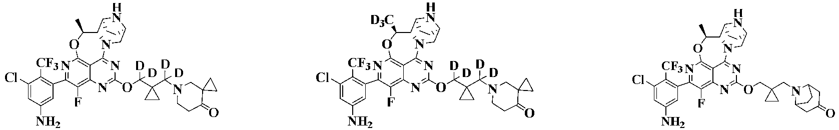

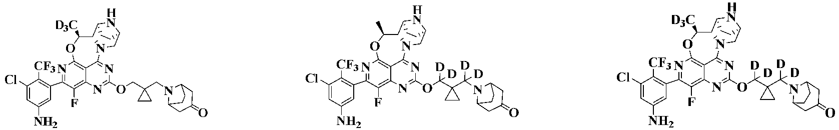

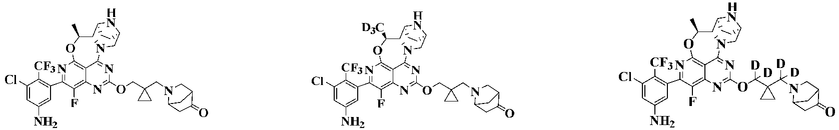

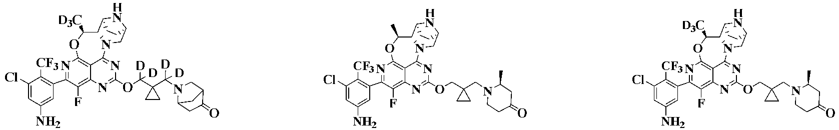

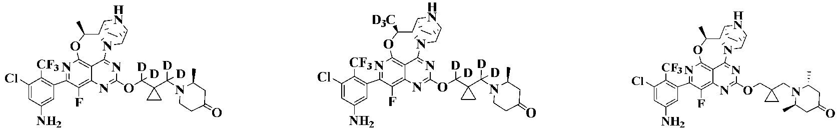

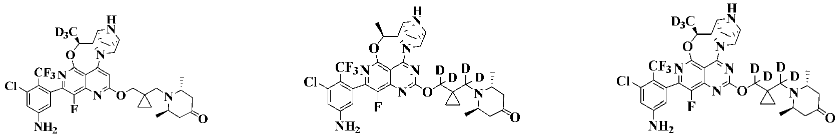

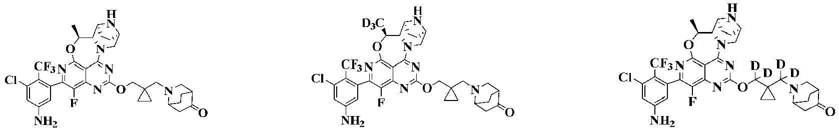

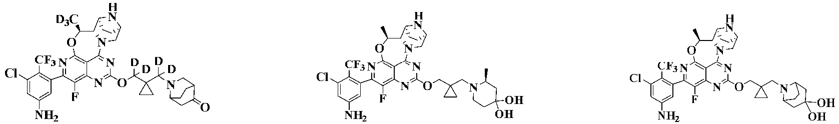

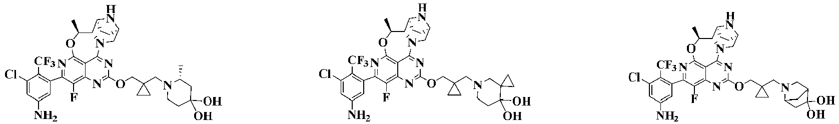

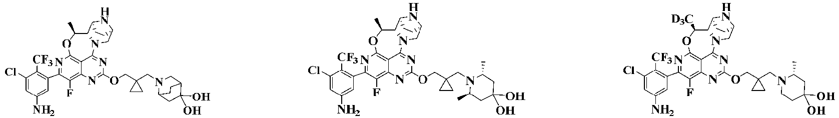

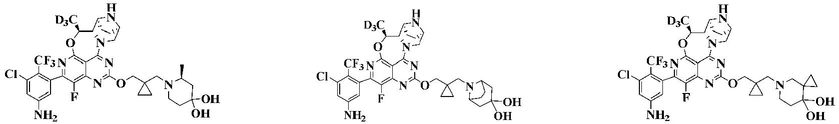

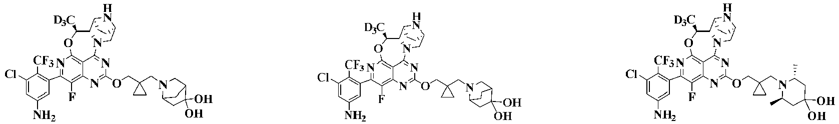

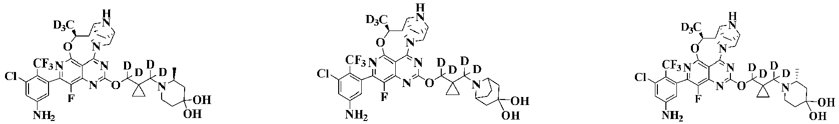

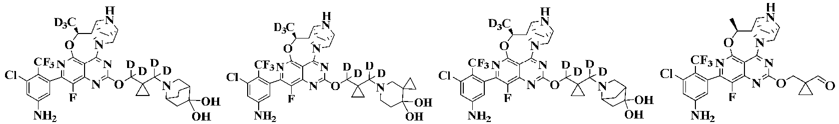

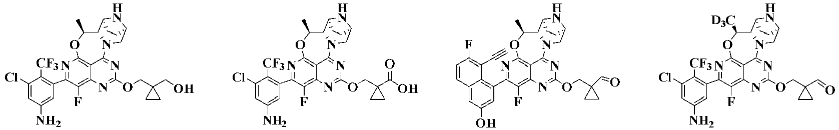

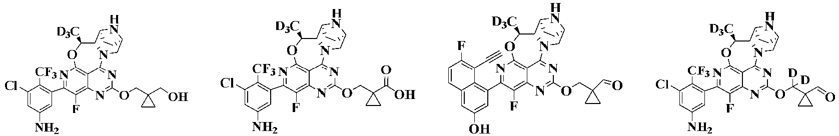

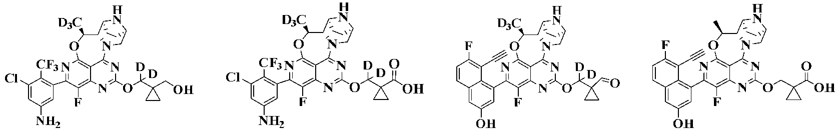

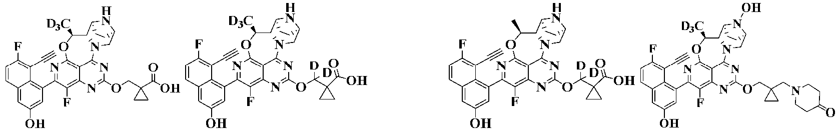

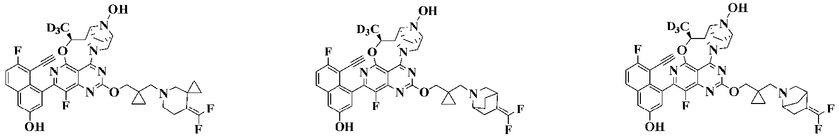

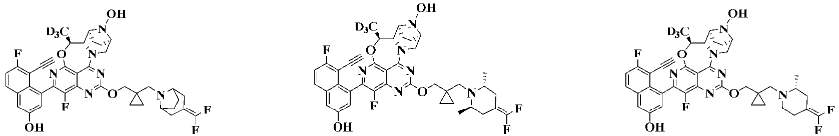

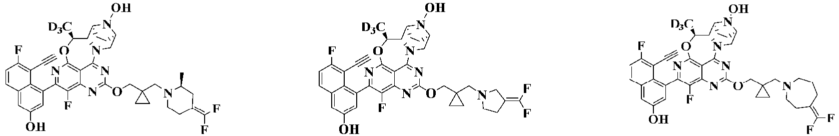

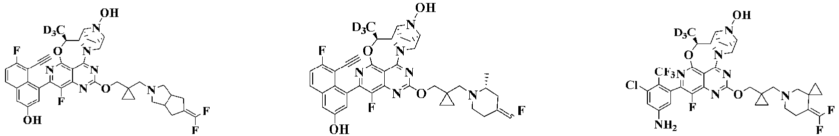

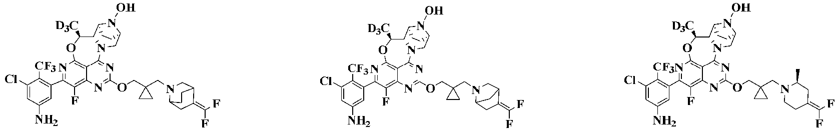

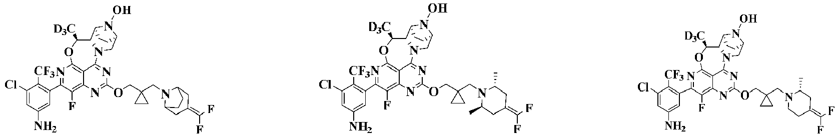

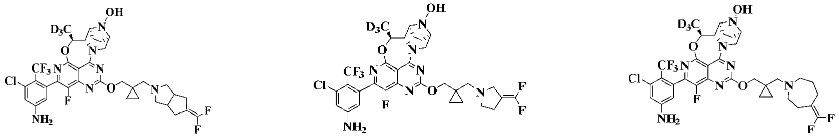

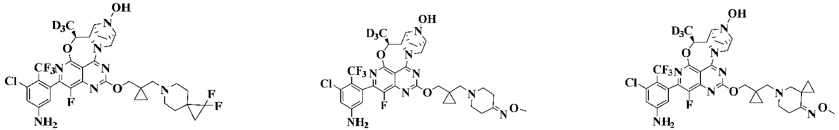

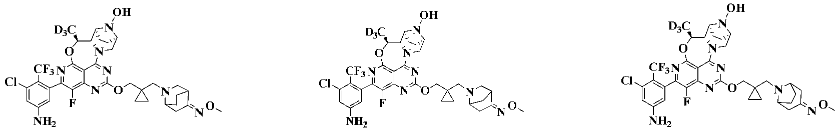

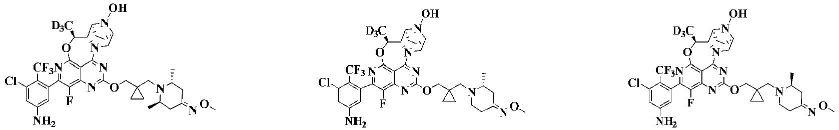

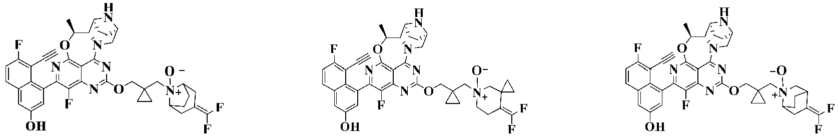

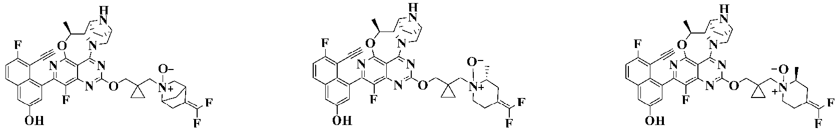

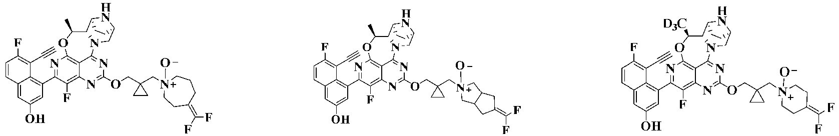

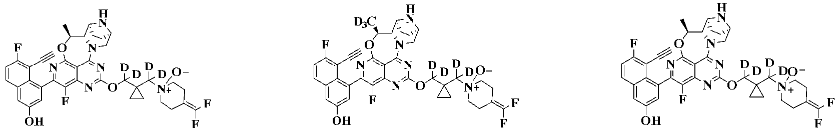

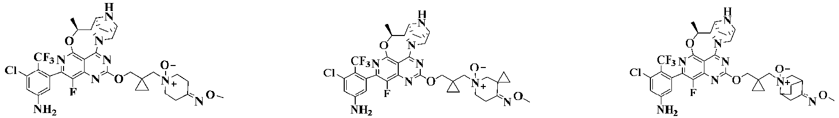

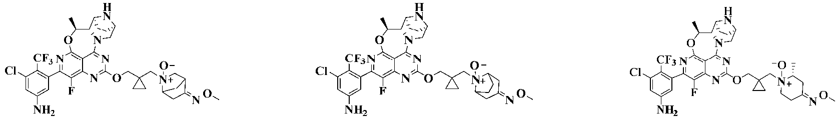

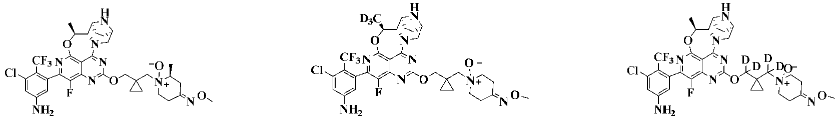

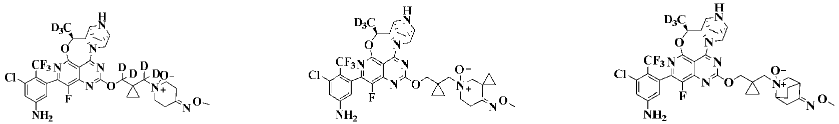

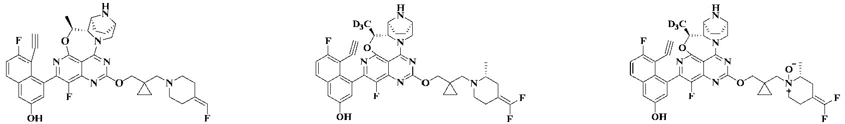

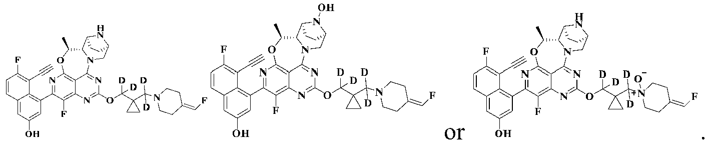

15. A method for preparing a compound represented by general formula (II-B), or an N-oxide, an oxo derivative or a pharmaceutically acceptable salt thereof, **characterised by** comprising the step of: 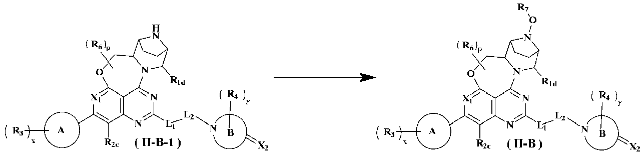
reacting a compound represented by general formula (II-B-1) with a peroxide to obtain the compound represented by general formula (II-B);
ring A, ring B, L₁, L₂, X₁, X₂, R_{1d}, R₂ₐ, R_{2c}, R₃, R₄, R₆, R₇, p, x, and y are as defined in any one of claims 1-13.

16. A method for preparing a compound represented by general formula (III-B), or an N-oxide, an oxo derivative or a pharmaceutically acceptable salt thereof, **characterised by** comprising the step of: 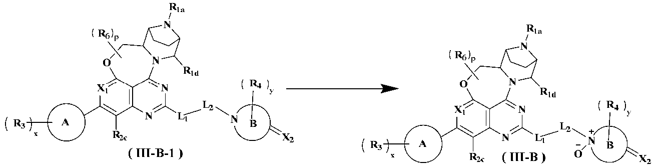
reacting a compound represented by general formula (III-B-1) with a peroxide to obtain the compound represented by general formula (III-B);
ring A, ring B, L₁, L₂, X₁, X₂, R₁ₐ, R_{1d}, R₂ₐ, R_{2c}, R₃, R₄, R₆, R₇, p, x, and y are as defined in any one of claims 1-13.

17. A pharmaceutical composition, **characterised by** comprising a therapeutically effective dose of the compound, or the N-oxide, the oxo derivative, or the pharmaceutically acceptable salt thereof according to any one of claims 1-14 and one or more pharmaceutically acceptable carriers, diluents, or excipients.

18. Use of the compound, or the N-oxide, the oxo derivative, or the pharmaceutically acceptable salt thereof according to any one of claims 1-14, or the pharmaceutical composition according to claim 17 in the preparation of a KRAS inhibitor drug; preferably a KRAS G12D, KRAS G12V, or KRAS G13D inhibitor drug.

19. Use of the compound, or the N-oxide, the oxo derivative, or the pharmaceutically acceptable salt thereof according to any one of claims 1-14, or the pharmaceutical composition according to claim 17 in the preparation of a drug for treating diseases or conditions such as Noonan syndrome, Leopard syndrome, leukaemia, neuroblastoma, melanoma, oesophageal cancer, head and neck tumour, brain cancer, breast cancer, lung cancer, liver cancer, gastric cancer, renal cancer, bile duct carcinoma, prostate cancer, ovarian cancer, pancreatic cancer, small intestine cancer, and colon cancer; preferably in the preparation of a drug for treating non-small cell lung cancer, colon cancer, pancreatic cancer, oesophageal cancer, and head and neck tumour.
